# EUROPEAN PATENT SPECIFICATION

(11) **EP 3 132 034 B1**
(45) Date of publication and mention of the grant of the patent: **19.08.2020**
(21) Application number: 15718982.0
(22) Date of filing: 14.04.2015
(51) Int. Cl.: C12N 15/09

(54) **THERAPEUTIC**
THERAPEUTIKUM
THÉRAPEUTIQUES

(30) Priority: 14.04.2014 GB 201406674; 01.08.2014 GB 201413719; 17.10.2014 GB 201418508
(43) Date of publication of application: 22.02.2017
(73) Proprietor: Nemesis Bioscience Ltd., PE38 0QQ (GB)
(72) Inventor: MIKAWA, Yoshikazu, Downham Market Norfolk PE38 0QQ (GB); LICHTENSTEIN, Conrad, Downham Market Norfolk PE38 0QQ (GB)
(74) Representative: Roberts, Michael Austin
(86) International application number: PCT/GB2015/051132
(87) International publication number: WO 2015/159068

(56) References cited:
- WO-A2-2010/075424
- Huang: "E. curi: A way of deactivating antibiotic resistance genes in E. coli", , 1 April 2012 (2012-04-01), XP002742963, Retrieved from the Internet: URL:http://uscusj.weebly.com/uploads/4/9/5 /1/4951238/jonathan__usj__v3.pdf [retrieved on 2015-08-03]
- CITORIK ROBERT J ET AL: "Sequence-specific antimicrobials using efficiently delivered RNA-guided nucleases", NATURE BIOTECHNOLOGY, vol. 32, no. 11, 1 November 2014 (2014-11-01), pages 1141-1145+2PP, XP002742964,
- BIKARD DAVID ET AL: "Exploiting CRISPR-Cas nucleases to produce sequence-specific antimicrobials", NATURE BIOTECHNOLOGY, vol. 32, no. 11, November 2014 (2014-11), pages 1146-1150, XP002742966,
- YOSEF IDO ET AL: "Temperate and lytic bacteriophages programmed to sensitize and kill antibiotic-resistant bacteria.", PROCEEDINGS OF THE NATIONAL ACADEMY OF SCIENCES OF THE UNITED STATES OF AMERICA, vol. 112, no. 23, 9 June 2015 (2015-06-09) , pages 7267-7272, XP002742965, ISSN: 1091-6490

## Description

The invention relates to recombinant polynucleotides, compositions and methods for interfering with antibiotic resistance genes, and/or replicons carrying such genes, in microorganisms in order to disable antibiotic resistance in the microorganisms.

Antibiotics, originally isolated from microorganisms such as Streptomyces, are a powerful way to treat infectious disease. However, very quickly bacteria acquired anti-microbial resistance (AMR) to antibiotics in response to selection pressure. One common route to AMR has been the acquisition of resistance genes evolved in the original antibiotic-producing microorganisms, via horizontal transmission on plasmid vectors. Such plasmids have in some instances acquired multiple antibiotic resistance genes carried by transposable elements and integrons. Host-encoded mutations that modify the bacterial protein target or prevent entry of the antibiotic have also occurred.

Resistance to antibiotics by microorganisms such as bacterial pathogens is one of our most serious health threats. Infections from resistant bacteria, for example, are now not uncommon, and some pathogens have even become resistant to multiple types or classes of antibiotics. The loss of effective antibiotics undermines our ability to fight infectious diseases and manage the infectious complications common in vulnerable patients, for example those undergoing chemotherapy for cancer, dialysis for renal failure, and surgery, especially organ transplantation, for which the ability to treat secondary infections is critical.

When first-line and second-line treatment options are limited by antibiotic resistance or are unavailable, healthcare providers are forced to use alternative antibiotics that may be more toxic to the patient, more expensive and less effective. Even when alternative treatments exist, patients with resistant infections are often more likely to die, while survivors may have significantly longer hospital stays, delayed recuperation, and long-term disability.

Many achievements of modern medicine are put at risk by AMR. Without effective antibiotics for care and prevention of infections, the success of treatments such as organ transplantation, cancer chemotherapy and major surgery would be compromised.
The growth of global trade and travel allows antibiotic resistant microorganisms to be spread rapidly through humans, other animals, and food.

Resistance mechanisms fall into four classes:
(1) enzymes that degrade antibiotics, including beta-lactamases that break the beta-lactam ring of the penicillin family of antibiotics;
(2) enzymes that modify antibiotics include aminoglycoside phosphotransferases that phosphorylate aminoglycoside antibiotics such as kanamycin; chloramphenicol acetyl-transferase (CAT) that acetylate chloramphenicol;
(3) efflux pumps that actively export antibiotics from cytoplasm out of the cell, such as the tetracycline efflux pump that is expressed in the presence of tetracycline, plus other pumps, conferring multidrug resistance, that are capable of exporting a range of antibiotics; and
(4) mutations that change the protein target of the antibiotic such that it is no longer inactivated by it; for example, beta-lactams are bactericidal because they inhibit penicillin-binding proteins (PBPs) that are required for peptidoglycan biosynthesis and bacterial cell wall integrity and PBP mutants with reduced binding to beta-lactams will not be inhibited.

Several approaches are currently being used or developed to address the problem of antibiotic resistance, including the following.

Firstly, new antibiotics, such as derivatives of existing drugs, have been developed. Fewer new antibiotic drugs have been developed, and many are more toxic so are used in the last resort. Microorganisms have acquired resistance to new antibiotics of both types.

Secondly, direct inhibition of resistance enzymes has been attempted. Examples include clavulanic acid, a beta-lactamase inhibitor which is used in combination with amoxycillin, a beta-lactam antibiotic (also called Augmentin). Other beta-lactamase antibiotic inhibitors include the carbapenems. But even here resistance has appeared. Blueberry Therapeutics and Avacta are developing peptide affimers to target mechanisms of resistance. Development of new inhibitors of antibiotic resistance enzymes requires a long pipeline of drug development. An alternative approach has been adopted by Dr Eric Brown at McMaster University, Canada, who is screening known drugs (already approved for use) with unrelated targets, for cryptic activity in inactivating antibody resistance mechanisms.

Thirdly, non-antibiotic bactericides have been used. For example, infection by bacteriophage was developed in the 1920's and although largely discontinued with the discovery of antibiotics, has been retained in certain countries. Current approaches use virulent, lytic bacteriophage that kill bacteria, including antibiotic resistant bacteria, but this opens the way for selection of bacterial variants that are resistant to bacteriophage infection. To obviate this, preparations containing a mixture of different strains of bacteriophage are being used. Another disadvantage of the use of such lytic bacteriophage in patients suffering from sepsis is that cell lysis and death by lytic bacteriophage can release endotoxins from the cell into the blood and can cause endotoxin shock.

Further compositions and methods for combating antibiotic resistant microorganisms are required.

According to an aspect of the present invention, there is provided a delivery vehicle for introducing a polynucleotide into an antibiotic-resistant microorganism, wherein the delivery vehicle is selected from the group consisting of a plasmid (such as a conjugative plasmid or other plasmid replicon), a nucleotide vector, linear double-stranded DNA, a non-virulent bacteriophage and a lysogenic bacteriophage, and comprises a recombinant polynucleotide for inactivation of DNA carrying at least one antibiotic resistance gene encoding an enzyme which confers antibiotic resistance to a microrganism, wherein the recombinant polynucleotide comprises a clustered regularly interspaced short palindromic repeat (CRISPR) array nucleic acid sequence having or transcribing multiple RNA guide molecules, wherein each RNA guide molecule:
(i) is transcribed from its own promoter sequence;
(ii) mediates the binding of a CRISPR associated (Cas) DNA-binding polypeptide or its functional equivalent or its modified version thereof to the at least one antibiotic resistance gene(s); and
(iii) has a spacer sequence sufficiently complementary to a target DNA sequence of the at least one antibiotic resistance gene for the at least one antibiotic resistance gene to be targeted and inactivated in the presence of a CRISPR associated (Cas) DNA-binding polypeptide or a functional equivalent or a modified version thereof,
   and wherein:
   either the Cas DNA-binding polypeptide or a functional equivalent or a modified version thereof is a modified Cas DNA-binding polypeptide which creates a deletion and reseals the target DNA sequence to inactivate the at least one antibiotic resistance gene to prevent double-strand break (DSB)-induced killing of the microorganism, such that the microorganism becomes sensitised to an antibiotic, or
   the Cas DNA-binding polypeptide or a functional equivalent or a modified version thereof creates a DSB in the target DNA sequence to inactivate the at least one antibiotic resistance gene, and the delivery vehicle further comprises a nucleotide sequence encoding a gene product which prevents direct killing of the microorganism due to the generation of the DSB in the at least one antibiotic resistance gene, such that the microorganism becomes sensitised to an antibiotic.

The delivery vehicle of the invention may further comprise a nucleic acid sequence which encodes the Cas DNA-binding polypeptide or its functional equivalent or its modified version.

The Cas DNA-binding polypeptide may be Cas9 or a functional equivalent or a modified version thereof.

The delivery vehicle of the invention may comprise a further RNA guide molecule which targets a gene involved in pathogenicity or other aspects of microbial metabolism, for example a gene involved in bacterial metabolism for biofilm production.

The modified Cas DNA-binding polypeptide may comprise a recombinase catalytic domain to reseal target DNA sequence.

The gene product that prevents direct killing of the microorganism may be a protein encoded by a replicon subject to degradation due to the DSB caused by the Cas DNA-binding polypeptide, or an antitoxin that neutralises the effect of a toxin or killer function carried by a replicon on which the target DNA sequence is located.

The CRISPR array nucleic acid sequence may have or transcribe one or more RNA guide molecules each comprising a spacer sequence sufficiently complementary to a target sequence of one or more beta-lactamase genes, for example one or more or all of the genes selected from the group consisting of : NDM, VIM, IMP, KPC, OXA, TEM, SHV, CTX, 0KP, LEN, GES, MIR, ACT, ACC, CMY, LAT, and FOX.

Another aspect of the invention is a composition comprising the delivery vehicle as defined herein.

The composition may be a pharmaceutical composition, a non-pathogenic microorganism such as a commensal bacterium for example in a probiotic formulation, or a dietary supplement. The composition may be formulated for topical, enteral or parenteral administration.

A further aspect of the invention is a composition as defined herein for use as a medicament.

Also provided according to the invention is a composition as defined herein for use in the treatment or prevention of an infection caused by an antibiotic-resistant microorganism comprising one or more antibiotic resistance genes targeted by the RNA guide molecules of the recombinant polynucleotide.

Further provided according to the invention a method of inactivating antibiotic resistance in an antibiotic-resistant microorganism, the method comprising introducing into the microorganism the delivery vehicle as defined herein.

Another aspect of the invention provides a host cell comprising the recombinant polynucleotide as defined herein. The host cell may be a commensal bacterium.

The present application discloses a recombinant polynucleotide comprising a clustered regularly interspaced short palindromic repeat (CRISPR) array nucleic acid sequence having or transcribing an RNA guide molecule with a spacer sequence sufficiently complementary to a target sequence of an antibiotic resistance gene in a microorganism for the antibiotic resistance gene to be inactivated in the presence of a CRISPR associated (Cas) DNA-binding polypeptide or a functional equivalent or a modified version thereof, thereby sensitising the microorganism to the antibiotic.

Also disclosed is a delivery vehicle for introducing a polynucleotide into a microorganism, such as an antibiotic-resistant microorganism, in which the delivery vehicle comprises a recombinant polynucleotide for inactivation of DNA carrying a gene encoding an antibiotic resistance enzyme which confers antibiotic resistance to the microrganism, and in which the recombinant polynucleotide comprises a clustered regularly interspaced short palindromic repeat (CRISPR) array nucleic acid sequence having or transcribing an RNA guide molecule with a spacer sequence sufficiently complementary to a target DNA sequence of the antibiotic resistance gene for the antibiotic resistance gene to be targeted and inactivated in the presence of a CRISPR associated (Cas) DNA-binding polypeptide or a functional equivalent or a modified version thereof, thereby sensitising the microorganism to the antibiotic, wherein the delivery vehicle is a non-virulent or a lysogenic bacteriophage.

One general aim of the present invention is inactivation of DNA carrying a gene encoding an antibiotic resistance enzyme using a CRISPR/Cas system. An advantage of the invention is that one or more existing antibiotics can be used to treat infectious disease, as microorganisms become re-sensitised to the antibiotics or are prevented from acquiring antibiotic resistance.

The target sequence of an antibiotic resistance gene may be a sequence flanking the gene itself which, if disrupted, inactivates the antibiotic resistance gene. For example, if the antibiotic resistance gene is located on a plasmid, the invention may encompass a target sequence in the plasmid.

In contrast to prior art approaches of inactivating antibiotic resistance enzymes, the present invention will not require new drug development and the concomitant regulatory approval required for each new drug. Rather, the invention provides a tool which can be applied to target and inactivate relevant antibiotic resistance genes directly rather than the gene products. For example, a gene encoding an antibiotic resistance enzyme, or a gene encoding a protein regulating the uptake and export of an antibiotic by altering the membrane permeability and efflux pump expression, respectively, can be targeted.

The CRISPR/Cas system is an RNA-mediated genome defense pathway that is part of a natural bacterial and archaeal immune system against nucleic acid invaders, analogous to the eukaryotic RNAi pathway (see for example Grissa et al., 2007, BMC Informatics 8: 172; Horvath & Barrangou, 2010, Science, 327: 167-170; Gasiunas et al., 2012, Proc. Natl Acad. Sci. USA 109: E2579; Marraffini & Sontheimer, 2008, Science, 322: 1843-1845; Garneau et.al.,2010, Nature 468: 67). Natural CRISPR systems contain a combination of Cas genes as well as non-coding RNA elements capable of programming the specificity of the CRISPR-mediated nucleic acid cleavage. Three types (I-III) of CRISPR systems have been identified thus far in a wide range of bacterial and archaeal hosts. Each CRISPR locus is composed of a series of short DNA direct repeats separated by non-repetitive spacer sequences. The spacer sequences, in nature, typically originate from foreign genetic elements such as bacteriophage and plasmids. As used herein, the series of repeats plus non-repetitive spacer sequences is known as a CRISPR array. The CRISPR array is transcribed and hybridised with repeat complementary tracrRNA followed by cleavage within the direct repeats and processed into short mature dual tracrRNA:crRNAs containing individual spacer sequences, which direct Cas nucleases to a target site (also known as a "protospacer"). For example, the Type II CRISPR/Cas9 system, a well-studied example, carries out a targeted DNA double-strand break ("DSB") in four steps. Firstly, two RNAs, the pre-crRNA array and tracrRNA, are transcribed from the CRISPR locus. Secondly, tracrRNA hybridises to the repeat regions of the pre-crRNA and mediates the processing of pre-crRNA into mature crRNAs (also referred to herein as "RNA guide molecules gRNA" containing individual or monomer spacer sequences. Thirdly, the mature crRNA:tracrRNA complex directs Cas9 protein in the form of a ribonucleoprotein to the target DNA via base-pairing between the spacer on the crRNA and the target site on the target DNA. Finally, Cas9 mediates cleavage of target DNA and creates a DSB.

In the present invention, as elaborated herein, modified CRISPR constructs are used to target antibiotic resistance genes. The recombinant polynucleotide of the invention using such a construct is also referred to herein as an "assassin construct" which is used to effect inactivation of such genes.

The main focus of using CRISPR technology to date has been for use as a DNA editing tool for reverse genetics, primarily in eukaryotes. However, WO2007/025097 describes the use of CRISPR technology for modulating resistance in a cell against an invading target nucleic acid or a transcription product thereof, especially against invading bacteriophages. Methods for downregulating prokaryotic gene expression using CRISPR technology to target mRNA transcribed by the genes have been suggested for example in WO2010/075424. WO2012/164565 describes a CRISPR system from *Lactoccocus* and use of the system for modulating resistance of a cell against an invading target nucleic acid or a transcription product thereof. The present invention, by contrast, concerns *inter alia* inactivation in an antibiotic-resistant microorganism of genes involved in conferring the antibiotic resistance.

According to the invention, the RNA guide molecule mediates binding of the Cas DNA-binding polypeptide or its functional equivalent or its modified version to the antibiotic resistance gene. This mirrors the natural system described above.

The Cas DNA-binding polypeptide or its functional equivalent or its modified version may also be capable of binding to RNA or other nucleic acid molecules. In other words, the requirement for the Cas DNA-binding polypeptide or its functional equivalent or its modified version to be capable of binding DNA does not exclude the polypepeptide or its functional equivalent or its modified version being capable of binding RNA or other nucleic acid molecules. The Cas DNA-binding polypeptide or its functional equivalent or its modified version may be referred to as a Cas nucleic acid-binding polypeptide or its functional equivalent or its modified version.

For certain applications, the microorganism may have a natural endogenous, or introduced engineered, Cas DNA-binding polypeptide or functional equivalent or modified version. This means that the recombinant polynucleotide of the invention is not required to encode the Cas DNA-binding polypeptide or functional equivalent or modified version. Alternatively, the recombinant polynucleotide of the invention may further comprise a nucleic acid sequence which encodes the Cas DNA-binding polypeptide or its functional equivalent or modified version. In another aspect, the recombinant polynucleotide of the invention does not encode the Cas DNA-binding polypeptide or its functional equivalent or modified version but may be used in conjunction with a separate polynucleotide which does. Other means for introducing the Cas DNA-binding polypeptide or its functional equivalent or its modified version into the microorganism may be used.

An exemplar Cas DNA-binding polypeptide according to the invention is Cas9 or a functional equivalent thereof or a modified version thereof.

In the recombinant polynucleotide according to various aspects of the invention, the CRISPR array nucleic acid sequence has or transcribes multiple RNA guide molecules each comprising a spacer sequence sufficiently complementary to a target sequence of the antibiotic resistance gene or one or more additional antibiotic resistance genes. The or each RNA guide molecule is transcribed from its own promoter sequence.

Having multiple RNA guide molecules allows different antibiotic resistance (or other types of) genes in a microorganism to be targeted and inactivated simultaneously.

The recombinant polynucleotide according to various aspects of the invention may additionally be designed to include an RNA guide molecule (such as a further RNA guide molecule) targeting a gene involved in pathogenicity or other aspects of microbial metabolism. For example, certain pathogens form biofilms that make it difficult for antibiotics to gain access to them. One or more genes involved in bacterial metabolism for biofilm production may be targeted.

Spacer sequence distal from a promoter are typically less efficiently transcribed. Ideally, multiple RNA guide molecules to different targets should be more or less equally represented. Thus, one promoter transcribing each RNA guide molecule is used (instead of relying on a long polycistronic RNA guide molecule [or precursor crRNA] transcription).

For example, there are many resistance genes encoding beta-lactamases (*bla* genes) giving resistance to a large range of different beta-lactam antibiotics. DNA constructs expressing multiple RNA guide molecules, which are each individually transcribed from their own such promoters, may be used to target a number of different *bla* genes.

Thus in aspects of the invention, the CRISPR array nucleic acid sequence may have or transcribe one or more RNA guide molecules each comprising a spacer sequence sufficiently complementary to a target sequence of one or more beta-lactamase genes.

For example, the one or more RNA guide molecules may target one or more or all of the genes selected from the group consisting of: NDM, VIM, IMP, KPC, OXA, TEM, SHV, CTX, OKP, LEN, GES, MIR, ACT, ACC, CMY, LAT, and FOX.

In particular, the one or more RNA guide molecules may comprise a spacer sequence sufficiently complementary to target sequences of the beta lactam family of antibiotic resistance genes, including one or more or all of the following: a first spacer sequence sufficiently complementary to target sequences for NDM-1, -2, -10; a second spacer sufficiently complementary to target sequences for VIM-1, -2, -4, -12, -19, -26, -27- 33, 34; a third spacer sufficiently complementary to target sequences for IMP-32, -38, -48; a fourth spacer sufficiently complementary to target sequences for KPC- 1, -2, -3, -4, -6, -7, -8,-11 , -12 , -14 , -15 , -16, -17; a fifth spacer sufficiently complementary to target sequences for OXA-48; a sixth spacer sufficiently complementary to target sequences for TEM-1,-1B, -3,-139, -162, -183, -192, -197, -198, -209, a seventh spacer sufficiently complementary to target sequences for SHV and its variants; and an eighth spacer sufficiently complementary to target sequences for CTX and its variants.

The antibiotic resistance gene to be inactivated may be located on a chromosome, or on an extrachromosomal replicating DNA molecule known as a replicon and including plasmids and bacteriophage.

The CRISPR/Cas system used according to an aspect of the invention generates a DSB in the target sequence. Where the target sequence is located on a chromosome or a replicon such as a bacterial chromosome or plasmid, then a DSB can lead to degradation and hence loss of the chromosome or replicon suffering such a DSB. If the target sequence is located on a bacterial chromosome then the cell may die directly as a consequence of the DSB. Additionally, some plasmids (including natural plasmids) carry killing functions that only become toxic if the cell loses the plasmid, which is a natural mechanism to ensure faithful inheritance of plasmids in dividing cells. If a plasmid carrying the target sequence of the antibiotic resistance gene also carries such a killing function, and the plasmid is lost as a result of the DSB generated, the cell may die (see Sengupta & Austin, 2011, Infect. Immun. 79: 2502-2509).

In the event that cell death caused by such DSB increases selection pressure for resistance against the recombinant polynucleotide according to various aspects of the present invention, this may be mitigated by, for example, employing a modified Cas DNA-binding polypeptide which seals the target site after generating a deletion to inactivate the target sequence of the antibiotic resistance gene, rather than generate a DSB.

Thus the Cas DNA-binding polypeptide according to various aspects of the invention may in certain aspects be substituted by a modified Cas DNA-binding polypeptide comprising a recombinase catalytic domain, wherein the modified Cas DNA-binding polypeptide does not generate DSBs but creates a deletion and reseals a site in the target sequence.

The modified Cas DNA-binding polypeptide may for example be a modified Cas9 protein comprising a recombinase catalytic domain.

The recombinant polynucleotide according to various aspects of the invention may further comprise a nucleotide sequence which encodes a gene conferring a selective advantage to the microorganism, for example thereby increasing the efficiency of delivery of the CRISPR/Cas system to the target microorganism. For example, the gene may confer a growth advantage over non-infected siblings, or genes encoding a bacteriocin - these are protein toxins produced by bacteria to kill or inhibit growth of other bacteria - and corresponding immunity polypeptide may be used.

The selective advantage to the microorganism may include or be one which prevents or diminishes the effect of loss of a replicon due to a DSB caused by Cas DNA-binding polypeptide. For example, the nucleotide sequence which encodes a gene conferring a selective advantage to the microorganism may encode an antitoxin that neutralises the effect of a toxin or killer function carried by a replicon on which the target sequence is located. Also, the nucleotide sequence which encodes a gene conferring a selective advantage to the microorganism may encode one or more proteins that are encoded by a replicon subject to degradation due to a DSB caused by Cas DNA-binding polypeptide.

In an aspect of the invention, there is provided a delivery vehicle for introducing a polynucleotide into a microorganism, in which the delivery vehicle comprises the recombinant polynucleotide as defined herein.

The delivery vehicle according to various some aspects of the invention may be a a non-virulent or a lysogenic bacteriophage. Alternatively, the delivery vehicle may be a plasmid such as a conjugative plasmid or other plasmid replicon, a nucleotide vector, or linear double-stranded DNA (dsDNA).

The delivery vehicle may be a non-virulent bacteriophage, such as bacteriophage M13, a filamentous phage that infects *Escherichia coli* cells, replicates and is secreted from the bacterial host cell without killing the bacterial host, which continues to grow and divide more slowly. Another suitable filamentous phage is NgoPhi6 isolated from *Neisseria gonorrhoeae* that is capable of infecting a variety of Gram negative bacteria without killing the host.

Alternatively, lysogenic phage may be used that do not always kills host cells following infection, but instead infect and become dormant prophage.

This invention may thus use a novel system to inactivate antibiotic resistance genes in bacteria primarily using bacteriophage that do not kill bacteria, and/or conjugative plasmids and/or direct DNA transformation, as the delivery mechanisms for the recombinant polynucleotide of the invention.

In a further aspect of the invention, there is provided a composition comprising the delivery vehicle as defined herein.

The composition may be a pharmaceutical composition, a non-pathogenic microorganism such as a commensal bacterium for example in a probiotic formulation, or a dietary supplement.

As used herein, a "pharmaceutical composition" refers to a preparation of one or more of the active agents (such the recombinant polynucleotide or the delivery vehicle as described herein) with other chemical components such as physiologically suitable carriers and excipients. The purpose of a pharmaceutical composition is to facilitate administration of the active agent to an organism.

The composition may be formulated for topical, enteral or parenteral administration.

Compositions of the present invention may, if desired, be presented in a pack, dispenser device or kit, each of which may contain one or more unit dosage forms containing the active agent(s). The pack, dispenser device or kit may be accompanied by instructions for administration.

Also provided according to the invention is the composition as defined herein for use as a medicament.

In another aspect there is provided according to the invention a composition as defined herein for use in the treatment or prevention of an infection caused by an antibiotic-resistant microorganism comprising an antibiotic resistance gene targeted by the RNA guide molecule of the recombinant polynucleotide.

Also disclosed is a method of treating or preventing an infection in a subject caused by an antibiotic-resistant microorganism comprising an antibiotic resistance gene, in which the method comprises the step of introducing into the microorganism a therapeutically effective amount of the composition as defined herein where the RNA guide molecule targets the antibiotic resistance gene, thereby inactivating the antibiotic resistance gene and sensitising the microorganism to the antibiotic.

The composition may be administered topically or orally. Alternatively the composition may be administered by intravenous, parenteral, ophthalmic or pulmonary administration.

Compositions of the present invention for administration topically can be in a form suitable for topical use such as, for example, an aerosol, cream, ointment, lotion or dusting powder.

Compositions provided herein may be formulated for administration by inhalation. For example, the compositions may be in a form as an aerosol, a mist or a powder. Thus compositions described herein may be delivered in the form of an aerosol spray presentation from pressurised packs or a nebuliser, with the use of a suitable propellant such as for example dichlorodifluoromethane, trichlorofluoromethane, dichlorotetrafluoroethane, carbon dioxide or other suitable gas. Where using a pressurised aerosol, a dosage unit may be determined by providing a valve to deliver a metered amount.

In the disclosed method, the subject may be a plant, a fish, a bird, a reptile or a mammal (such as a human).

According to the disclosed method, the delivery vehicle may be transferred from the microorganism directly into another microorganism (such as antibiotic-resistant microorganism) by plasmid conjugation or bacteriophage infection.

The disclosed method may further comprise a step of simultaneously or subsequently administering to the subject an antibiotic to which a microorganism has become sensitised.

A further aspect of the invention provides a method of inactivating antibiotic resistance in a microorganism, the method comprising introducing into the microorganism of the delivery vehicle as defined herein.

Also provided according to the invention is a host cell comprising the recombinant polynucleotide defined here. The host cell may, for example, be a commensal bacterium.

Microorganisms targeted by various aspects of the invention may be on a body surface, localised (for example, contained within an organ, at a site of a surgical wound or other wound, within an abscess), or may be systemic. Included is the treatment of bacterial infections that are amenable to therapy by topical application for example using bacteriophage of the invention.

The present disclosure also encompasses coating of surfaces other than body surfaces with the recombinant polynucleotide, delivery vehicle or composition of the present invention, for example wound dressings or medical device surfaces.

Further aspects, features and non-limiting embodiments of the present invention will now be described below with reference to the following drawings:

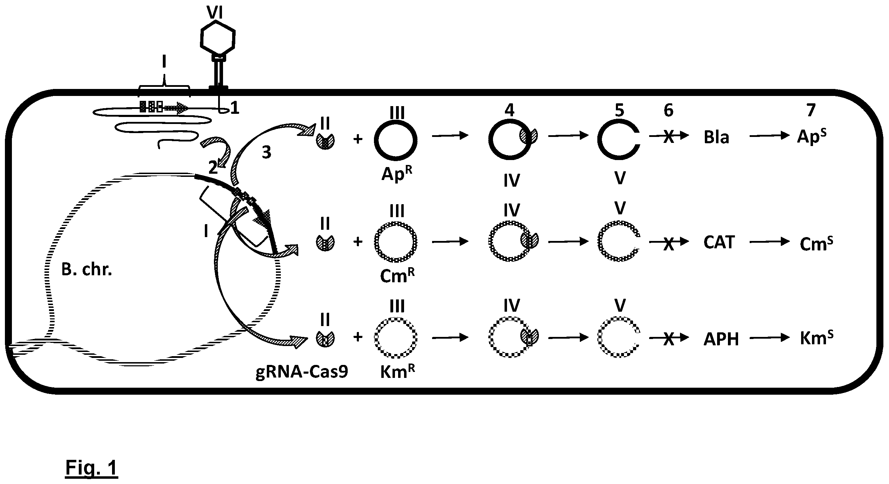

**Figure 1****. CRISPR/Cas9-mediated bacterial immunisation against antibiotic resistant genes on a plasmid: I,** CRISPR/Cas locus, where boxes denote different spacer sequences targeting different antibiotic resistance genes; **II,** gRNA-Cas9 where boxes denote different gRNAs targeting each antibiotic resistance gene; **III,** plasmid harbouring antibiotic resistance gene; **IV,** target recognition and positioning of Cas9; **V,** cleaved plasmid, **VI:** bacteriophage. **Ap^{R}** = ampicillin resistant, **Cm^{R}** = chloramphenicol resistant, **Km^{R}** = kanamycin resistant. **Ap^{S}** = ampicillin sensitive, **Cm^{S}** = chloramphenicol sensitive, **Km^{S} =** kanamycin sensitive. **Bla=** beta-lactamase, **CAT** = chloramphenicol acetyl transferase, **APH** = aminoglycoside phosphotransferase. **1. Injection** - CRISPR/Cas9 is injected into the bacterial cell along with phage DNA by bacteria-specific phage infection. **2. Lysogenisation** - Phage DNA is lysogenised and integrates into the bacterial host chromosome (**B**. **chr**.). **3. crRNA biogenesis and assembly of Cas9** - Pre-crRNA is transcribed and hybridised with tracrRNA and processed to make mature crRNA:tracrRNA (an RNA guide molecule, or "gRNA"), which is assembled with Cas9. **4. Recognition** - These gRNA-Cas9 complexes recognise target DNA on the plasmid. **5. Cleavage** - The gRNA-Cas9 complexes cleave DNA at the sites recognised by crRNAs. **6. Inactivation** - This leads to inactivation of the production of antibiotic resistant enzymes; 7. **Sensitive** - Thus, the bacterial cell becomes sensitive to various antibiotics.

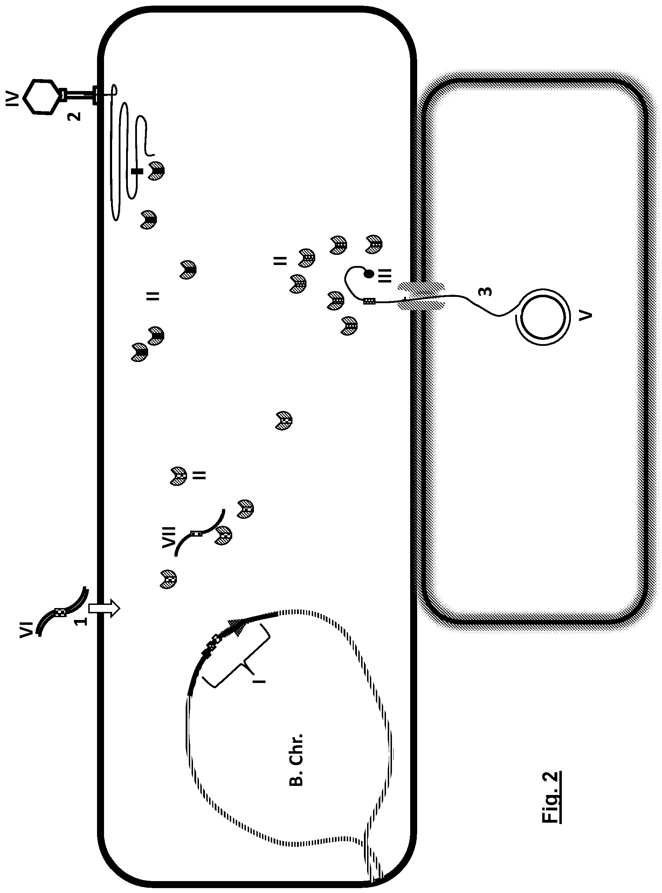

**Figure 2****. Preventing non-pathogenic bacteria and asymptomatic pathogens from a future encounter with antibiotic resistance genes: I,** CRISPR/Cas locus, exemplified here as present in the bacterial chromosome (**B. Chr.**) and where boxes denote different spacer sequences targeting different antibiotic resistance genes; **II,** gRNA-Cas9, where boxes denote different gRNAs targeting each antibiotic resistance gene; **III,** relaxase -an enzyme that makes a strand-specific, sequence-specific nick in double-stranded DNA to initiate conjugal transfer of plasmid DNA; **IV,** bacteriophage, **V,** plasmid; **VI,** double-stranded DNA; **VII,** single-stranded DNA. Three entry pathways to encounter future antibiotic resistance genes are shown. **1. Transformation-** Entry of naked DNA carrying antibiotic resistance gene into the bacterial cell, **2. Bacteriophage-mediated transduction-** Phage carrying antibiotic resistance gene infects a bacterial cell. **3 Conjugation-** Plasmid carrying antibiotic resistance gene is conjugally transferred from a donor bacterial cell to a recipient bacterial cell. When these antibiotic resistance genes are introduced into the "immunised" cell; it is pre-armed with a CRISPR/Cas locus encoding a gRNA-Cas9 that finds the specific target sequence on the antibiotic resistance gene and cleaves it to disrupt the gene.

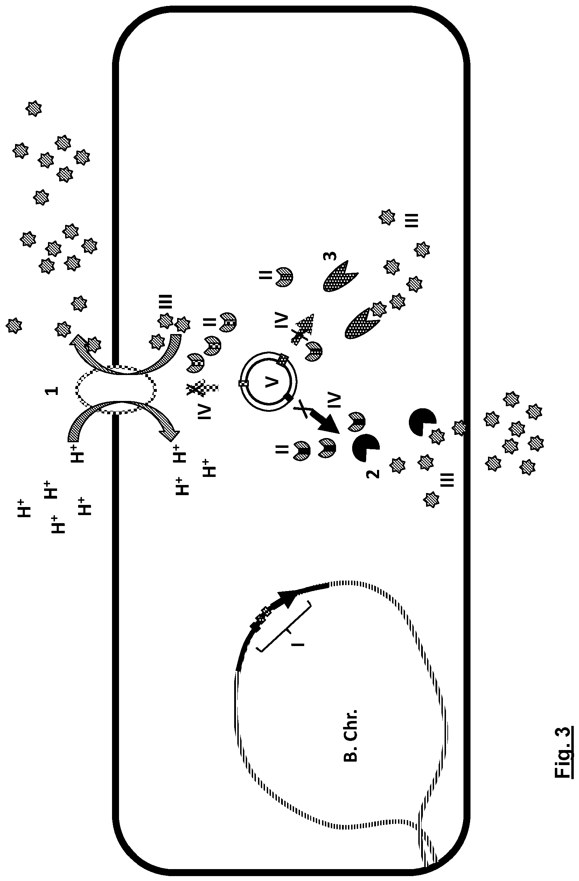

**Figure 3****. Re-sensitising symptomatic pathogens to antibiotics: I,** CRISPR/Cas locus in the bacterial chromosome (**B. Chr.**), where boxes denote different spacer sequences targeting each antibiotic resistance gene; **II,** gRNA-Cas9, where boxes denote different gRNAs targeting each antibiotic resistance gene; **III,** antibiotics; **IV,** protein synthesis is disrupted by cleaving corresponding gene; **V,** plasmid. This figure shows an asymptomatic pathogen becoming symptomatic because of an opportunistic infection. The pathogen contains plasmid DNA that provides various antibiotic resistance agents such as **1. Efflux pumps-** capable of pumping antibiotics out of the bacterial cell, **2 Antibiotic degradation enzymes**-and **3. Antibiotic modification enzymes.** Each antibiotic resistant agent is disrupted (**X**) by gRNA-Cas9-mediated site-specific cleavage of corresponding genes.

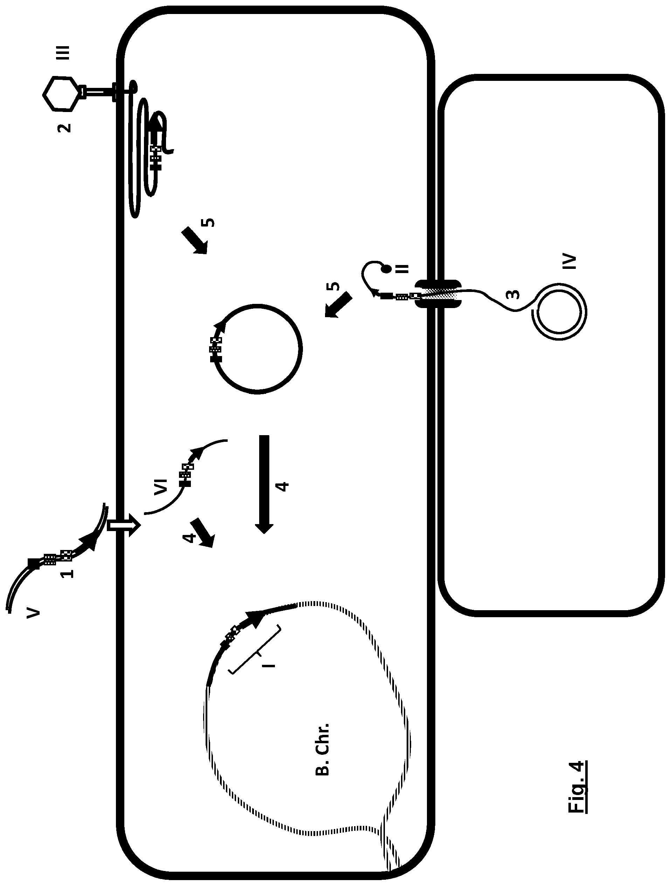

**Figure 4****. Three possible CRISPR/Cas delivery routes: I,** CRISPR/Cas locus where boxes denote different spacer sequences targeting each antibiotic resistance gene; **II,** relaxase -an enzyme that makes a strand-specific, sequence-specific nick in double-stranded DNA to initiate conjugal transfer of plasmid DNA; **III,** bacteriophage; **IV,** plasmid; **V,** double-stranded DNA; **VI,** single-stranded DNA. This figure shows three possible delivery routes of CRISPR/Cas9 expression construct. **1. Transformation-** Entry of naked DNA carrying CRISPR/Cas9 into the bacterial cell via a DNA receptor on the cell surface, followed by integration (**4**) into the bacterial chromosome (**B**. **Chr**.), **2. Bacteriophage-mediated transduction-** Phage carrying CRISPR/Cas9 infects a bacterial cell, followed by circularisation (**5**) and then phage-mediated integration (**4**) into the bacterial chromosome (**B**. **Chr.). 3 Conjugation-** Plasmid carrying CRISPR/Cas9 is conjugally transferred from a donor bacterial cell to a recipient bacterial cell, followed by circularisation (**5**) and plasmid replication. In all cases, boxes in the CRISPR/Cas9 locus denote different spacer sequences targeting each antibiotic resistance gene.

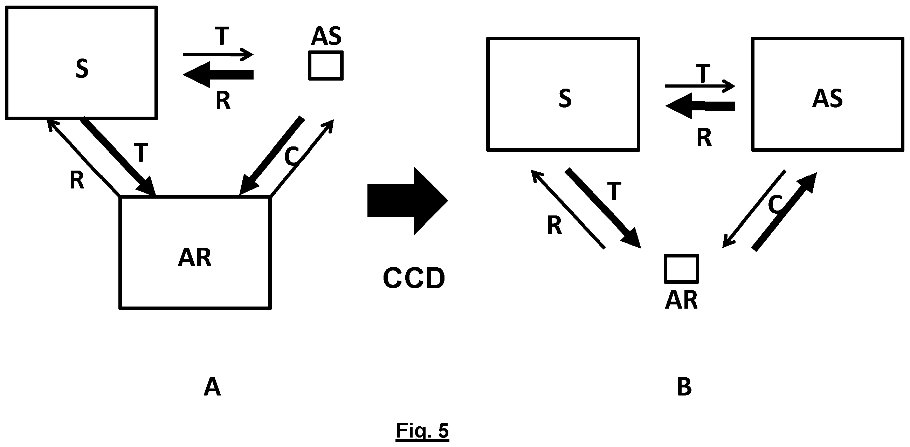

**Figure 5****. Structure of bidirectional antibiotic resistant transmission model (see also** Am J Epidemiol. 2013;178(4):508-520**) and the effect of antibiotic resistance gene inactivation therapy: A**, Antibiotic Resistance is Dominant; B, Antibiotic Sensitivity is Dominant; **S,** Susceptible, **AS,** Antibiotic Sensitive; **AR,** Antibiotic Resistant; **T,** Transmission; **R,** Recovery, **C,** Conversion, **CCD,** CRISPR/Cas9 delivery. Antibiotic resistance genes in an antibiotic resistant state and the encounter of antibiotic resistance genes in a sensitive state are disrupted for example by gRNA-mediated Cas9 cleavage. Antibiotic resistance gene inactivation therapy converts the bidirectional model (A) to an almost unidirectional conversion model (B) by increasing the conversion rate from antibiotic resistant to sensitive and decreasing the conversion rate from antibiotic sensitive to resistant. The area of each square represents the population density of each state. The width of the arrows is proportional to the magnitude of each parameter (transmission, recovery and conversion), i.e. transmission of antibiotic resistant bacteria is assumed to be higher than antibiotic sensitive bacteria in this figure. Recovery from the infection of pathogens sensitive to antibiotics is higher than for antibiotic resistant pathogens. In the presence of antibiotics, antibiotic-sensitive pathogens are converted to antibiotic resistant pathogens much more than the reverse conversion from resistant to sensitive pathogens because of the selection pressure. The treatment aim is to reverse these conversion parameters to drive the antibiotic resistant pathogen population to a sensitive state by introducing CRISPR/Cas9 into the bacteria.

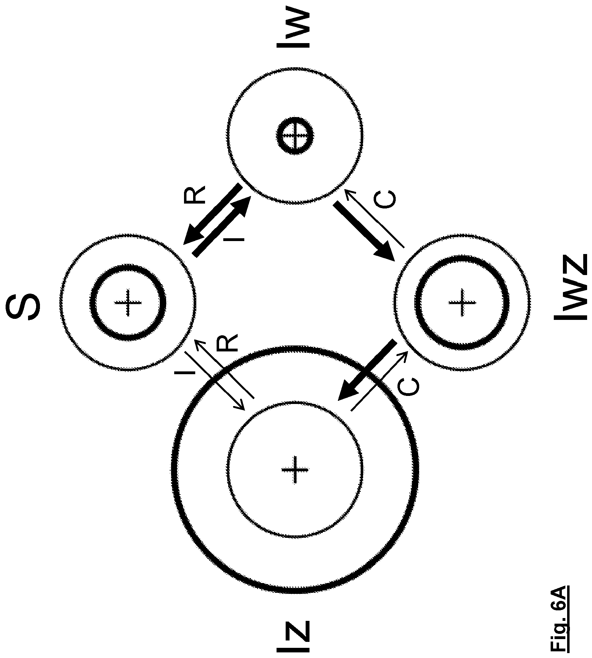

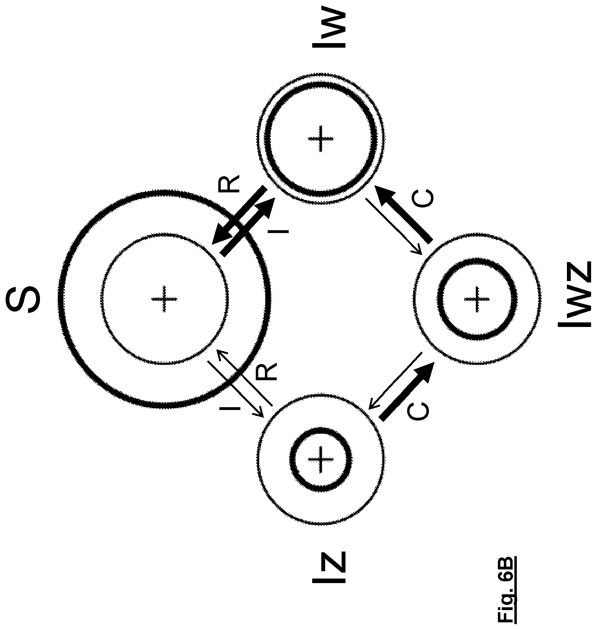

**Figure 6****. Structure of superinfection antibiotic resistant transmission model and the effect of antibiotic resistance gene inactivation therapy:** The bidirectional antibiotic resistance transmission model depicted in Figure 5 can additionally or alternatively be described by the following superinfection antibiotic resistance transmission model (see also Am J Epidemiol. 2013;178(4):508-520, as for Figure 5). When the antibiotic resistance genes are disrupted for example by gRNA-mediated Cas9 cleavage, this allows the population shift from the antibiotic resistant state to the antibiotic sensitive state via the superinfection state. Disruption of antibiotic resistance genes increases the conversion rate from the antibiotic resistant to the sensitive state. This figure gives a comparison of the relative population density before (Figure 6A) and after (Figure 6B) applying the CRISPR-Cas system. The population is composed of **S,** Susceptible; **Iw,** sensitive; **Iz,** resistant; and **Iwz,** superinfection state. The area of each circle is proportional to the relative population density in each state. In this simulation, initial density in each state is assumed to be identical (0.25 each, represented by a thin-lined circle). Thick lined circles represent the population density at the equilibrium state. CRISPR-Cas system is clearly contributing to an increase of the population in the susceptible state, thus the recovery rate in the equilibrium state (i.e. S is expanding in B) and to reduce the population in the resistant state (i.e. Iz is shrinking in B). The width of the arrows is proportional to the magnitude of each parameter (infection, recovery and state conversion), i.e. the infection of antibiotic resistant bacteria is assumed to be lower than that of antibiotic sensitive bacteria in this figure. Recovery from the infection of the antibiotic sensitive pathogens is higher than that from the infection of the antibiotic resistant pathogens, because antibiotics are effective on the sensitive strains. In the presence of antibiotics, the antibiotic sensitive pathogens are converted to the antibiotic resistant pathogens much more than the reverse conversion because of the antibiotic selection pressure. The aim of the treatment is to reverse these conversion parameters and to drive the antibiotic resistant pathogen population to the sensitive state.

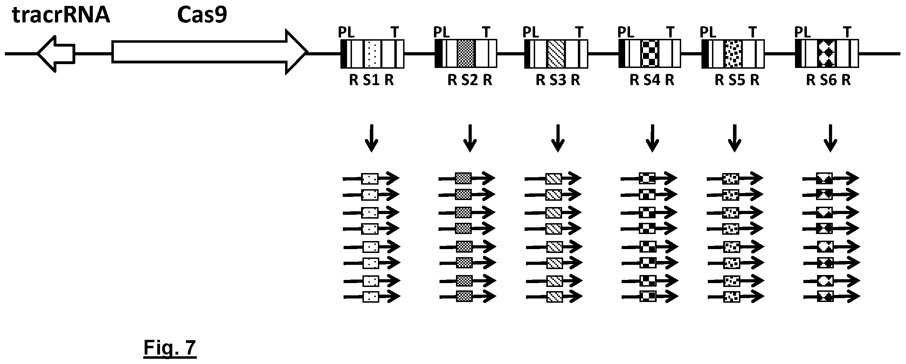

**Figure 7****. Structure of CRISPR locus:** This figure shows CRISPR locus containing six spacers targeting six different regions. Each crRNA is transcribed monocistronically from the same promoters denoted P to control the transcription level identical for each target. Each crRNA transcript starts with a leader sequence L and terminates with a terminator sequence T. Transcripts of each pre-crRNA are shown as arrows and boxes containing different spacer sequences are indicated by unique shading.

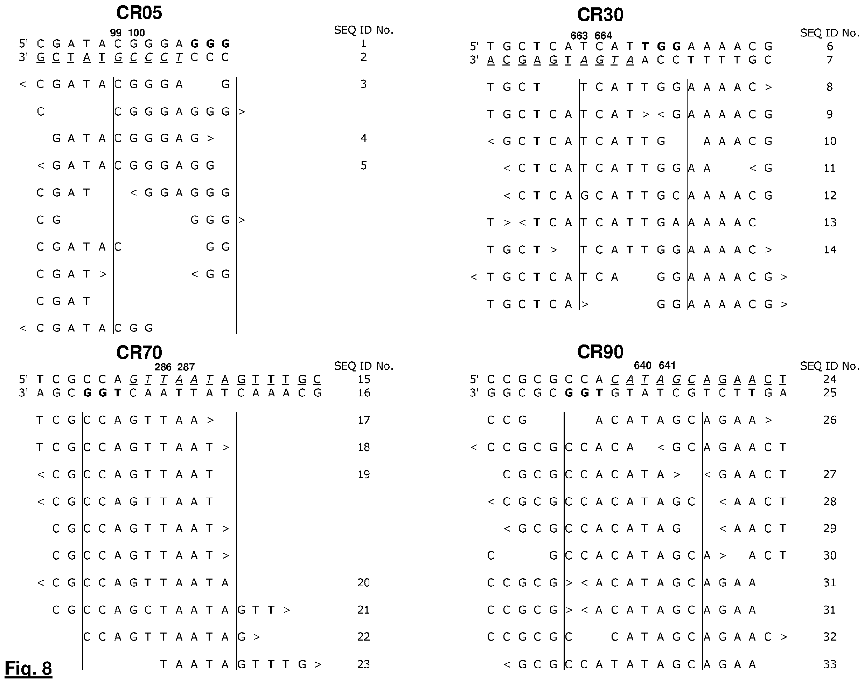

**Figure 8****. Mapping short bacterial off-target sequence on the *bla* gene sequence.** The figure shows the local alignment of bacterial short sequences (SEQ ID NOs: 3-5, 8-14, 17-23 and 26-33) mapped to the beta lactamase gene. Beta lactamase sequence (SEQ ID NOs: 1-2, 6-7, 15-16 and 24-25) is shown in grey in the top of each panel, PAM (protospacer adjacent motif) sequence is shown in black. Base pairing region with crRNA is underlined, off-target seed sequence on the bacterial genome is italicised. Off-target seed sequence, including PAM sequence, is indicated by two vertical lines. Two numbers on the sequence of beta lactamase gene are the expected two base positions where the phosphodiester bond is cleaved by Cas9 (see Example 1 below).

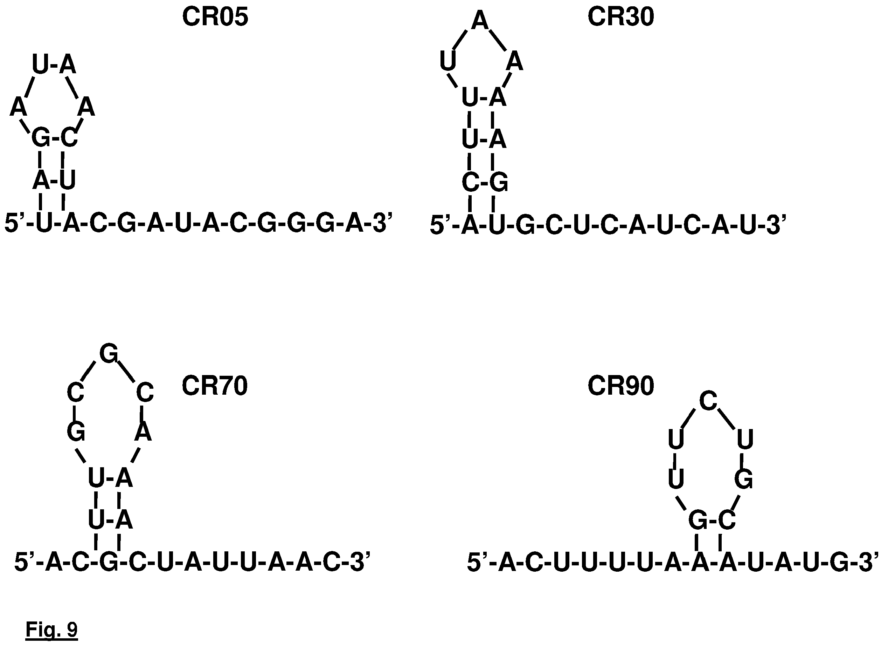

**Figure 9****. Predicted crRNA secondary structure.** With reference to Figure 8 and Example 1, predicted secondary structures of the crRNA sequences using mFold are shown. The sequences of CR05, CR30, CR70 and CR90 shown in Figure 9 correspond to SEQ ID NOs: 34-37, respectively.

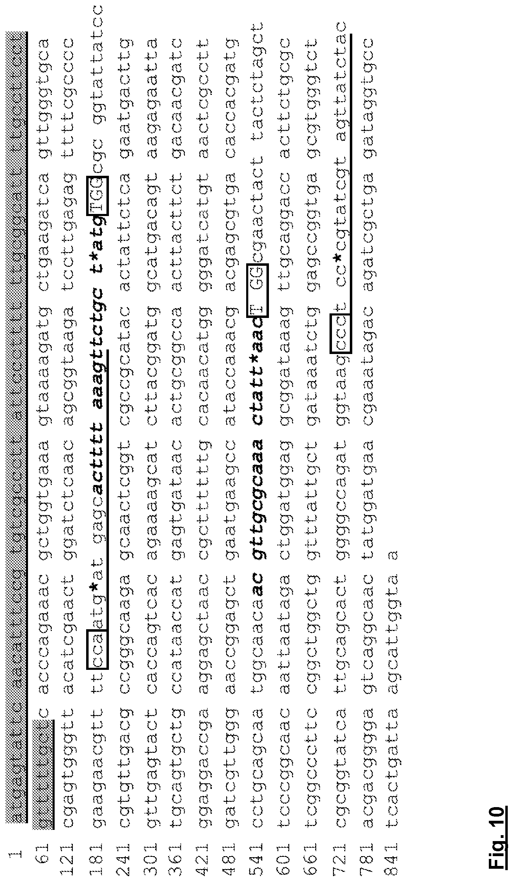

**Figure 10****. Expected cleavage positions on beta-lactamase gene from pBR322 (SEQ ID NO: 38).** Two protospacer sequences, which are base-pairing with crRNA spacer sequences, are underlined (protospacer strand). Two anti-protospacer sequences, which are displaced when protospacer sequences are base pairing with crRNA spacer sequences, are bold italicised (anti-protospacer strand). Associated PAM sequences are indicated either boxed small letters (on protospacer strand) or boxed capital letters (on anti-protospacer strand). Expected cleavage positions are indicated by asterisks. Leader sequence is underlined from 1st to 69th base and highlighted in grey.

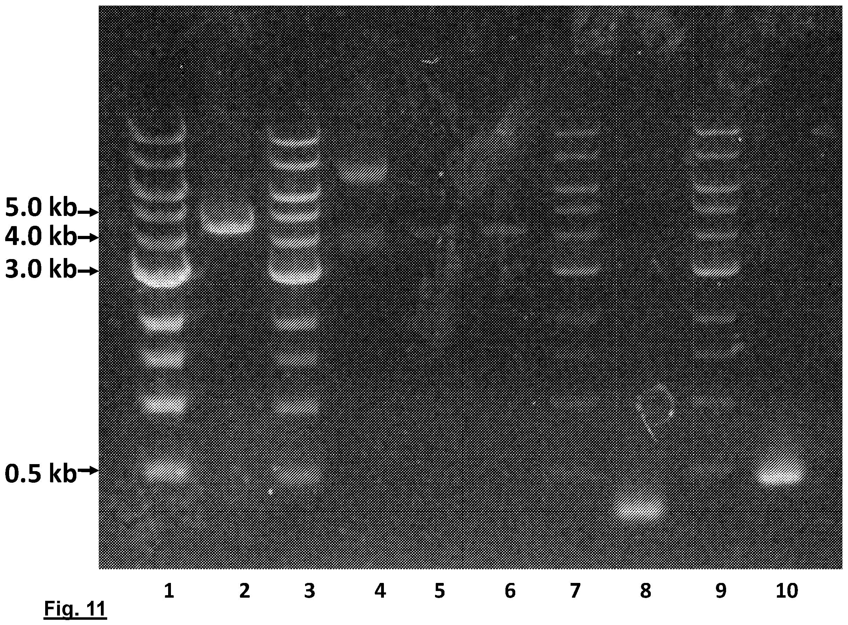

**Figure 11****. Photograph of electrophoretically separated DNA products on a 0.8% agarose gel showing PCR amplicons generated in Example** 2 **from each of the three regions plus the EcoRV digested vector pACYC184.** The marked lanes are as follows: 1*-NEB (N3232S) 1 Kb molecular markers, 2 µg/lane; 2 - Fragment 1, tracrRNA-Cas9 region : 4758 bp; 3* - NEB (N3232S) 1 Kb molecular markers, 0.75 µg/lane; 4 - pACYC184 uncut; 5 - pACYC184 EcoRV cut: 4245 bp; 6 - pACYC184 EcoRV cut: 4245 bp; 7* - NEB (N3232S) 1 Kb molecular markers, 0.12 µg/lane; 8 - Fragment 2, leader and first direct repeat region : 276 bp; 9* - NEB (N3232S) 1 Kb molecular markers, 0.2 µg/lane; and 10 - Fragment 3, Second direct repeat region : 452 bp. Note: * The mass of the each PCR amplicon is estimated by comparing the intensity of the appropriate marker band using NIH ImageJ 1.48v software(http://imagej.nih.gov/ij/).

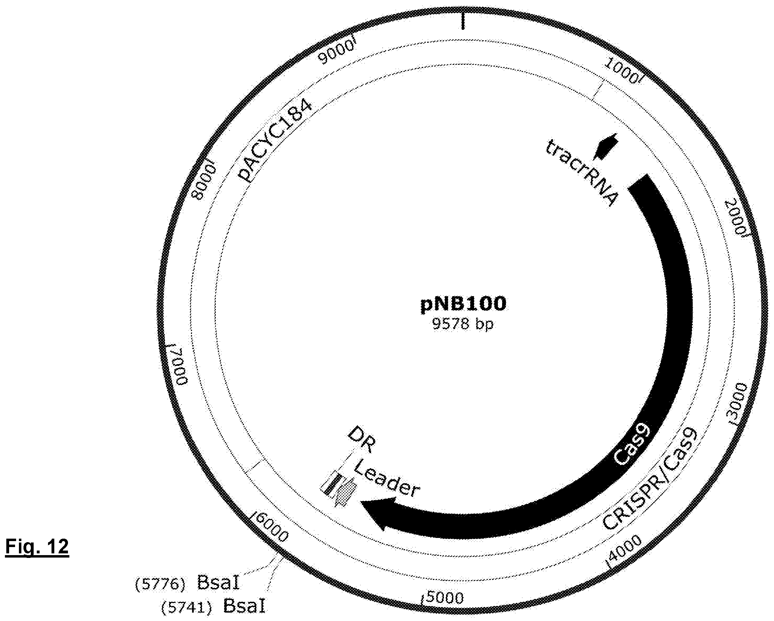

**Figure 12****. Plasmid map of pNB100 constructed in Example 2.** The plasmid map was drawn by SnapGene viewer ver. 2.4.3 free version (http://www.snapgene.com). Two direct repeats (DR) are shown as narrow white rectanglular boxes adjacent to the 3' end of leader sequence.

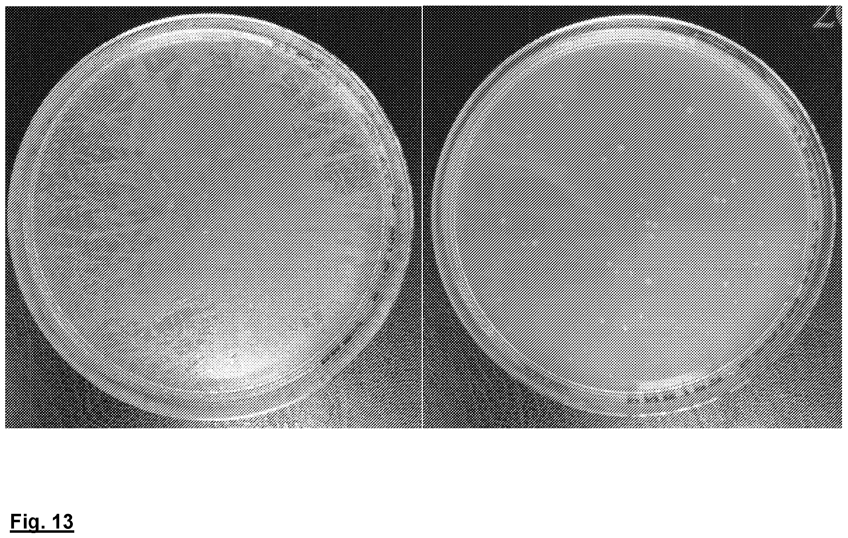

**Figure 13****. Photographs show results of "Nemesis symbiotic activity" (NSA) according to an embodiment of the invention by bacterial cell mating (see Example 2).** The left plate shows JA200 pNT3 x DH5α pNB100 in Ap100Cm35, while the right plate shows JA200 pNT3 x DH5α pNB102 in Ap100Cm35, both plated at 5 x 10⁷ cells /ml.

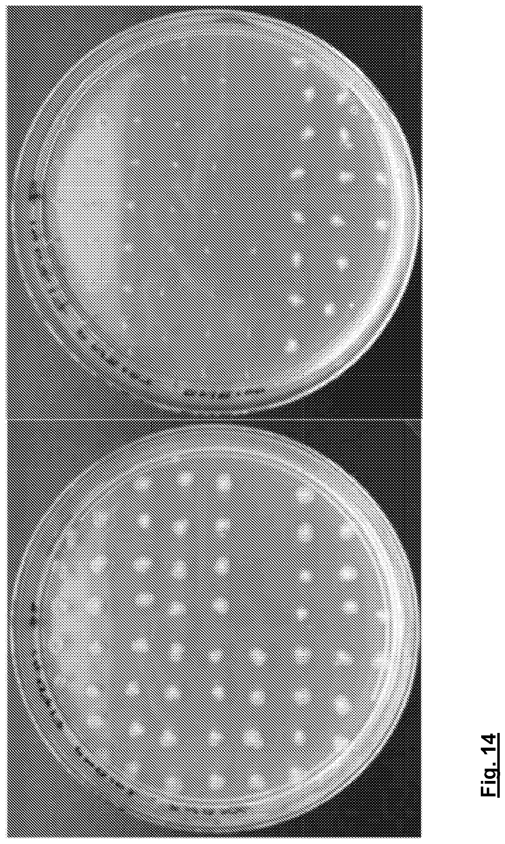

**Figure 14****. Photographs show results of NSA according to another embodiment of the invention by plasmid transformation (see Example 2).** Left: LB Cm35 plate. Colonies 1-40 are DH5α pBR322 transformed with pNB102; Colonies 45-60 are DH5α pBR322 transformed with pNB100. All colonies show resistance to Cm carried on plasmids pNB100 and pNB102; Right: LB Ap100 plate. Note that colonies 1-40 have lost ApR following transformation with pNB102 carrying the spacer region targeted against the beta-lactamase gene carried on the plasmid pBR322 in strain NBEc001, thereby demonstrating Nemesis symbiotic activity. pNB100 lacking this spacer region but carrying the Cas9 gene is unable to inactivate the beta-lactamase gene.

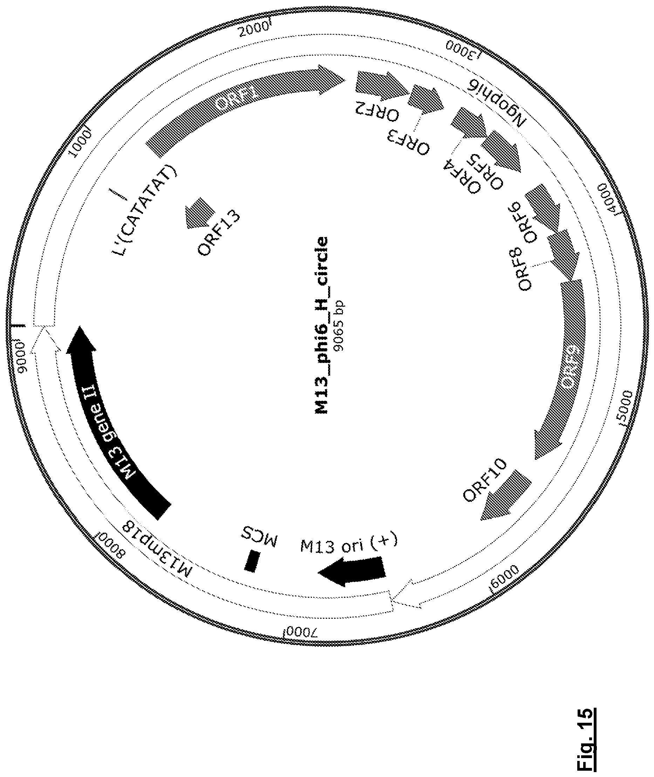

**Figure 15****. Shows a phagemid of Ngophi6.** ORF11 and ORF7 of Ngophi6 are deleted from Ngophi6 genome. Coding sequences are represented by the arrows indicating the translation polarity of each ORF. The corresponding gene nomencrature of each Ngophi6 phage ORF to M13 are ORF1(gll), ORF2 (gV), ORF4 (gVIII), ORFV (gVIII), ORF8 (gVI), ORF9 (gl). M13 gene nomencretures are in the parenthesis. The second T of the putative left integration site L = CTTATAT is changed to A to give L' = CATATAT to avoid integration. MCS = Multiple cloning site. The location of Ngophi6 and M13mp18 are indicated by the large two open arrows.

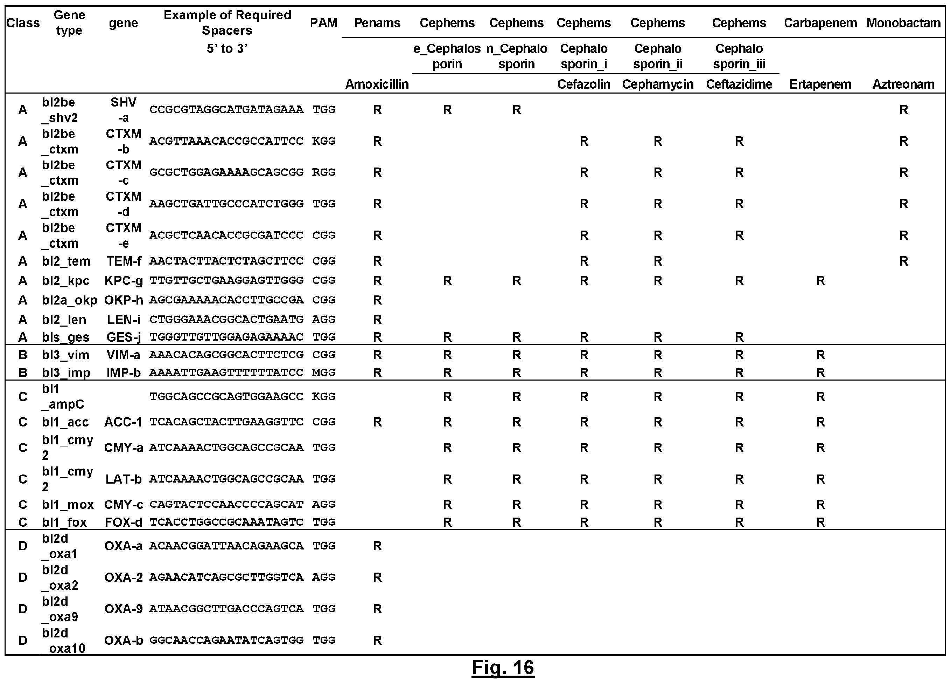

**Figure 16****.** Shows a set of spacer sequences (SEQ ID NOs: 39-60) that encode 20 guide RNA molecules targeted against 117 different *bla* genes identified in the NCBI ARDB database for *Klebsiella pneumoniae* (see Example 5). Candidate spacer sequences were identified to disrupt all *the Klebsiella pneumoniae* beta lactamase genes found in the ARDB database. Beta lactamase gene sequences are collected from the ARDB database with the keyword *Klebsiella pneumoniae.* Redundant sequences were removed and unique sequences used for multiple sequence alignment using web program Clustal Omega. One canonical sequence was chosen from each cluster and the 20 nt spacer sequences predicted by the web program Jack Lin's CRISPR/Cas9 gRNA finder were collected. The spacer sequence is chosen to maximise the ratio of the proto-spacer sequence found in the sequences belonging to the same branch. Thus each of the example spacer sequences shown in the 4^{th} column has the capability to disrupt the genes in the third column. Beta lactamase genes used in this analysis are: SHV-a = 1, 2, 2a, 5, 5a, 11, 12, 14, 26, 27, 28, 31, 33, 38, 43, 44, 48, 55, 56, 60, 61, 62, 71, 73, 74, 75, 76, 77, 78, 79, 80, 81, 82,85, 89, 92, 98, 99, 101, 103, 106, 107, 108, 109, CTXM-b = 1, 3, 10, 12, 15, 22, 32, 54, 60, 62, 68, CTXM-c = 13, 14, 16, 18, 19, 24, 26, CTXM-d = 2, 35, 59, CTXM-e = 26, 63, TEM-f = 1, 1b, 3, ESBL, 139, KPC-g = 1, 2, 3, 4, OKP-h =A11, A12, A16, A17, B13, B-SB613, 6, LEN-i = 2, 17, 18, 19, 20, 21, 22, 24,GES-j = 1, 3, 4, 5, VIM-a = 1, 2, 4, 12, 19, IMP-b = 4, 8, CMY-a= 2, 4, 25, 31, 36, LAT-b = 1, 2, CMY-c = 1, 8b, 10, 19, FOX-d = 1, 5, 7, OXA-a = 1, 30, 47, OXA-2, OXA-9, OXA-b = 10, 17. Beta lactam antibiotics are classified into four classes, penams, cephems, carbapenems and monobactams. One antibiotic name is listed as an example under each class. The beta lactamase, which can open the beta lactam ring is indicated by R. For example, carbapenem is inactivated by KPC. If it is desired to re-sensitise bacteria to carbapenem, the spacer sequence 5'-TTGTTGCTGAAGGAGTTGGG should be employed into spacer array to inactivate KPC genes. Note that the spacer sequence for CMY-a can also be employed for LAT-b cleavage. The example of spacer sequences are shown from 5' to 3' direction.

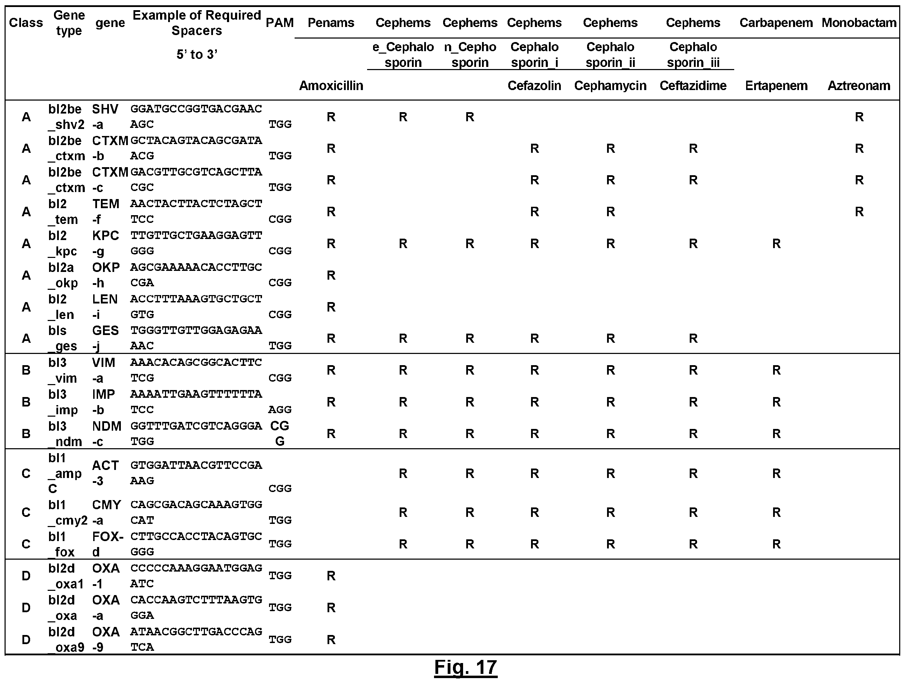

**Figure 17****.** Shows a set of spacer sequences (SEQ ID NOs: 61-77) that encode 17 guide RNA molecules targeted against 154 different *bla* genes identified in the CARD database for *Klebsiella pneumoniae* (see Example 5). Candidate spacer sequences were identified to disrupt all *the Klebsiella pneumoniae* beta lactamase genes found in the CARD database. This table was created with the same method explained in the figure legend in Figure 16. The example of spacer sequences are shown from 5' to 3' direction.

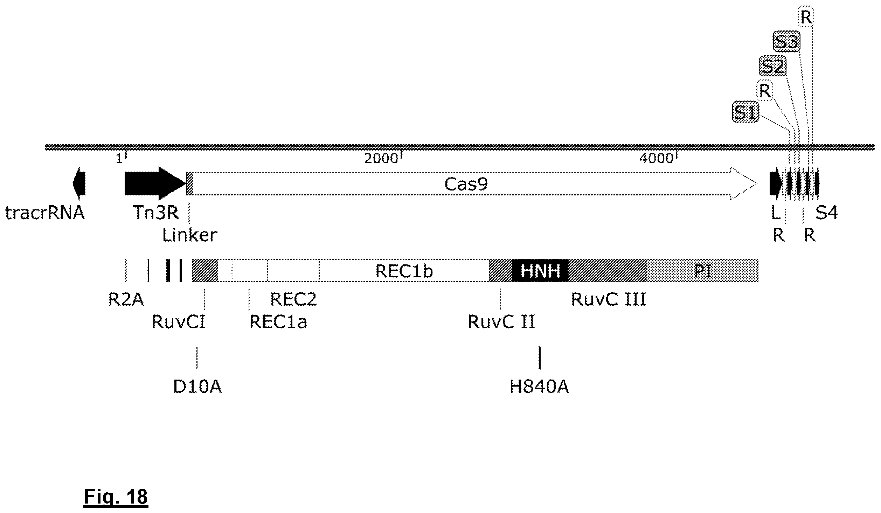

**Figure 18****. Map of a modified Cas DNA-binding polypeptide, Cas9R.** A genetic fusion between Cas9 and Tn3 resolvase. Resolvase and Cas9 are indicated by arrows. The direction of the arrowhead represents the transcription polarity. Functional domain names of Cas9 are shown in the boxes below Cas9 open arrow. This Cas9 is the endonuclease activity deficient mutant dCas9, with amino acid substitutions D10A in RuvCl domain, H840A in HNH domain (as described by Tsai et al. [2014, Nature Biotechnology 32: 569-576]). A mutant Tn3 resolvase (as described by Proudfoot et al. [2011 PLoS ONE 6(4): e19537]) is fused to the N-terminus of this dCas9 via a 12 mer polypeptide linker. Positions of some of these substituted amino acid residues reducing the specificity of the recombination site are indicated by short vertical bars in the N terminal domain, residues 1-148, of the resolvase. The full list of these substitutions is: R2A, E56K, G101S, D102Y, M103I, Q105L, V107F, E132A, L135R. In the Cas9 regions of the fusion: RuvCI, II, III, HNH and PI (PAM interaction) domains are nuclease domains, REC1a and REC1b are recognition domains of repeat and anti-repeat RNA, respectively. REC2 domain does not have any contact to the protospacer-gRNA heteroduplex. Four CRISPR spacer sequences S1, S2, S3 and S4 are arrayed under the expression of one CRISPR leader sequence and are required to bring about the Cas9R-mediated recombination event by the mutant Tn3 resolvase leading to the deletion and re-ligation of the target sequence. Tn3R = Tn3 Resolvase, R = Direct repeat, L = Leader sequence.

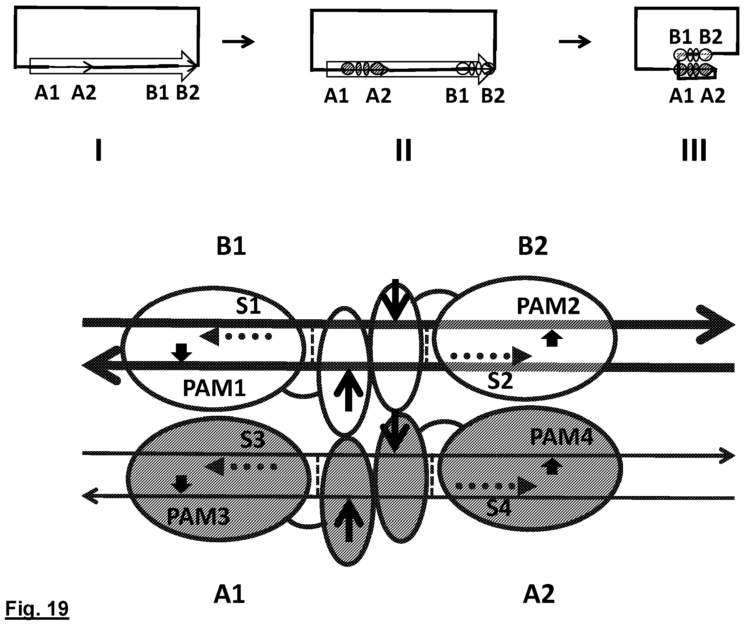

**Figure 19****. Schematic showing site-specific positioning of resolvase by gRNA directed Cas9.** The open arrow in step I is the target antibiotic resistance gene on the plasmid for inactivation. Each recombination site A (A1, A2) and B (B1, B2) are recognised by gRNA independently and correctly positioned resolvases are dimerised in close proximity (step II). Dimers in each recombination site A1+A2 and B1+B2 are tetramerised to form a synapse (step III). The synaptic complex (III) is enlarged, gRNAs are presented as dotted arrows designated S1, S2, S3 and S4. Large ovals represent dCas9, longitudinal ovals are resolvases connected via linker peptides. White and grey longitudinal ovals are resolvase catalytic domains dimerising on the recombination site B and A, respectively. The vertical arrows indicate the cleavage position on the recombination sites by resolvase. The thin horizontal parallel arrows represent DNA containing the recombination site A1+A2 and the thick horizontal parallel arrows represent DNA containing the recombination sites B1+B2. The arrowhead shows the 3' end of the DNA sequence. Short black block arrows are locations of each of the PAM sequences.

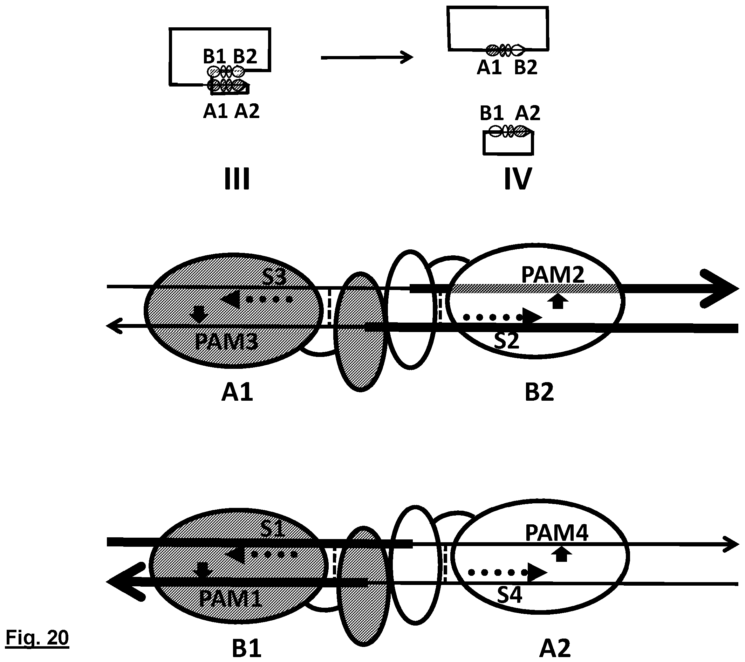

**Figure 20****. Schematic showing exchanging half site of the recombination site A1+A2 and B1+B2 followed by strand resolution and sealing break point.** Half-site of recombination A1 and B1 are exchanged and ligated and resolved. The region of the target antibiotic gene is resolved as a circular DNA, while the rest of the chromosomal or plasmid replicon is re-circularised (step IV). Short black block arrows are locations of each PAM sequences after resolution.

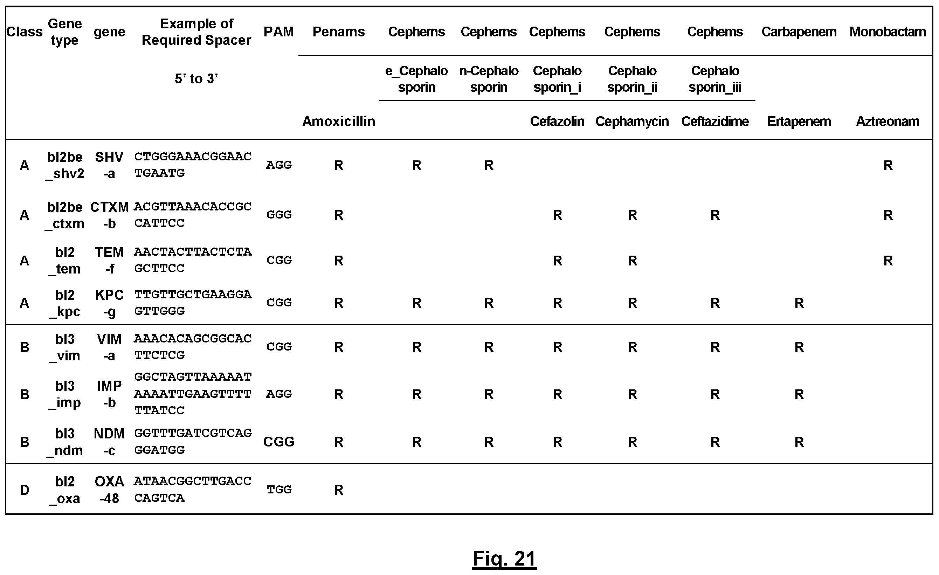

**Figure 21****.** Shows a set of spacer sequences (SEQ ID NOs: 78-85) that encode 8 guide RNA molecules targeted against the class A genes, SHV-a, CTX-M-b, TEM-c, KPC-d; the class B genes VIM-e, IMP-f, NDM-g and the class D gene, OXA-48 where SHV-a = 1, 1a, 2, 2a, 5, 5a, 11, 12, 14, 18, 20, 21, 22, 23, 26, 27, 28, 31, 32, 33, 38, 43, 44, 48, 52, 55, 56, 60, 61, 62, 71, 73, 74, 75, 76, 77, 78, 79, 80, 81, 82, 85, 89, 92, 98, 99, 100, 101, 103, 106, 107, 108, 109, 110, 111, 121, 136, 134, 137, 140, 143, 144, 147, 148, 149, 150, 151, 152, 153, 154, 155, 157, 158, 159, 160, 161, 162, 163, 164, 165, 168, 172, 173, 178, 179; CTXM-b = 1, 3, 10, 12, 15, 19, 22, 32, 52, 54, 59, 60, 62, 68, 71, 80, 81, 99, 141, 147; TEM-c = 1, 1B, 3, 139, 162, 183, 192, 197, 198, 209; KPC-d = 1, 2, 3, 4, 6, 7, 8, 11, 12, 14, 15, 16, 17 ; VIM-e = 1, 2, 4, 19, 26, 27, 33, 34; IMP-f = 4, 8, 32, 38; and NDM-g = 1, 9, 10 (see Example 7).

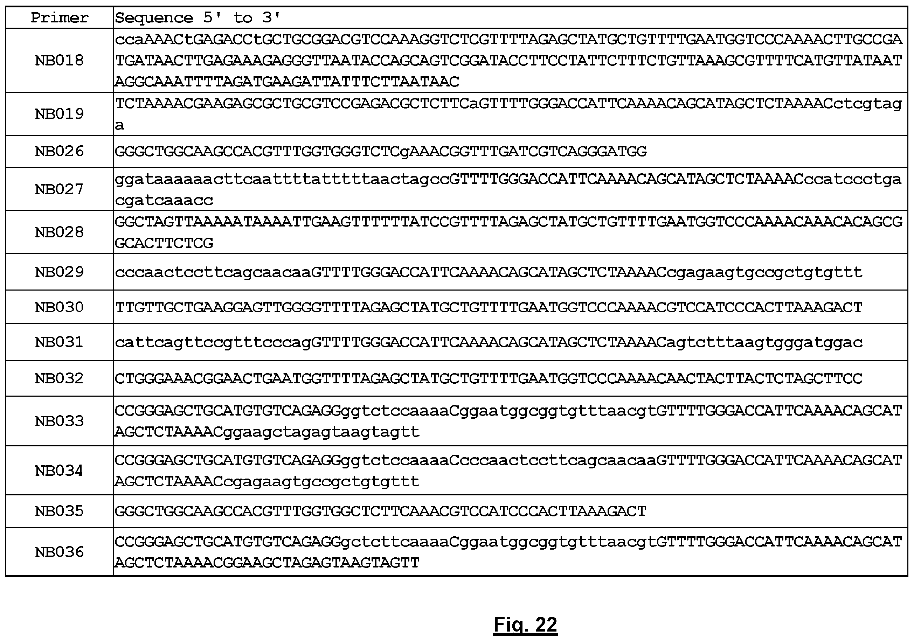

**Figure 22****.** Shows a table giving the sequences (SEQ ID NOs: 86-98) of the oligonucleotides used in the construction of plasmids pNB200, 202, 203, 104A, 104B and 108 (see Example 7).

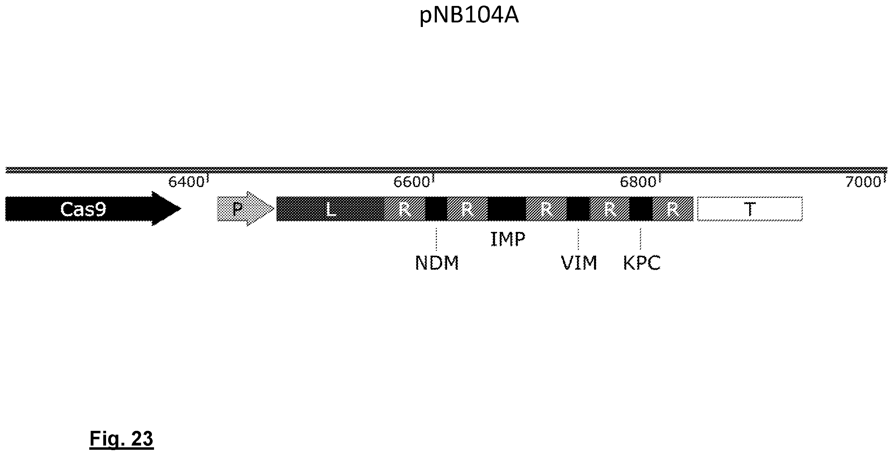

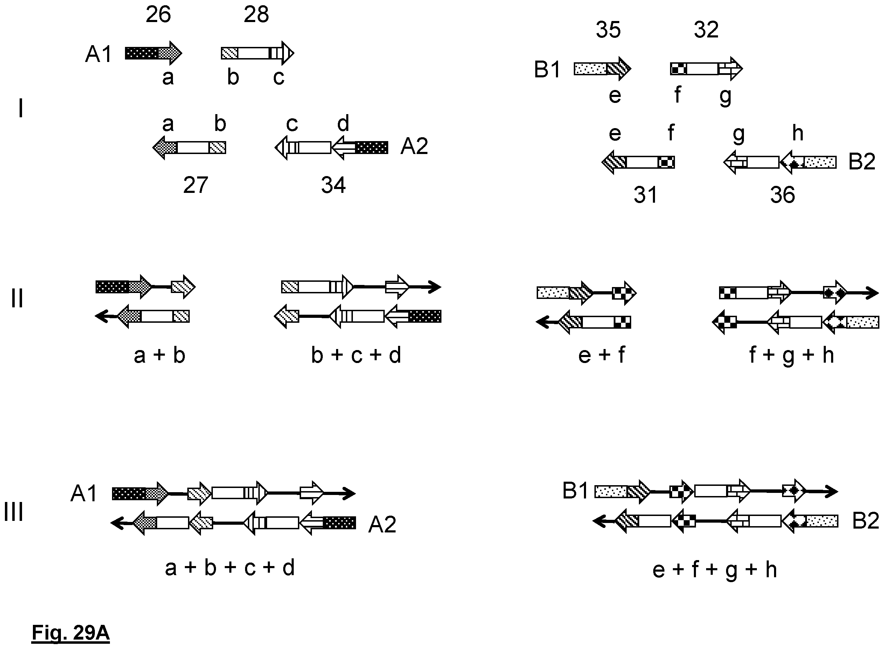

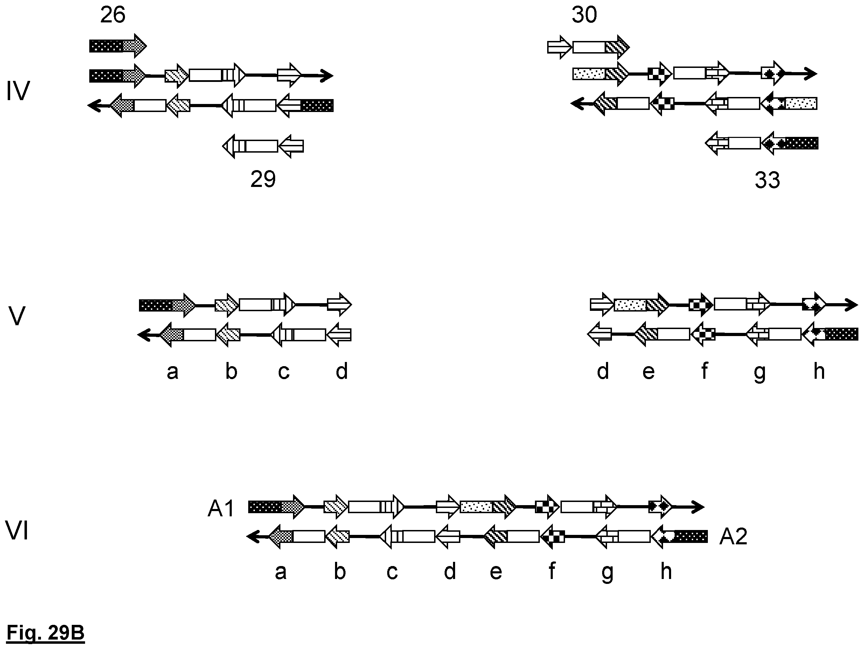

**Figure 23****. Plasmid map of pNB104A constructed in Example 7.** The plasmid map was drawn by SnapGene viewer ver. 2.4.3 free version (http://www.snapgene.com). The tetramer spacer concatemer (Figure 29A) was digested with *Bsa*I, whose restriction site is located in A1 and A2, and ligated to *Bsa*I spacer cloning sites on pNB202 to give pNB203. The single promoter and spacer region (6221-7001) on pNB104A is shown. P = Promoter, L = Leader, R = Direct repeat, S = Spacer, T = Tail. The concatenated spacers (targeted against NDM, IMP, VIM and KPC) are located downstream of the single promoter.

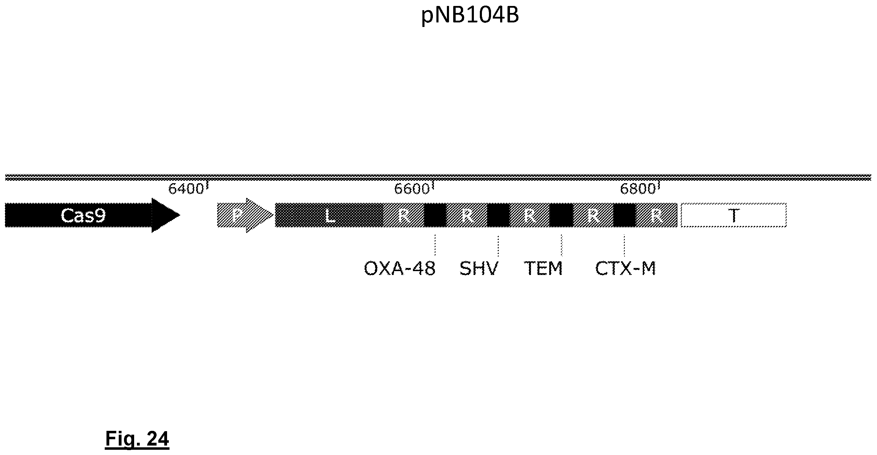

**Figure 24****. Plasmid map of pNB104B constructed in Example 7.** The plasmid map was drawn by SnapGene viewer ver. 2.4.3 free version (http://www.snapgene.com). The single promoter regions (6221-6987) on pNB104B is shown. P = Promoter, L = Leader, R = Direct repeat, S = Spacer, T = Tail. The concatenated spacers (targeted against OXA-48, SHV, TEM and CTX-M) are located under expression from the single promoter.

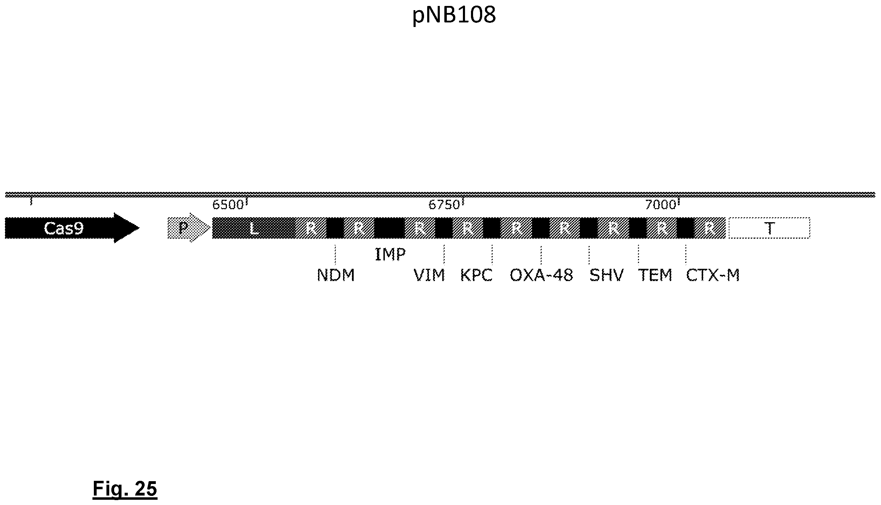

**Figure 25****. Plasmid map of pNB108 constructed in Example 7.** The plasmid map was drawn by SnapGene viewer ver. 2.4.3 free version (http://www.snapgene.com). The octamer spacer concatemer (Figure 29B) was digested with *Bsa*I, whose restriction site is located in A1 and A2, and ligated to *Bsa*I spacer cloning sites on pNB100 to give pNB108. The single promoter and spacer region (6221-7225) on pNB108 is shown. P = Promoter, L = Leader, R = Direct repeat, S = Spacer, T = Tail. The concatenated spacers (targeted against NDM, IMP, VIM, KPC, OXA-48, SHV, TEM and CTX-M) are located under the single promoter.

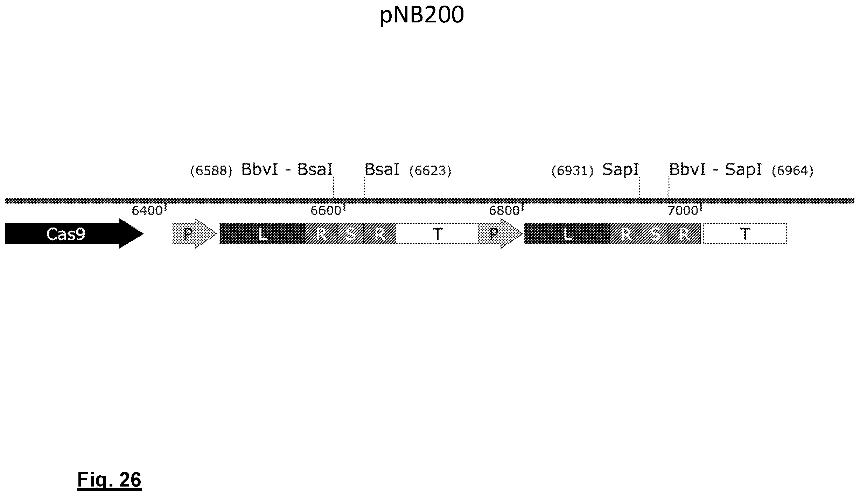

**Figure 26****. Plasmid map of pNB200 constructed in Example 7.** The plasmid map was drawn by SnapGene viewer ver. 2.4.3 free version (http://www.snapgene.com). The dual promoter cassette was synthesised by PCR from the template pNB100 with primer pair NB018 and NB019, the amplicon was digested with *Bbv*I and ligated to the *Bsa*I site of pNB100 to give pNB200, the small *Bsa*I fragment of pNB100, from position 5776-5741 (see Figure 12) is replaced in the process. The dual promoter and two spacer cloning region (6221-7382) on pNB200 is shown. P = Promoter, L = Leader, R = Direct repeat, S = Spacer, T = Tail. The restriction enzymes *Bsa*I and *Sap*I are utilised to clone upstream and downstream spacer sequences, respectively.

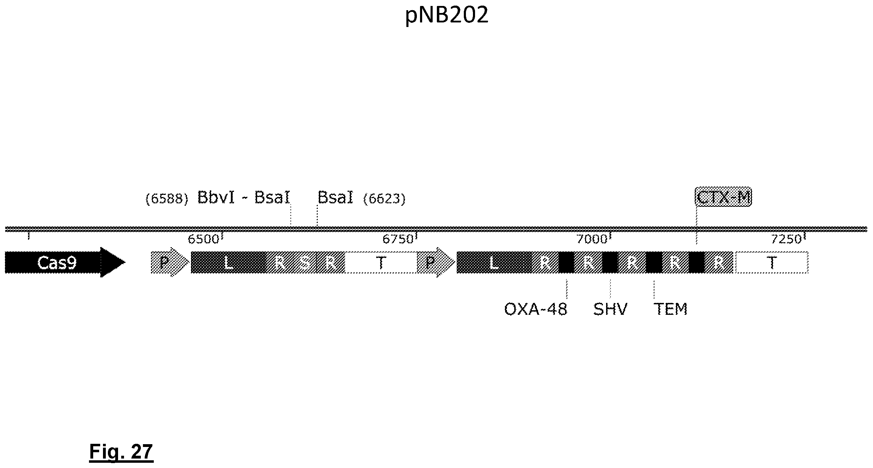

**Figure 27****. Plasmid map of pNB202 constructed in Example 7.** The plasmid map was drawn by SnapGene viewer ver. 2.4.3 free version (http://www.snapgene.com). The tetramer spacer concatemer (Figure 29A) was digested with *Sap*I*,* whose restriction site is located in B1 and B2, and ligated to *Sap*I spacer cloning sites on pNB200 to give pNB202. The dual promoter and spacer regions (6221-7329) on pNB202 is shown. P = Promoter, L = Leader, R = Direct repeat, S = Spacer, T = Tail. The concatenated spacers (targeted against OXA-48, SHV, TEM and CTX-M) are located downstream of the second promoter.

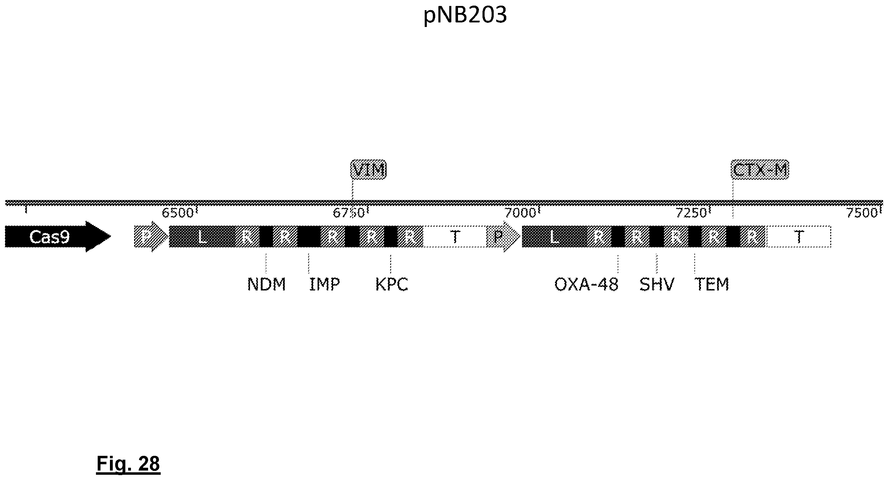

**Figure 28****. Plasmid map of pNB203 constructed in Example 7.** The plasmid map was drawn by SnapGene viewer ver. 2.4.3 free version (http://www.snapgene.com). The tetra spacer concatemer a+b+c+d shown in Figure 29A was digested with *Bsa*I, whose restriction site is located in A1 and A2, and ligated to *Bsa*I spacer cloning sites on pNB202 to give pNB203. The dual promoter and spacer regions (6221-7501) on pNB203 is shown. P = Promoter, L = Leader, R = Direct repeat, S = Spacer, T = Tail. The concatenated spacers (targeted against NDM, IMP, VIM and KPC) are located downstream of the first promoter. The concatenated spacers (targeted against OXA-48, SHV, TEM and CTX-M) are located downstream of the second promoter.
**Figure 29A****. Tetramer spacer concatenation in Example 7.** The numbers associating oligos are corresponding to the primer numbers listed in Figure 22. Oligos are pairewise annealed between 26 and 27, 28 and 34, 35 and 31, 32 and 36 via a, c, e and g unique spacer region (I), respectively and extended in individual tubes (II). Dimer concatemer from 26 and 27 concatenate spacer a and b. Dimer concatemer from 28 and 34 concatenate spacer b, c and d. Dimer concatemer from 35 and 31 concatenate spacer e and f. Dimer concatemer from 32 and 36 concatenate spacer f, g and h (II). Concatenated dimmers a+b and b+c+d, e+f and f+g+h are further hybridised via b and f spacer region, respectively and extended to concatenate four spacers a, b, c and d or e, f, g and h (III).
   The tetramer spacer concatemer e+f+g+h was digested with *Sap*I*,* whose restriction site is located in B1 and B2, and ligated to *Sap*I spacer cloning sites on pNB200 to give pNB202. Tetra spacer concatemer a+b+c+d was digested with *Bsa*I, whose restriction site is located in A1 and A2, and ligated to *Bsa*I spacer cloning sites on pNB202 to give pNB203. a = 20 mer spacer for NDM, b = 20 mer spacer for IMP, c = 20 mer spacer for VIM, d = 20 mer spacer for KPC, e = 20 mer spacer for OXA-48, f = 20 mer spacer for SHV, g = 20 mer spacer for TEM, h = 20 mer spacer for CTX-M.
**Figure 29B****. Octamer spacer concatenation in Example 7.** The tetramer spacer concatemer a+b+c+d and e+f+g+h were amplified with primer pair NB026 and NB029, NB030 and NB033, respectively (V), and hybridise tetra concatemer via spacer d region followed by extension to yield octamer spacer a+b+c+d+e+f+g+h. This octamer was digested with *Bsa*I and ligated to *Bsa*I, whose restriction site is located in A1 and A2, sand ligated to *Bsa*I spacer cloning sites on pNB100 to give pNB108. a = 20 mer spacer for NDM, b = 20 mer spacer for IMP, c = 20 mer spacer for VIM, d = 20 mer spacer for KPC, e = 20 mer spacer for OXA-48, f = 20 mer spacer for SHV, g = 20 mer spacer for TEM, h = 20 mer spacer for CTX-M.

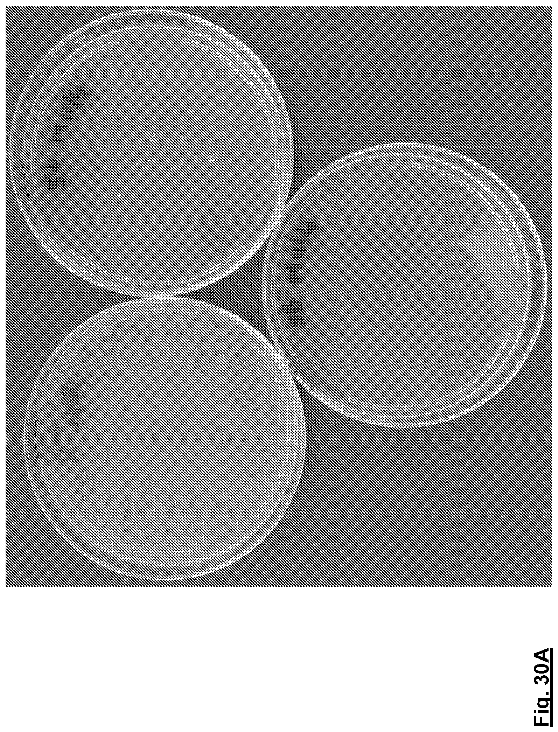

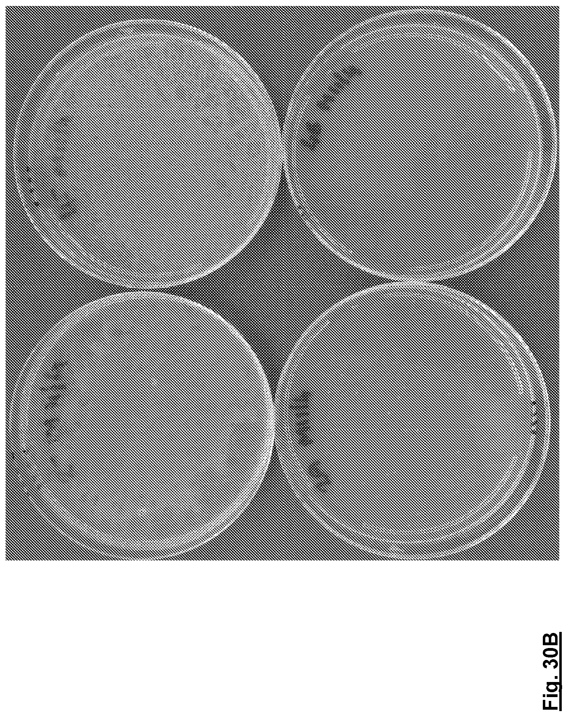

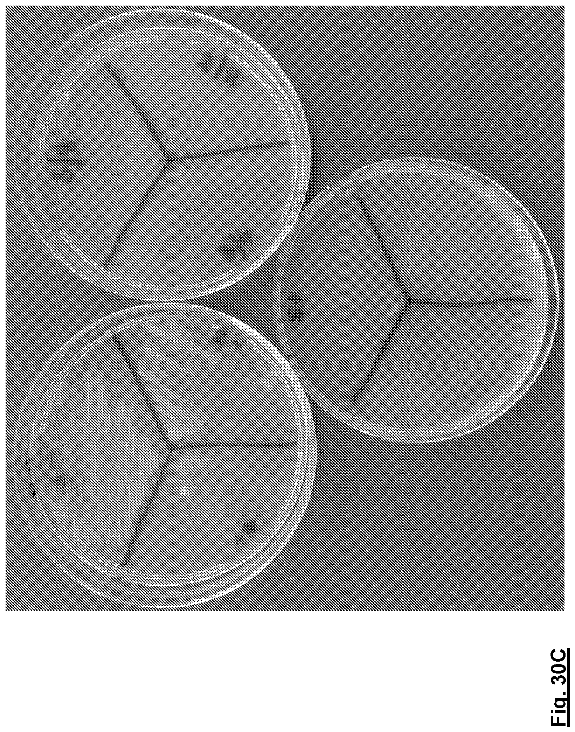

**Figure 30****. Photographs showing results of "Nemesis symbiotic activity" (NSA) according to an embodiment of the invention by bacterial cell mating (see Example 7).** Fig 30A, top left plate shows JA200 pNT3 x DH5α pNB100 in Ap100Cm35, while top right plate shows JA200 pNT3 x DH5α pNB102 in Ap100Cm35. Fig 30B shows NCTC13440 x DH5α pNB100, the top left plate and NCTC13353 x DH5α pNB100, the top right plate; and NCTC13440 x DH5α pNB104A the bottom left plate and NCTC13353 x DH5α pNB104B, the bottom right plate all in Ap100Cm35. Fig 30C shows JA200 pNT3 x DH5α pNB100 (5-), NCTC13440 x DH5α pNB100 (2-) and NCTC13353 x DH5α pNB100 (4-), top left plate; and JA200 pNT3 x DH5α pNB108 (5/8), NCTC13440 x DH5α pNB108 (2/8) and NCTC13353 x DH5α pNB108 (4/8), top right plate and JA200 pNT3 x DH5α pNB100 (5+), bottom plate, all in Ap1 00Cm35.

As used herein, the term "antibiotic" refers to a classical antibiotic that is produced by a microorganism that is antagonistic to the growth of other microorganisms and also encompasses more generally an antimicrobial agent that is capable of killing or inhibiting the growth of a microorganism, including chemically synthesised versions and variants of naturally occurring antibiotics.

The term "sufficiently complementary" means that the sequence identity of the spacer sequence and the target sequence is such that the RNA guide molecule comprising the spacer sequence is able to hybridise, preferably specifically and selectively, with the target sequence, thereby allowing for inactivation of the antibiotic resistance gene comprising the target sequence via the CRISPR/Cas system described herein. For example, the spacer sequence may have at least 60%, 65%, 70%, 75%, 80%, 85%, 90%, 95%, 96%, 97%, 98%, 99% or 100% sequence identity over its entire length with the target sequence.

The term "functional equivalent" as used herein refers to a polypeptide which is capable of the same activity as a Cas DNA-binding polypeptide (or, as used herein, a Cas nucleic acid-binding polypeptide). The "functional equivalent" may have the same qualitative biological property as the Cas DNA-binding polypeptide. "Functional equivalents" include, but are not limited to, fragments or derivatives of a native Cas DNA-binding polypeptide and its fragments, provided that the equivalents have a biological activity in common with a corresponding native sequence polypeptide. Although structural identity is not necessarily required for common biological activity, in one aspect the functional equivalent may have at least 50%, 55%, 60% 65%, 70%, 75%, 80%, 85%, 90%, 95%, 96%, 97%, 98%, 99% or 100% sequence identity over its entire length with a Cas DNA-binding polypeptide, for example Cas9 (Ferretti et al, 2001, PNAS, 98 No. 8: 4658-4663, Gene ID: 901176, Cas9 GI: 15675041; SEQ ID NO: 99).

The term "Cas DNA-binding polypeptide" encompasses a full-length Cas polypeptide, an enzymatically active fragment of a Cas polypeptide, and enzymatically active derivatives of a Cas polypeptide or fragment thereof. Suitable derivatives of a Cas polypeptide or a fragment thereof include but are not limited to mutants, fusions, covalent modifications of a Cas protein or a fragment thereof.

The term "modified" Cas DNA-binding polypeptide encompasses Cas DNA-binding polypeptides as defined above except that the DSB catalytic function of the polypeptide is replaced by a DNA sealing function due for example to the presence of a recombinase catalytic domain. Further features of such modified Cas DNA-binding polypeptides are described herein.

Sequence identity between nucleotide or amino acid sequences can be determined by comparing an alignment of the sequences. When an equivalent position in the compared sequences is occupied by the same base or amino acid, then the molecules are identical at that position. Scoring an alignment as a percentage of identity is a function of the number of identical amino acids or bases at positions shared by the compared sequences. When comparing sequences, optimal alignments may require gaps to be introduced into one or more of the sequences to take into consideration possible insertions and deletions in the sequences. Sequence comparison methods may employ gap penalties so that, for the same number of identical molecules in sequences being compared, a sequence alignment with as few gaps as possible, reflecting higher relatedness between the two compared sequences, will achieve a higher score than one with many gaps. Calculation of maximum percent identity involves the production of an optimal alignment, taking into consideration gap penalties.

Suitable computer programs for carrying out sequence comparisons are widely available in the commercial and public sector. Examples include MatGat (Campanella et al., 2003, BMC Bioinformatics 4: 29; program available from http://bitincka.com/ledion/matgat), Gap (Needleman & Wunsch, 1970, J. Mol. Biol. 48: 443-453), FASTA (Altschul et al., 1990, J. Mol. Biol. 215: 403-410; program available from http://www.ebi.ac.uk/fasta), Clustal W 2.0 and X 2.0 (Larkin et al., 2007, Bioinformatics 23: 2947-2948; program available from http://www.ebi.ac.uk/tools/clustalw2) and EMBOSS Pairwise Alignment Algorithms (Needleman & Wunsch, 1970, supra; Kruskal, 1983, In: Time warps, string edits and macromolecules: the theory and practice of sequence comparison, Sankoff & Kruskal (eds), pp 1-44, Addison Wesley; programs available from http://www.ebi.ac.uk/tools/emboss/align). All programs may be run using default parameters.

For example, sequence comparisons may be undertaken using the "needle" method of the EMBOSS Pairwise Alignment Algorithms, which determines an optimum alignment (including gaps) of two sequences when considered over their entire length and provides a percentage identity score. Default parameters for amino acid sequence comparisons ("Protein Molecule" option) may be Gap Extend penalty: 0.5, Gap Open penalty: 10.0, Matrix: Blosum 62. Default parameters for nucleotide sequence comparisons ("DNA Molecule" option) may be Gap Extend penalty: 0.5, Gap Open penalty: 10.0, Matrix: DNAfull.

In one aspect of the invention, a sequence comparison may be performed over the full length of the reference sequence.

As used herein, the term "gene" refers to a DNA sequence from which a polypeptide is encoded or a non-coding, functional RNA is transcribed.

The term "antibiotic resistance gene" encompasses a gene, or the encoding portion thereof, which encodes a product or transcribes a functional RNA that confers antibiotic resistance. For example, the antibiotic resistance gene may be a gene or the encoding portion thereof which contributes to any of the four resistance mechanisms described above. The antibiotic resistance gene may for example encode (1) an enzyme which degrades an antibiotic, (2) an enzyme which modifies an antibiotic, (3) a pump such as an efflux pump, or (4) a mutated target which suppresses the effect of the antibiotic.

The term "polynucleotide" refers to a polymeric form of nucleotide of any length, for example RNA (such as mRNA) or DNA. The term also includes, particularly for oligonucleotide markers, the known types of modifications, for example, labels which are known in the art, methylation, "caps", substitution of one or more of the naturally occurring nucleotides with an analog, internucleotide modifications, such as, for example, those with unchanged linkages, e.g., methyl phosphates, phosphotriesters, phosphoamidates, carbamates, etc. and with charged linkages.

The term "polypeptide" as used herein refers to a polymer of amino acids. The term does not refer to a specific length of the polymer, so peptides, oligopeptides and proteins are included within the definition of polypeptide. The term "polypeptide" may include post-expression modifications, for example, glycosylations, acetylations, phosphorylations and the like. Included within the definition of "polypeptide" are, for example, polypeptides containing one or more analogues of an amino acid (including, for example, unnatural amino acids), polypeptides with substituted linkages, as well as other modifications known in the art both naturally occurring and non-naturally occurring.

The term "microorganism" encompasses prokaryotes such as bacteria and archaea (for example, those belonging to the the Euryarchaeota and Crenarchaeota). Bacteria include both Gram positive and Gram negative bacteria. Some species of clinically significant, pathogenic fungi are included in the definition of microorganisms, for example members of the genus *Candida, Aspergillus, Cryptococcus, Histoplasma, Pneumocystis* and *Stachybotrys.*

Various aspects of the invention are shown in the figures and described below.

In the present invention, an advantage of using a bacteriophage or conjugative plasmid that comprises the recombinant polynucleotide of the invention is that each serves as a "Trojan horse" that, following infection of the bacteriophage, or following plasmid conjugation, results in the insertion of the "assassin construct" into the target bacteria or other microorganism.

The assassin construct once inserted into the target microorganism also provides an immunisation of the cells against the future arrival of a plasmid harbouring antibiotic resistance genes (see Figure 2), in addition, of course to disrupting such genes already present (see Figures 1 and 3).

The assassin constructs then begin the process of degradation of the antibiotic resistance genes. If a DSB created by the Cas DNA-binding protein of the invention destroys a replicon carrying such an antibiotic resistant gene then a microorganism harbouring the antibiotic resistance gene may be killed directly by an assassin contruct. If the microorganism survives the DSB, the resistance gene will be inactivated, and a patient may then be treated with the antibiotic(s) to which the microorganism has now become sensitised.

Importantly, there should be no or reduced direct selection pressure acting against this event if and until patients are subsequently treated with antibiotics. Thus there should be little or no direct selection for bacteriophage (or plasmid) resistance in the pathogenic bacteria or other microorganisms and therefore no or less establishment of an "evolutionary arms race" - sometimes a significant limiting feature of the known use of bacteriophage directly as bactericidal agents.

In the event that DSB-induced killing of a microorganism increases selection pressure for resistance to a bacteriophage or conjugative plasmid delivery agent, the problem could be mitigated by, for example, using a modified Cas DNA polypeptide as defined herein.

This present invention provides potential agents for oral, topical and probiotic, dietary supplement delivery as well as an epidemiological tool to silently inactivate antibiotic resistance genes in pathogenic bacteria or other microorganisms (see Figure 3). Patients scheduled for surgery, or other treatment in hospital, may well be treated with recombinant bacteriophage carrying CRISPR/Cas9 (or other) assassin constructs targeted against antibiotic resistance genes prophylactically in advance of hospital admission. In this way, pathogens present in their microbiome can be directly killed or purged of antibiotic resistance genes in anticipation of any post-operative infection that might occur requiring treatment with antibiotics.

Thus this present invention provides an epidemiological tool to silently inactivate antibiotic resistance genes in pathogenic bacteria.

To effect exemplification of the invention, a set of CRISPR/Cas9 assassins targeted against selected antibiotics may be constructed. Variables are the bacteriophage (also referred to herein synonymously simply as "phage"), or conjugative plasmid, or DNA, delivery agent: a range of bacteriophage and plasmid agents may be developed that are specific to a range of important bacterial pathogens.

The extent to which a single generalised bacteriophage or plasmid delivery agent needs to be modified to target different bacterial pathogens depends on the details of the specificity of the interactions of either the phage proteins involved in the phage life-cycle, or the plasmid biology and the pathogenic bacterial target.

Both lysogenic phage that infect hosts and become dormant as prophage, as well as non-virulent phage that replicate but do not kill the host, may be developed. With regards to lysogenic phage specificity, only the lysogenic life cycle of the phage and hence the specificity involved in (i) entry of the phage into the bacterial cell and (ii) its subsequent integration into an attachment site in the target chromosome is required. Integration may not be needed and may be replaced by using the phage to deliver a plasmid that can then excise, for example by cre-lox recombination, and replicate independently in the cell. Non-virulent M13 phage and derivatives thereof may be used.

A functional lytic cycle in lysogenic phage may be retained such that low levels of entry into the lytic cycle in lysogenised bacteria will generate new phage that can go on to subsequently infect other bacteria (either pathogenic bacteria or non-pathogenic bacteria, to provide immunity). From the point of view of the epidemiological spread of the phage in the pathogenic population this may not be necessary and a single initial infection may suffice. This can be tested experimentally. Optimal conditions for efficient infection and the appropriate multiplicity of infection are identified.

Experiments may be performed in a model system (see Figure 1). For example, *E. coli* carrying multiple drug resistance, for example, to ampicillin, chloramphenicol, and kanamycin or targeted against commonly used antibiotics against which resistance is widespread.

Experiments may also be performed with a target pathogen, for example, *Klebsiella pneumoniae* carbapenemase ("KPC"). KPC is responsible for infection and death in hospitals in the UK.

**Delivery by bacteriophage:** The phage delivery system (see for example Figure 2, route 2) may be suitable for the treatment of wounds and burns infected by antibiotic-resistant bacteria.

Delivery of the assassin construct targeted against these antibiotics by two different *E.coli* lysogenic bacteriophage: bacteriophage lambda and bacteriophage Mu may be used. Bacteriophage lambda has a specific integration site in the host chromosome known as the lambda attachment site. Bacteriophage Mu is able to integrate randomly into the host genome and has the advantage that no specific attachment sites in bacteria are required.

Phage may also be used to deliver a plasmid replicon containing the recombinant polynucleotide of the invention that excises by cre-lox recombination following infection as discussed above or by use of phage P1 that replicates as an episome. The specificity involved in successful infection is recognition of a membrane protein on the bacterial cell surface. In the case of lambda this is the maltose permease protein that transports the sugar maltose into the bacterial cell. In the case of bacteriophage Mu the receptor is LPS.

Male-specific phage M13 that infect *E.coli* cells carrying the F-factor plasmid may also be used to deliver CRISPR/Cas9 constructs (or other assassin constructs of the invention) targeted against one or more antibiotic resistance genes. M13 is a well studied phage, which replicates, but does not kill the bacterial host.

Successful elimination of resistance to target antibiotics following the lyogenisation by recombinant bacteriophage, or infection by M13 recombinants carrying the assassin construct directed against these antibiotic resistance genes is evaluated.

Studies on other closely related Enterobacteriaceae carrying resistance to the same antibiotics may be performed. These may include *Salmonella typhimurium,* and *Shigella flexneri* in addition to *Klebsiella pneumoniae* discussed above.

In order to do this, modified phage are constructed with different genes encoding the tail fibre protein of the bacteriophage in order to allow it to interact with a different receptor present on the bacterial surface. Lysogenic phage, or male-specific phage, like M13, that are natural hosts of these bacteria may be used.

The phenomenon of phase variation whereby bacteriophage are able to switch their host specificity by the inversion, at a low frequency, of a DNA segment to control gene expression and express alternative tail fibre proteins may also be exploited. And in that way develop phage delivery systems that are versatile. For example phage Mu carries an invertible G segment (regulated by a Mu-encoded site-specific recombinase, Gin) giving rise to two phage types G(+) able to infect *E.coli* K12 and G(-) able to infect *Enterobacter cloacae, Citrobacter freundii Serratia marcescens* and *Erwinia carotovora.*

Another example is the *Neisseria gonorrhoeae* filamentous phage NgoPhi6, or modified forms thereof. The natural (wild type) phage has a wide host range for Gram negative bacteria (alpha, beta and gamma proteobacteria) - see Piekarowicz et al. (2014 J. Virol. 88: 1002 - 1010). The natural phage is not lytic but lysogenic and has an integration site on the host genome. In one aspect of the invention, the ability of the phage to integrate into the host chromosome is removed and the phage is engineered to replicate independently. The substitution of the left integration site L, CTTATAT with L', CATATAT will eliminate the integration event. In order to replicate the phage genome extra-chromosomally, the M13 ori and M13 gene II can be used to mimic the M13 phage replication. The modification may be kept to a minimum to maintain the ability of the progeny production from the infected bacteria. Although the maximum packaging size is unknown, a phage DNA of around 12 Kb is experimentally demonstrated as packageable and phage progeny produced from the bacteria infected with this phage are infectable. The length of the phage is around 4 microns, which is 4.4 times longer than M13 phage particle, and indicates the higher packaging capacity of DNA longer than M13 - i.e. assuming that the packaging size of DNA is proportional to the size of the phage particle length, DNA packaging capacity of the NgoPhi6 phage will 4 microns/0.9 microns x 6407 nt = 28475 nt, large enough for packaging phage genome along with a recombinant polynucleotide of the invention, including for example a CRISPR-Cas9 construct if required. The exemplified structure of the phage vector (phagemid) meets the requirements - see Figure 15.

**Delivery by conjugative plasmids:** Delivery of the assassin constructs targeted against these antibiotics by selected broad-host range conjugative plasmids may be evaluated (see Figure 4, route 3). Here, the plasmids may be delivered by a benign non-pathogenic host. The application here may be for the Gl tract as a probiotic and may be used prophylactically. In this aspect, the possible lethal effects of DSBs caused by Cas DNA-binding protein are not a concern since the plasmid will not be transferred to the recipient.

Similarly demonstration of the successful elimination of resistance to the three target antibiotics following conjugal transfer of plasmids carrying the assassin cargo may be evaluated.

**Delivery by transformation of linear DNA:** Delivery of the assassin construct targeted against these antibiotics by linear double-stranded DNA transformation (see Figure 4, route 1) may also be performed. Here, the DNA is delivered via DNA receptor on the surface of the bacteria from the bacterial surrounding environment.

### Examples

The present invention is illustrated by the following non-limiting examples.

### Example 1

The aim of Example 1 is to demonstrate a proof-of-concept for resurrection of antibiotic efficacy by introduction of a CRISPR/Cas9 construct in non-pathogenic bacterial strains of *Escherichia coli.*

A CRlSPR/Cas9 construct is made directly from genomic DNA isolated from the *Streptococcus pyogenes* strain SF370, which is obtained from: http://www,straininfo.net/strains/1178GG/browser;jsessionid=A07A638D6D2472EA2FEDBD3 A1928F347.straininfo2. Here the Cas9 coding region plus adjacent regulatory regions and DNA encoding the tracrRNA is extracted by PCR, using sequence-specific DNA primers and cloned into a suitable plasmid cloning vector. A unit repeat of CRISPR array comprising the direct repeats flanking a spacer sequence is similarly extracted by PCR and modified to replace the spacer sequence by a cloning site to allow the subsequent introduction of spacer sequences designed to target DNA regions of choice such as antibiotic resistance genes. It is useful to have a positive selection for bacterial transformants carrying the desired recombinants in which such a spacer sequence has been successfully cloned into the cloning site. Appendix 3 gives an example of such a positive selection.

Alternatively an equivalent CRISPR/Cas9 gene targeting construct, though lacking a positive selection for recombinants, is obtained from a pCas9 plasmid such as for example available from Addgene: http://www.addgene.org/42876/. This plasmid carries the Cas9 gene plus a DNA sequences encoding tracrRNA and CRISPR array with a unique cloning site in order to introduce the spacer sequence desired to target a given DNA sequence for cleavage by the Cas9 endonuclease. For exemplification purposes, use of this existing pCas9 plasmid is described below.

Example 1 shows that bacteria carrying a beta-lactamase (bla) gene conferring resistance to the beta-lactam antibiotic, ampicillin become sensitive to ampicillin following introduction of a modified CRISPR/Cas9 construct targeted against the *bla* gene: the CRISPR/Cas9/anti-*bla* construct.

### Materials and Methods

### A. Bacterial strains, plasmids and phage

### 1. Bacterial strains:

DH5α is (F- endA1 glnV44 thi-1 recA1 relA1 gyrA96 deoR nupG Φ80d*lac*ZΔM15 Δ(*lacZYA-argF*)U169, hsdR17(r_{K}⁻ m_{K}⁺),λ-).
JM109 is (endA1 glnV44 thi-1 relA1 gyrA96 recA1 mcrB⁺ Δ(lac-proAB) e14- [F' traD36 proAB⁺ lacl^{q} lacZΔM15]hsdR17(r_{K}⁻m_{K}⁺)).

### 2. Plasmids:

pUC18 (*Ori* pMB1); pCas9 (pACYC184-based vector with *Ori* p15A, CRISPR locus plus Cas9 gene, CM^{R}); pCRISPR (*Ori* pMB1, CRISPR, Kn^{R}).

### 3. Phage: M13mp18

In Example 1A, in one strain *E.coli* DH5α and carrying pBR322 a medium copy plasmid or alternatively a low copy plasmid, the CRISPR/Cas9/anti-*bla* construct is delivered, by naked DNA transformation in plasmid pCas9, designated pCas9::anti-*bla*. The already present plasmid pBR322, or the low copy plasmid expresses the beta-lactamase derived from bacterial transposon Tn3; pBR322 also carries resistance to tetracycline. As pCas9::anti-*bla* carries resistance to chloramphenicol, selection for cells maintaining pCas9::anti-*bla* is achieved by addition of chloramphenicol to the growth medium. In a separate negative control experiment DH5α cells with pBR332, or a low copy plasmid are transformed with pCas9 (that is a plasmid in all respects like pCas9::anti-*bla* but lacking anti-*bla* which is the spacer sequence targeted against the *bla* gene: it is predicted that pCas9, in lacking the anti-*bla* would not be able to attack and inactivate the *bla* gene. Again pCas9 is maintained by the presence of chloramphenicol.

Beta-lactamase activity can be detected by nitrocefin, which is a chromogenic derivative of cephalosporin, when the beta-lactam ring is hydrolysed, ultraviolet absorption of intact nitrocefin is shifted to around 500 nm, which allows visual detection of the presence of beta lactamase.

Let the total number of bacteria resistant to chloramphenicol be N₀ and the number of beta-lactamase resistant bacteria be Nᵣ. Growth of Nᵣ manifest as red colonies in the presence of nitrocefin. Let the number of beta lactamase sensitive bacteria be N_{w}. Growth of N_{w} colonies manifest as white colonies in the presence of nitrocefin. Thus the efficacy of CRISPR/Cas9/anti-*bla-*mediated inactivation of the *bla*gene efficiency is calculated by measuring the fraction of white colonies, i.e.Nw/N0=(N0-Nr)/N0.

Alternatively beta lactamase activity is seen directly challenging the bacteria on the LB plate containing ampicillin. Total bacteria resistant to chloramphenicol is N₀, ampicillin resistant bacteria is Nₐ, then CRISPR/Cas9/anti-*bla*-mediated inactivation of the *bla* gene efficiency can be defined by measuring the fraction of the ampicillin sensitive colony,1-Na/N0.

In Example 1B, an M13 phage delivery system is used to introduce CRISPR/Cas9/anti-*bla* construct into the *E coli* strain, JM109, expressing beta lactamase gene from a resident plasmid.

M13::CRISPR/Cas9/anti-*bla* phage recombinant is prepared as follows: the CRISPR/Cas9/anti-*bla* construct is isolated from pCas9::anti-*bla* by digesting this construct with *Sal*I and *Xba*I*.* The digested fragment size is 5796 bp. The fragment is cloned at *Sal*I (GTCGAC) and *Xba*I (TCTAGA) site of M13mp18 RF I. The entire size of the M13mp18 containing CRISPR/Cas9/ anti-*bla* construct is 13035 bp. This M13 recombinant phage DNA is transformed to *E.coli* JM109, a bacterial strain carrying the F' plasmid and recombinant M13 phage extruded from the bacteria is purified and used to introduce the CRISPR/Cas9 construct to *E.coli* JM109 harbouring pBR322. As a negative control, an equivalent M13::CRISPR/Cas9 phage recombinant lacking anti-*bl*a region is prepared from pCas9 by restriction enzyme *Sal*I and *Xba*I and the amplicon is cloned at *Sal*I and *Xba*I site of M13mp18 RF I.

When M13 infects the bacterial cells, it does not kill them, but the phage DNA replicates inside the cell and expresses phage genes. Thus M13 phage infection with an M13::CRISPR/Cas9/anti-*bla* phage recombinant should result in inactivation of the *bla* gene, in contrast, in a negative control, to infection by an M13::CRISPR/Cas9 phage recombinant lacking anti-*bla* region. Because M13 phage infection slows down the rate of cell growth, when infected cells grown on a lawn of cells yield turbid plaques of slow growing infected cells. If nitrocefin is added to the growth medium, the efficacy of CRISPR/Cas9/anti-*bla-*mediated inactivation of the *bla* gene efficiency is calculated by measuring the fraction of white colonies to red red colonies, when plaques are picked and grown as colonies to remove the background lawn. Plaques may also be picked and plated onto LB plate with/without ampicillin to score the ratio of ampicillin sensitive to ampicillin resistant colonies that will result.

### B. Selection of spacer sequence from the target sequence

We can use Guide RNA Target Design Tool (see; https://wwws.blueheronbio.com/external/tools/gRNASrc.jsp) from BlueHeron to select spacer sequence from the target. This program simply returns the 20 nt spacer sequence with the appropriate PAM (protospacer adjacent motif) sequence in the 3' end and GC content. It does not consider secondary structure stability and sequence specificity. Secondary structure prediction and specificity search is performed manually.

We choose the actual spacer sequence from the candidate sequences obtained in the above program, which should meet the following two criteria: 1) low tendency to form stable secondary structure of crRNA, 2) no target DNA on the host genomic DNA. It may be very difficult to find a unique sequence to satisfy criterion No.2. Considering mismatched target data from Figure 3 E in Jinek et al Science 337, 816 (2012), criterion No.2 is relaxed to allow a matched sequence up to the 12th nucleotide position in the target sequence (the first nucleotide position is counted from just next to the PAM sequence). In other words, when the first 12 mer protospacer sequence of the target sequence is completely matched to the 12 mer sequence of crRNA spacer sequence in the 3' end, but the rest of the sequence is not matched, it is assumed that target dsDNA is not cleaved. The specificity check of the protospacer sequence along the *E.coli* K12 genome sequence is performed by BLAST. The bla sequence is searched against the subject sequence *Escherichia coli str.* substr. MG1655 (http://www.ncbi.nlm.nih.gov/nucleotide/556503834?report=genbank&log$=nuclalign&blast_r ank=1&RID=JUYB76FX014), and each of any matched chromosomal sequence is mapped against the *bla* sequence for counting the seed sequence from the canonical PAM (NGG) sequence. Secondary structures can be predicted by mFold. (http://mfold.rna.albany.edu/?q=mfold/RNA-Folding-Form) to choose the sequence whose G is large as possible, preferably to be positive for crRNA spacer secondary structure. The following is the way to select the appropriate spacer sequences from the *bla* sequence.

**Table 1. Candidate anti-protospacer sequence.**

| Seq | Location | anti-protospacer seq | SEQ ID NO | MG1655 | pCas9 | pBR322 | PAM | GC | ΔG |
|---|---|---|---|---|---|---|---|---|---|
| | | | | Off targ | Off targ | Off targ | | | |
| 05 | 83-102(F) | TAGATAACTACGATACGGGA | 100 | CGGGA | None | None | GGG | 0.48 | -0.40 |
| 05 | 83-102(F) | TAGATAACTACGATACGGGA | 100 | 5 | | | GGG | 0.48 | +0.50 |
| 22 | 442-461(F) | GATCGTTGTCAGAAGTAAGT | 101 | AGTAAGT | AGTAAGT | None | TGG | 0.43 | -0.80 |
| 22 | 442-461(F) | GATCGTTGTCAGAAGTAAGT | 101 | 7 | 7 | | TGG | 0.43 | -0.50 |
| 30 | 647-666(F) | ACTTTAAAAGTGCTCATCAT | 102 | TCAT | ATCAT | None | TGG | 0.35 | -1.30 |
| 30 | 647-666(F) | ACTTTAAAAGTGCTCATCAT | 102 | 4 | 5 | | TGG | 0.35 | -1.30 |
| 35 | 767-786 (F) | TTTACTTTCACCAGCGTTTC | 103 | GCGTTTC | TTTC | None | TGG | 0.43 | +1.30 |
| 35 | 767-786 (F) | TTTACTTTCACCAGCGTTTC | 103 | 7 | 4 | | TGG | 0.43 | +2.20 |
| 65 | 231-250 (RC) | ATTAATAGACTGGATGGAGG | 104 | GATGGAGG | GAGG | GAGG | CGG | 0.48 | +0.00 |
| 65 | 231-250 (RC) | ATTAATAGACTGGATGGAGG | 104 | 8 | 4 | 4 | CGG | 0.48 | +0.90 |
| 66 | 234-253 (RC) | ACAATTAATAGACTGGATGG | 105 | GATGG | None | None | AGG | 0.39 | -0.30 |
| 66 | 234-253 (RC) | ACAATTAATAGACTGGATGG | 105 | 5 | | | AGG | 0.39 | +0.20 |
| 67 | 237-256 (RC) | GCAACAATTAATAGACTGGA | 106 | GACTGGA | CTGGA | CTGGA | TGG | 0.39 | -0.20 |
| 67 | 237-256 (RC) | GCAACAATTAATAGACTGGA | 106 | 7 | 5 | 5 | TGG | 0.39 | -0.20 |
| 68 | 241-260 (RC) | CCCGGCAACAATTAATAGAC | 107 | AATAGAC | AGAC | None | TGG | 0.48 | +1.80 |
| 68 | 241-260 (RC) | CCCGGCAACAATTAATAGAC | 107 | 7 | 4 | | TGG | 0.48 | +2.60 |
| 69 | 259-278 (RC) | AACTACTTACTCTAGCTTCC | 108 | AGCTTCC | GCTTCC | None | CGG | 0.48 | +0.60 |
| 69 | 259-278 (RC) | AACTACTTACTCTAGCTTCC | 108 | 7 | 6 | | CGG | 0.48 | +1.60 |
| 70 | 284-303 (RC) | ACGTTGCGCAAACTATTAAC | 109 | TATTAAC | None | TAAC | TGG | 0.43 | -1.90 |
| 70 | 284-303 (RC) | ACGTTGCGCAAACTATTAAC | 109 | 7 | | 4 | TGG | 0.43 | -1.90 |
| 73 | 375-394 (RC) | TGTAACTCGCCTTGATCGTT | 110 | TCGTT | CGTT | CGTT | GGG | 0.52 | -0.50 |
| 73 | 375-394 (RC) | TGTAACTCGCCTTGATCGTT | 110 | 5 | 4 | 4 | GGG | 0.52 | +0.10 |
| 74 | 376-395 (RC) | ATGTAACTCGCCTTGATCGT | 111 | TTGATCGT | TCGT | None | TGG | 0.48 | -0.50 |
| 74 | 376-395 (RC) | ATGTAACTCGCCTTGATCGT | 111 | 8 | 4 | | TGG | 0.48 | +0.10 |
| 81 | 443-462(RC) | AACTTACTTCTGACAACGAT | 112 | CAACGAT | None | None | CGG | 0.43 | +0.40 |
| 81 | 443-462(RC) | AACTTACTTCTGACAACGAT | 112 | 7 | | | CGG | 0.43 | +0.40 |
| 84 | 528-547(RC) | AGTCACAGAAAAGCATCTTA | 113 | GCATCTTA | None | None | CGG | 0.43 | -0.50 |
| 84 | 528-547(RC) | AGTCACAGAAAAGCATCTTA | 113 | 8 | | | CGG | 0.43 | +0.50 |
| 90 | 638-657(RC) | ACTTTTAAAGTTCTGCTATG | 114 | GCTATG | None | None | TGG | 0.35 | -0.70 |
| 90 | 638-657(RC) | ACTTTTAAAGTTCTGCTATG | 114 | 6 | | | TGG | 0.35 | +0.10 |

All the target sequence from the *bla* gene was obtained using Guide RNA Target Design Tool (https://wwws.blueheronbio.com/external/tools/gRNASrc.jsp) from BlueHeron. There are 98 target candidate sequences returned. Bacterial off-target chromosomal short similar sequences are mapped against the *bla* gene followed by counting the seed sequence from the canonical PAM sequence. Choose the sequences whose seed sequence number is less than eight and whose Gibbs free energy is relatively large. The summary of the property of the selected target sequences is shown in Table 1. This table also shows nucleotide length of the seed sequence of the off-target sequences on pCas9 and pBR322.

### Oligo cassette sequence for spacer sequence

The following four spacer sequences are crRNA generating cassettes targeting beta-lactamase on pBR322 in *E*. *coli* as a host strain, which meet the above two criteria. crRNA CR05 cleaves phosphodiester bond between 762nd base C and 763rd base C, CR30 cleaves phosphodiester bond between 198th base G and 199th base A, CR70 cleaves phosphodiester bonds between 575th base T and 576th base A and CR90 cleaves phosphodiester bonds between 221st base T and 222nd base A on the beta-lactamase gene.

Adaptors for single targets on the beta-lactamase gene.
CR05
   5'-AAACTAGATAACTACGATACGGGAg SEQ ID NO: 115 atctattgatgctatgccctcAAAA-5' SEQ ID NO: 116
CR30
   5'-AAACACTTTAAAAGTGCTCATCATg SEQ ID NO: 117 tgaaattttcacgagtagtacAAAA-5' SEQ ID NO: 118 *Dra*I
CR70
   5'-AAACACGTTGCGCAAACTATTAACg SEQ ID NO: 119 tgcaacgcgtttgataattgcAAAA-5' SEQ ID NO: 120 *Acl*I
CR90
   5'-AAACACTTTTAAAGTTCTGCTATGg SEQ ID NO: 121 tgaaaatttcaagacgataccAAAA-5' SEQ ID NO: 122 *Dra*I

Adaptor for dual targets on the beta-lactamase gene.
Internal direct repeat sequence is italicised and underlined.
CR30+CR90 SEQ ID NO: 123

The 5' end of each oligo is phosphorylated and ready for cloning at *Bsa*I sites.
Sites of six base cutter restriction endonucleses are underlined, which are useful to screen the recombinants. We can also employ one of the cassette oligos as a primer to screen the recombinants by PCR together with another unique primer for the plasmid vector.

### Appendix 1

### pCas9 plasmid sequence (SEQ ID NO: 125)

Cas 9 gene, CRISPR expression locus and tracrRNA (all from *S*. *pyogenes*)

The reverse complement of tracrRNA is in lowercase, Cas9 coding sequence (SEQ ID NO: 126) is boxed and direct repeat sequence in CRISPR is in bold. Promoter sequences were predicted by neural network algorithm (http://www.fruitfly.org/seq_tools/promoter.html). The two unique sites, *Sal* I (GTCGAC) and *Xba* I (TCTAGA) highlighted in bold italicised black are utilised to isolate the CRISPR/Cas9 construct for cloning into M13mp18. The pACYC184 backbone sequence is italicised.

The information of Appendix 1 above is alternatively presented as follows.

### pCas9 plasmid sequence (SEQ ID NO: 125)

Cas 9 gene, CRISPR expression locus and tracrRNA (all from *S*. *pyogenes*)

Backbone vector pACYC184 sequence is italicised, sequence positions are numbered from G of EcoRI site underlined. The reverse complement of tracrRNA is in italicised bold located from 1844 to 1929, Cas9 initiation and termination codons are indicated by bold three letters, starting at nucleotide No. 2225 and ending at 6331 followed by leader sequence 6389-6483 indicated by italicised bold letters, first, second and third direct repeat sequences are underlined, between the first and second direct repeat in which spacer cloning region is located.

This spacer cloning region contains two inverted *Bsa*I sites indicated by bold italicised letters 5'-GAGACC-3' and 5'-GGTCTC-3' for creating 5' four bases protruding spacer cloning sites 5'-GTTT-3' and 5'-TTTT-3', respectively. Promoter sequences were predicted as above, indicated by lower case for forward promoter for Cas9 and leader sequence and italicised lower case for reverse promoter for tracrRNA, the putative transcription start site is indicated by bold uppercase. The two unique sites, Sa*l* I (GTCGAC) and *Xba* I (TCTAGA) highlighted in bold italicised black are utilised to isolate the CRISPR/Cas9 construct for cloning into M13mp18.

### Appendix 2

### Modification of spacer cloning site on pCas9

This system exploits the fact that expression of a minigene yields a certain oligopeptide that depletes the tRNA pool in the host cell, when induced for expression, which leads to the disruption of protein synthesis (Tenson T, Vega-Herrera J, Kloss P, Guarneros G, Mankin AS. J. Bacteriol. 1999; 181:1617-1622). Applying this concept to cloning the spacer sequence: when the spacer sequence is inserted this interrupts the minigene and the oligopeptide expression does not occur, thus tRNA is not depleted, thus only the bacteria harbouring a plasmid with insert can survive under conditions of induced expression; whilst bacteria harbouring the plasmid without insert cannot grow. The following (SEQ ID NO: 129) is the sequence structure. In order to construct this structure, bacteria should express the *lac* I repressor constitutively to switch off minigene transcription. Recombinant selection should be performed in the presence of IPTG to induce transcription.

*Bsa*I recognition sites are underlined. lac operator 3 and 1 are bold italicised. Tac promoter is in bold letters, -35 and -10 region in the promoter sequence are underlined, Shine-Dalgarno sequence is boxed, first dipeptide is in bold M and R followed by three consecutive termination codons italicised TAA. Tryptophan terminator signal sequence is employed, indicated by italicised letters.

### Appendix 3

### Expected cleavage site on target sequence using CR90 spacer sequence

### First processing event

tracrRNA hybridises to the direct repeat region of pre-crRNA indicated by upper case letters using the sequence underlined. Bacterial RNase III cleaves the double-stranded RNA region at indicated position "I", first processing event.

### Structure after 1st processing event

The first processing event may also be depicted as follows: tracrRNAs indicated by lower case letters hybridise to the direct repeat region of pre-crRNA indicated by upper case bold black letters. Bacterial RNase III cleaves the double-stranded RNA region at indicated position with arrows, which is first processing event. Phosphodiester bond between 22^{nd} and 23^{rd} base in the first and second direct repeat are cleaved. Italicised spacer sequence CR90 is boxed.

### Second processing event

The 2nd cleavage point (*) is around 20 nt away from the 3' end of the spacer sequence. "Note that the 2nd processing event occurs at a specific distance from the 1st cleavage within the repeats. Considering that spacer sequences are not identical among each other, it is thus likely that the 2nd processing event within the spacers is distance-dependent rather than sequence-dependent" (see Supplementary figure 2 legend in Nature. Mar 31, 2011;471 (7340):602-607).

Although the above-referenced article does not particularly specify the enzyme and/or mechanism involved in this 2nd processing event, it is most likely Cas9 is involved in this 2nd cleavage. Considering this cleavage position within the spacer sequence, the whole spacer sequence is not contributing to the target recognition, instead only 20 nt spacer sequence is utilised for hybridisation.

The second processing event may also be depicted as follows: The 2nd cleavage point indicated by arrows around 20 nt away from the 3' end of the spacer sequence in the above figure.

The above note quoted from Nature, and our comment, applies.
The italicised spacer sequence CR90 is in box.

### Target DNA cleavage

The part of the target beta-lactamase DNA sequence containing CR90 is shown. crRNA hybridises to its complementary sequence of the target region, Cas9 cleavage points are indicated by dot "." and the PAM sequence tgg is indicated in the box.

An alternative depiction of the target DNA cleavage is shown below. Here, the part of the target beta-lactamase DNA sequence containing CR90 proto-spacer sequence is shown in lower case bold black letters. crRNA hybridises to its complementary sequence of the proto-spacer strand, Cas9 cleavage points are indicated by dot arrows and the PAM sequence tgg is under lined.

### Example 2

The following experiments describe some proof-of-concept experiments performed to demonstrate that the CRISPR-Cas9 system can be used to inactivate antibiotic resistance in bacteria. They describe the construction of a generally applicable DNA cassette, described in the Examples to deliver the CRISPR-Cas9 system plus a derivative carrying a spacer sequence targeted against an antibiotic resistance gene for delivery by naked DNA transformation and bacteriophage infection and also to demonstrate inhibition of the spread of antibiotic resistance by plasmid conjugation.

### Construction of pNB100

pNB100 is a vector to express the CRISPR-Cas9 system in *E.coli* with the appropriate unique restriction site, *Bsa* I, to clone any desired spacer sequence between two direct repeats in the CRISPR locus. The backbone of the vector is derived from pACYC184 and the CRISPR-cas9 locus is inserted into Eco RV site of the vector. Three regions of the CRISPR-cas9 locus were amplified by PCR from the genomic DNA of *Streptococcus pyogenes* strain SF370, purchased from the ATCC, and assembled by Gibson assembly (Gibson DG, et.al. Nature Methods 2009; 6: 343-345) along with the pACYC184 vector in the reaction. The sequence of the final construct was verified by Sanger sequencing. The CRISPR-Cas9 activity was confirmed using a derivative of pNB100, pNB102, carrying a spacer sequence targeted against the *beta lactamase* genes of the bacterial transposons Tn3 and Tn1.

### The amplified sequence of the three regions and amplicon image on the gel

The following sequences are the three regions amplified by PCR. Underlined sequences are template-specific primer sequences, bold letters are overlapping sequences used for Gibson assembly.
1. Fragment 1 (SEQ ID NO: 140), tracrRNA-cas9 : amplicon length = 4758 bp Forward primer is from 854170 to 854193 and reverse primer is from 858867 to 858848 on *S*. *pyogenes* SF370 genomic DNA.
2. Fragment 2 (SEQ ID NO: 141), Leader and first direct repeat: amplicon length = 276 bp Forward primer is from 860648 to 860671 and reverse primer is from 860862 to 860806 on *S*. *pyogenes* genomic DNA.
3. Fragment 3 (SEQ ID NO: 144), Second direct repeat: amplicon length = 452 bp Forward primer is from 861221 to 861276 and reverse primer is from861613 to 861594 on *S*. *pyogenes* genomic DNA. Decamer sequence from 861246-861255 GGTCTCCATT (SEQ ID NO: 142), which contains *Bsa*I recognition sequence on the genomic DNA, was substituted with GGTCCCAAAA (SEQ ID NO: 143) to destroy *Bsa*I recognition sequence and convert the 7th truncated direct repeat in the CRISPR array on the genome to the canonical 2nd direct repeat sequence in this vector.

PCR conditions to generate the three fragments were:

| | Fragment 1 | Fragment 2 | Fragment 3 |
|---|---|---|---|
| 5X Q5 Reaction Buffer | 1 x | 1 x | 1 x |
| 10 mM dNTPs | 200 µM | 200 µM | 200 µM |
| 10 µM Forward Primer | 0.5 µM | 0.5 µM | 0.5 µM |
| 10 µM Reverse Primer | 0.5 µM | 0.5 µM | 0.5 µM |
| *S*. *pyogenes* DNA 50 - 100 ng/ul | 1 ng/µl | 1 ng/µl | 1 ng/µl |
| Q5 High-Fidelity DNA Polymerase 2 U/µL(NEB) | 0.04 U/µl | 0.02 U/µl | 0.02 U/µl |
| Thermocycling condition | | | |
| Initial Denaturation | 98°C_60 sec | 98°C_60 sec | 98°C_60 sec |
| 35 Cycles | 98°C_10 sec | 98°C_10 sec | 98°C_10 sec |
| | 64°C_30 sec | 62°C_30 sec | 62°C_30 sec |
| | 72°C_240 sec | 72°C_30 sec | 72°C_30 sec |
| Final Extension | 72°C_120sec | 72°C_120 sec | 72°C_120 sec |
| Hold | 4 °C | 4 °C | 4 °C |

Results showing the PCR amplicons are provided in Figure 11.

### Assembly of pNB100 from three PCR amplicons, tracrRNA-cas9, leader and first direct repeat, second direct repeat; plus pACYC184 digested with EcoRV

We employed a Gibson assembly kit from NEB (E5510) and followed the protocol provided by the manufacturer to assemble the above three PCR amplicons along with pACYC184. The component of each fragment in the assembling reaction is shown in the following table.

| | |
|---|---|
| 0.1 pmol/µL Fragment 1 | 0.2 pmol |
| 0.2 pmol/µL Fragment 2 | 0.2 pmol |
| 0.2 pmol/µL Fragment 3 | 0.2 pmol |
| 0.01 pmol/µL pACYC184 | 0.04 pmol |
| Fragments 1 :: Fragment 2 : Fragment 3 : vector | 5: 5 : 5 : 1 |
| Gibson Assembly Master Mix (2X) | 1x |
| Incubation | 50°C for 1 hr |

2 µL of the assembly reaction was transformed to DH5α competent cells (purchased from New England Biolabs) followed by selection on chloramphenicol (35µg/mL) LB plates. The recombinants were screened by PCR using the three primer sets used for obtaining the initial three fragments. The plasmid templates giving three desired amplicons were isolated from the candidate clones and were subjected to sequence analysis.

### The sequence of the final construct of pNB100 (SEQ ID NO: 145)

The total number of nucleotides is 9578 bp. The backbone vector pACYC184 sequence is italicised, sequence positions are numbered from G of *Eco*RI site (GAATTC) underlined. tracrRNA is located at nucleotide No. from 1889 to 1974 indicated bold letters, Cas9 initiation and termination codons are indicated by bold three letters, starting at nucleotide No. 2270 and ending at 6376 followed by leader sequence 6462-6556 indicated by italicised bold letters, first and second direct repeat sequences are underlined, between which spacer cloning region (30 mer) is located. This spacer cloning region contains two inverted *Bsa*I sites indicated by bold italicised letters 5'-GAGACC-3' and 5'-GGTCTC-3' for creating 5' four bases protruding spacer cloning sites 5'-GTTT-3' and 5'-TTTT-3', respectively and one unique *Aat*II (5'-GACGTC-3') site also indicated by bold italicised to reduce self-ligation in the event of incomplete *Bsa*I digestion. Note the transition and transversion base changes G6573A, A6779T that were detected by Sanger sequencing and are shown in bold letters, respectively. However, these point mutations do not affect the CRISPR-Cas9 activity, which will be shown in the later section. The two unique sites, *Sal*I (GTCGAC) and *Xba*I (TCTAGA) highlighted in bold italicised letters are utilised to isolate the CRISPR/Cas9 construct for cloning into M13mp18.

A plasmid map of pNB100 is shown in Figure 12.

The desired spacer sequence can be cloned in the clockwise direction between *Bsa*I sites. This vector contains the p15A origin at 1393-848 and *cat* (chloramphenicol resistant) gene at 219-9137. Cutting positions of each restriction enzyme, indicated in the parentheses, refer to the position of the 5' cutting sites on the top strand within the recognition sequence.

### Construction of pNB102

pNB100 was digested with *Bsa*I and *Aat*II followed by purification using Agencourt ampure beads. The spacer sequence CR30 was employed from the discussion in B. Selection of spacer sequence from the target sequence in the Materials and Methods section above. The CR30 sequence is as follows :
5'-AAACACTTTAAAAGTGCTCATCATg (SEQ ID NO: 117)
tgaaattttcacgagtagtacAAAA-5' (SEQ ID NO: 118)

This double-stranded DNA cassette is generated by denaturation at 95 C for 1 min and re-annealing at -1 degree every min to 30 C in the 1 x T4 ligase buffer (50 mM Tris-HCI(pH 7.5 at 25 C), 10 mM MgCl2, 1 mM ATP 10 mM DTT) plus 50 mM NaClfollowing the kinase reaction to add a phosphate moiety in the 5' terminus of each oligo. The annealed cassette contains 5' protruding four base compatible bases on both ends for the sites created on pNB102 by *Bsa*I digestion. This CR30 cassette was ligated to pNB100 by T4 DNA ligase and transformed to DH5α competent cells (purchased from New England Biolabs). The transformants were selected on chloramphenicol LB plate and were screened by PCR with the bottom sequence of the CR30 cassette as a reverse primer and a forward primer CF1: 5'-acgttgacgaatcttggagc, which anneals at 6209-6228 region on the recombinant plasmid to generate 409 bp PCR amplicon. PCR positive clones were sequenced to confirm the CR30 spacer sequence and this recombinant clone is designated as pNB102 and used for *in vitro beta lactamase-*gene disruption experiments. CR30 spacer anneals to the sense strand of *beta lactamase-*gene and cleaves the phosphodiester bonds between 188th and 189th nucleotide on the sense and antisense strand.

### Construction of M13mp18::NB102

pNB102 was digested with unique restriction sites *Sal*I and *Xba*I to generate two fragments 6044 bp and 3524 bp. The fragment length was calculated from the 5' end of the restricted sites in the top strand within the restriction recognition sites. The 6044 bp fragments containing CRISPR locus with CR30 spacer sequence in the CRISPR array was separated from the 3524 bp fragment and purified on the preparative 1 % agarose gel. M13 mp18 was digested with *Sal*I*- Xba*I*,* followed by Agencourt ampure purification to remove the six bases *Sal*I*-Xba*I fragment from the reaction. These two purified fragments were combined and ligated by T4 DNA ligase and transformed to DH5αF'Iq competent cells (purchased from New England Biolabs). Transformed cells were plated along with freshly grown DH5αF'Iq cells as a phage indicator and IPTG/X-gal solution as a blue-white colour indicator with molten top agar to LB plate. White plaques collected from the lawn were screened by PCR for the presence of the CR30 spacer sequence. The correct phage constructs obtained were purified by two times single plaque isolation. The entire sequence length of the final construct is 13288 bp. This spacer CR30 positive recombinant is designated as M13mp18::NB102 and was used for the bla-gene inactivation experiments mediated by M13 phage delivery.

### Delivery of CRISPR-Cas9 constructs to bacteria

Having constructed the CRISPR-Cas9 plasmid pNB100 and the derivative plasmid pNB102 carrying a spacer insertion targeted against the beta-lactamase (*bla*) genes encoded by the bacterial transposable elements Tn1 and Tn3, we then sought to demonstrate, using three delivery methods, (i) plasmid conjugation, (ii) plasmid DNA transformation, (iii) bacteriophage infection, that bacterial cells carrying copies of the CRISPR-Cas9 construct with bla-spacer insertion would be unable to grow in the presence of the beta-lactam antibiotic ampicillin.

Constructs that are able to inactivate target genes, including antibiotic resistant genes, via the CRISPR-Cas system, and which are an aspect of the present invention are also referred to herein as "Nemesis symbiotics".

### "Nemesis symbiotic activity" (NSA) assay by plasmid conjugation: a prophylaxis experiment

We demonstrate here that Nemesis symbiotics can prevent the spread of antibiotic resistance by inhibiting conjugal transfer of conjugative plasmids carrying antibiotic resistance genes from a donor cell to a recipient cell carrying the Nemesis symbiotics. To do this we mated a recipient cell carrying the Nemesis symbiotics with a donor cell carrying a conjugative plasmid, plus a multicopy mobilisable plasmid carrying the *bla* gene encoding ampicillin resistance. In a successful mating, the conjugative plasmid will transfer itself plus the mobilisable plasmid carrying ampicillin resistance to the recipient. Exconjugants may be selected for resistance to both chloramphenicol present on a non-mobilisable plasmid in the recipient and ampicillin received from the donor. Successful Nemesis symbiotic activity will reduce the efficiency of transfer of ampicillin resistance.

The recipient cell DH5α (F- endA1 glnV44 thi-1 recA1 relA1 gyrA96 deoR nupG Φ80dlacZΔM15 Δ(lacZYA-argF)U169, hsdR17(rK- mK+), λ-) was purchased from New England Biolabs and transformed with the plasmids pNB100 or pNB102 or pACYC184, where plasmids encode chloramphenicol resistance and both pNB100 and pNB102 carry CRISPR-Cas9 but only pNB102 carries the spacer sequence targeted against the beta-lactamase gene. The plasmid pACYC184 is the non-mobilisable parent plasmid used for the construction of pNB100 and pNB102 as described above.

The donor strain JA200 (F+ thr-1, leu-6, DE(trpE)5, recA, lacY, thi, gal, xyl, ara, mtl) also carrying plasmid pNT3 is described by Saka et al. DNA Research 12, 63-68 (2005). The plasmid pNT3 is a mobilisable plasmid carrying the *bla* gene of Tn1.

A single colony of the donor JA200 pNT3 was picked from a Luria broth (LB) plate containing 100 µg/mL Ampicillin and grown shaking at 37°C overnight in 1mL LB medium with 100 µg/mL Ampicillin. A single colony each of the recipients, DH5α pNB100 and DH5α pNB102 was picked from a LB plate containing 35 µg/mL Chloramphenicol and grown shaking at 37°Covernight in 1mL LB with 35 µg/mL Chloramphenicol. To wash cells to remove antibiotics, 50µL of cells were added to 1mL LB in Eppendorf tubes and centrifuged 60 sec at 12500 rpm. Cells were resuspended in 50µL LB. To set up the mating JA200 pNT3 was spotted onto an LB plate, then 2µL of each DH5α carrying pNB100 and pNB102 were added to this spot. Separate 2µL spottings of donor and recipients were also performed (i.e. not mated). Plates were incubated at 37°C for 4 hours. Cells were removed resuspended in LB and 100µL plated on LB plates containing both 100 µg/mL Ampicillin and 35 µg/mL Chloramphenicol (LB ApCm). 100µL of 10,000 fold (10^-4) dilutions were also plated on LB plates and incubated at 37°C overnight. The resultant colonies were counted as shown in the Table below.

| **Cells** | **LB ApCm plates** | **LB plates 10^{∧}-4 dilution** | **Nemesis symbiotic activity** |
|---|---|---|---|
| JA200 pNT3 x DH5α pNB100 | Confluent | Approx. 500 | Negative |
| JA200 pNT3 x DH5α pNB102 | 37 | Approx. 500 | Positive |
| JA200 pNT3 x DH5α pACYC184 | Confluent | Approx. 500 | Negative |
| JA200 pNT3 | 0 | Not done | Not applicable |
| DH5α pNB100 | 0 | Not done | Not applicable |
| DH5α pNB102 | 0 | Not done | Not applicable |
| DH5α pACYC184 | 0 | Not done | Not applicable |

Photographs in Figure 13 show platings of the matings between: (A) JA200 pNT3 x DH5α pNB100 (as expected lacking Nemesis symbiotic activity); and (B) JA200 pNT3 x DH5α pNB102 (showing Nemesis symbiotic activity).

The 10^-4 dilution plated on LB plates, gave approximately 500 colonies a count of 5 x 10^7 cells per mL in the mated cell suspension and for the JA200 pNT3 x DH5α pNB102 only 3.7 x 10^ 2 cells/mL were able to grown on the LB Ap1 00Cm35 plates. Thus assuming half the cells are recipients/ex-conjugants then: 3.7 x 10^ 2 cells /ml divided by 2.5 x 10^7 gives a mating efficiency for the recipient carrying pNB102 of 1.2 x 5 x 10^ -5

The experiment demonstrated a significant reduction in CmRApR exconjugants in matings with DH5α carrying pNB102 versus pNB100 x JA200 pNT3.

In order to measure the relative mating efficiencies more accurately, after a liquid mating cells were plated on LB plates to titre all cells, LB Ap100 plates to titre donors plus exconjugants, LB Cm plates to titre recipients plus exconjugants and LB Ap100Cm35 to titre exconjugants only.

For the liquid mating overnight cultures of 10µL of JA200 pNT3 were mixed with 10µL of recipients DH5α pNB100 or DH5α pNB102 200µL of LB added and tubes incubated overnight at 37°C. Mating mixtures were diluted 10^-1, 10^-3, 10^-5 in LB and 50µL of dilutions plated on LB, LB Ap100Cm35, LB Ap100 and LB Cm35 plates and plates incubated overnight at 37°C. The table below summarises the cell titres obtained.

| **Mated with JA200 pNT3** | **LB All cells** | **LB Ap Cm Exconjugants** | **LB Cm recipients and exconjugants** | **LB Ap donors and exconjugants** | **mating effic/donor** | **mating effic/recipient** |
|---|---|---|---|---|---|---|
| pNB100 | 4.14 x 10 ^8 | 2.80 x 10 ^7 | 1.40 x 10 ^8 | 2.64 x 10 ^8 | 1.06 x 10^ -1 | 2.00 x 10^ -1 |
| pNB102 | 5.16 x 10 ^8 | 7.20 x 10 ^3 | 1.78 x 10 ^8 | 3.82 x 10 ^8 | 1.88 x 10^-5 | 4.04 x 10^ -5 |

The number of cells on LB Cm plus LB Ap plates should equal the number of cells on LB plates. For pNB100 1.40 x 10^8 (Cm plates) plus 2.64 x 10^8 (Ap plates) = 4.04 x 10^8 which agrees very well with 4.14 x 10^8 on LB plates. For pNB102 1.78 x 10^8 (Cm plates) plus 3.82 10^8 (Ap plates) = 5.6 x 10^8 which agrees very well with 5.16 x 10^8 on LB plates.

In conclusion, the data show that after overnight mating in liquid culture, there is a 5,000 fold reduction in mating efficiency per recipient comparing pNB102 with the spacer to pNB100 lacking the spacer.

### NSA Assay by plasmid transformation

In this experiment, we demonstrate that introduction of Nemesis symbiotics to recipient cells by DNA transformation inactivates antibiotic resistance in the transformants.

In order to obtain a tester strain, DH5α competent cells purchased from New England Biolabs were transformed with pBR322 (carrying the *bla* gene derived from Tn3) and selected on LB Ap100 plates. Competent cells of the derived strain DH5α pBR322 were then prepared using the CaCl2 protocol 25 (1.116) as described by Sambrook and Russell in Molecular Cloning: A Laboratory Manual (3rd Edition, 2001) and subsequently transformed with plasmids pNB100, pNB102 and pACYC184 with selection for CmR. Transformant colonies were then picked onto LB Cm35 and LB Ap100 plates. Primary transformants were replica toothpicked onto both LB Cm35 and LB Ap100 plates and incubated overnight at 37°C.

The results, depicted in Figure 14, show that all colonies toothpicked from DH5α pBR322 transformed by pNB100 (lacking the bla gene target spacer sequence) remain resistant to ampicillin. In contrast all colonies toothpicked from DH5α pBR322 transformed by pNB102 have lost ampicillin resistance, so demonstrating Nemesis symbiotic activity.

The experiments above do not give a value for the fraction of primary transformants where NSA has inactivated the *bla* gene. To address this, single colonies from the primary transformants were picked into 1mL LB and diluted 10^-3 in LB. Then 100µL plated onto plates as follows, and results scored. The results showed that following transformation of DH5α pBR322 with pNB102 fewer than 10^-6 cells retain ApR. Nemesis symbiotic activity is very efficient.

### NSA Assay by bacteriophage M13 infection

In this experiment, we demonstrate that introduction of Nemesis symbiotics to recipient cells by bacteriophage infection inactivate antibiotic resistance in the transformants. We chose the male-specific filamentous phage M13 as the delivery agent for the Nemesis symbiotic construct. An M13 derivative M13mp18::NB102 carrying and the Cas9 CRISPR plus *bla* gene target spacer region of pNB102 was used to deliver the Nemesis symbiotic by infection of an F+ strain, JA200, carrying ampicillin resistance on the plasmid pNT3. 0.2mL of a fresh culture of this strain was added to 3 mL of LB top agar (Luria broth with 0.7% agar) and poured onto an LB plate. Then 2µL of phage stocks of M13mp18::NB102 (10^8 pfu/mL) and as a control M13mp18 were spotted onto the lawn and the plate was incubated 8 hours at 37°C. Plaques were picked into 1.5 mL LB and grown shaking o/n at 37°C. A control strain DH5α lacking ampicillin resistance was also cultured overnight from a single colony picked into 1.5 mL LB.

### Nitrocefin assay for beta lactamase activity was performed:

1mL of the culture of cells was centrifuged for 60 sec at 12,500 rpm in microfuge then 2µL of stock nitrocefin (10mg/mL in DMSO) was added to 1mL of cell supernatant and absorbance of the degradation product of nitrocefin was measured at 482 nm in a spectrophotometer several time points after addition of nitrocefin. The Table below summarises the results.

| **Strain** | **30 sec** | **60 sec** | **2 min** | **5 min** |
|---|---|---|---|---|
| JA200 pNT3 infected by M13mp8 | 0.5 | 0.64 | 0.73 | 0.79 |
| JA200 pNT3 infected by M13mp8::NB102 | 0.08 | 0.09 | 0.11 | 0.15 |
| DH5α | 0.09 | 0.07 | 0.06 | 0.05 |

The experiments reported above provide the proof-of-concept that, in the model organism, *Escherichia coli,* DNA constructs carrying the Cas9 CRISPR region plus a spacer region with sequences directed against a target region of the beta-lactamase gene can inactivate ampicillin resistance when delivered by naked DNA transformation and bacteriophage infection as well as prevent transfer of ampicillin resistance by plasmid conjugation. It is apparent that Nemesis symbiotics of the invention can be applied to pathogenic bacteria and for other antibiotic resistance genes.

### Example 3

The aim of Example 3 is to extend the proof-of-concept for resurrection of antibiotic efficacy by introduction of a CRlSPR/Cas9 construct in a pathogenic bacterial strain of *Klebsiella pneumoniae.* Resistance to a newer class of beta-lactam antibiotics, the Carbapenems has emerged in Enterobacteriaceae by the acquisition of new *bla* genes that encode beta-lactamases able to degrade even the Carbpenems. *Klebsiella pneumoniae* strains with resistance to Carbapenems, KPC are important causes of morbidity and mortality among hospital-acquired and long-term care-associated infections. Noteworthy is the very high mortality (around 30-70%) among patients with bacteraemia or pulmonary infections.

Example 3 shows that a *Klebsiella pneumoniae* carrying a beta-lactamase (*bla*) gene conferring resistance to the beta-lactam antibiotic, ampicillin become sensitive to ampicillin following introduction of a modified CRISPR/Cas9 construct targeted against the *bla* gene. The same CRISPR/Cas9/anti-*bla* construct used in Example 1 or Example 2 is used and is delivered to *Klebsiella pneumoniae* by conjugation with a donor strain carrying a conjugative plasmid with CRlSPR/Cas9/anti-*bla*. Ex-conjugant recipient *Klebsiella pneumoniae* carrying the plasmid with CRlSPR/Cas9/anti-*bla.* construct is selected for on appropriate media with counter-selection against the donor cell and *Klebsiella pneumoniae* cells that failed to receive the plasmid.

As for Example 1 and Example 2, efficacy of the CRISPR/Cas9/anti-*bla* contstruct is evaluated by comparison to a negative control of a *Klebsiella pneumoniae* ex-conjugant strain that received a plasmid with CRISPR/Cas9 *(i.e*. lacking the anti-*bla* region). As in Example 1 and Example 2, the beta-lactamase activity can be detected by nitrocefin to count white versus red colonies. Also as in Example 1 and Example 2, alternatively beta lactamase activity is seen directly challenging the bacteria on the LB plate with/without ampicillin and measuring the fraction of the ampicillin sensitive colonies versus ampicillin resistant ones.

The identical experiment to that described above is also evaluated using instead a CRlSPR/Cas9/anti-*blaCb*, where the CRISPR/Cas9/anti-*blaCb* construct carries a region, *anti-blaCb,* targeted against a *bla* gene encoding a beta-lactamase able to break down carbapenems and present in that particular *Klebsiella pneumoniae* strain.

### Example 4

Example 4 provides delivery routes for the therapeutic constructs. These routes all apply to veterinary as well as human applications to be delivered orally, topically, probiotically and for use in surgical irrigation fluids and wound dressings.

Orally: Phage containing assassin construct as the active ingredient may be administered orally as a stabilised therapeutic preparation: either - administered before antibiotic therapy or administered in the form of an adjuvant complexed to an antibiotic. Conjugative plasmids containing assassin construct as the active ingredient may be administered as a culture of commensal bacteria carrying these plasmids in order to transmit these plasmids to gut flora thereby generating prophylactic protection against future infection with antibiotic resistant bacterial pathogens.

Topically: Phage containing assassin construct as the active ingredient may be administered topically as a stabilised medication (ointment, spray, powder etc): either - administered before antibiotic topical medication; or administered in the form of a complex with an antibiotic medication. Topical application of conjugative plasmids containing assassin construct as the active ingredient may be via a stabilised culture of commensal bacteria carrying these plasmids in order to transmit the plasmids to gut flora thereby generating prophylactic protection against future infection with antibiotic resistant bacterial pathogens.

Probiotically: Phage containing assassin construct as the active ingredient may be administered probiotically as a stabilised culture of commensal bacteria (e.g. Lactobacillus spp) carrying these plasmids in order to transmit the plasmids to gut flora thereby generating prophylactic protection against future infection with antibiotic resistant bacterial pathogens. One example of this may be the probiotic, prophylactic administration of such a preparation to patients in settings with a high risk of infection with antibiotic resistant bacterial pathogens such as hospitals, care homes, schools, transplantation centres etc. Another example may be the administration of such a preparation to livestock via animal feeds, thereby limiting the rise and horizontal transmission of antibiotic resistant bacteria. The use of the present invention in livestock thus represents one means for (indirect) prophylactic treatment of antibiotic resistant bacteria in humans.

Surgical irrigation fluids: Phage containing assassin construct as the active ingredient may be added to surgical irrigation fluids and also sprays for disinfecting fomites. Stabilised commensal bacterial cultures containing conjugative plasmids incorporating assassin constructs may be added as coatings to surgical wipes and to wound dressings.

### Example 5

In this example, a construct is designed to include multiple RNA guide molecules where each RNA guide molecule is transcribed by its own promoter.

Figure 16 exemplifies a set of spacer sequences that we have identified encoding 20 guide RNA molecules targeted against 117 different *bla* genes identified in the NCBI ARDB database for *Klebsiella pneumoniae.* The beta lactamase gene type, spacer sequence and antibiotic resistance profile in *Klebsiella pneumoniae* obtained from NCBI ARDB database are shown.

Beta lactamase gene sequences were collected from the ARDB database with the keyword *Klebsiella pneumoniae.* Redundant sequences were removed and unique sequences used for multiple sequence alignment using web program Clustal Omega. One canonical sequence was chosen from each cluster and the 20 nt spacer sequences predicted by the web program Jack Lin's CRISPR/Cas9 gRNA finder collected. The spacer sequence was chosen to maximise the ratio of the proto-spacer sequence found in the sequences belonging to the same branch. Each of the example spacer sequences shown in the 4^{th} column has the capability to disrupt the genes in the third column.

Beta lactamase genes used in this analysis are: SHV-a = 1, 2, 2a, 5, 5a, 11, 12, 14, 26, 27, 28, 31, 33, 38, 43, 44, 48, 55, 56, 60, 61, 62, 71, 73, 74, 75, 76, 77, 78, 79, 80, 81, 82,85, 89, 92, 98, 99, 101, 103, 106, 107, 108, 109, CTXM-b = 1, 3, 10, 12, 15, 22, 32, 54, 60, 62, 68, CTXM-c = 13, 14, 16, 18, 19, 24, 26, CTXM-d = 2, 35, 59, CTXM-e = 26, 63, TEM-f = 1, 1b, 3, ESBL, 139, KPC-g = 1, 2, 3, 4, OKP-h = A11, A12, A16, A17, B13, B-SB613, 6, LEN-i = 2, 17, 18, 19, 20, 21, 22, 24,GES-j = 1, 3, 4, 5, VIM-a = 1, 2, 4, 12, 19, IMP-b = 4, 8, CMY-a = 2, 4, 25, 31, 36, LAT-b = 1, 2, CMY-c = 1, 8b, 10, 19, FOX-d = 1, 5, 7, OXA-a = 1, 30, 47, OXA-2, OXA-9, OXA-b = 10, 17.

Beta lactam antibiotics were classified into four classes, penams, cephems, carbapenem and monobactam. One antibiotic name is listed in Figure 16 as an example under each class. The beta lactamase, which can open the beta lactam ring is indicated by R. For example, carbapenem is inactivated by KPC. To re-sensitise bacteria to carbapenem, the spacer sequence 5'-TTGTTGCTGAAGGAGTTGGG (SEQ ID NO: 45) should be employed into the spacer array and inactivate KPC genes. Note that the spacer sequence for CMY-a can be employed to LAT-b cleavage.

Similarly, Figure 17 exemplifies a set of spacer sequences encoding 17 guide RNA molecules targeted against 154 different *bla* genes identified in the CARD database for *Klebsiella pneumoniae.* Beta lactamase gene type, spacer sequence and antibiotic resistance profile in *Klebsiella pneumoniae* obtained from the IIDR CARD database are shown.

Beta lactamase gene sequences were collected by filtering all the collected beta lactamase genes with the keyword *Klebsiella pneumoniae* and subjected to multiple sequence alignment using web program Clustal Omega. One canonical sequence from each cluster was chosen and the 20 nt spacer sequences predicted by the web program Jack Lin's CRISPR/Cas9 gRNA finder collected. The spacer sequence was chosen to maximise the ratio of the proto-spacer sequence found in the sequences belonging to the same branch. The each of the example spacer sequences shown in the 4^{th} raw has the capability to disrupt the genes in the third column.

Beta lactamase genes used in this analysis are: SHV-a = 1a, 5, 2a,11, 18, 20, 21, 22, 23, 28, 31, 32, 52, 55, 98, 99, 100, 106, 107, 110, 111, 121, 134, 136, 137, 140, 143, 144, 147, 148, 149,150, 151, 152, 153, 154, 155, 157, 158, 159, 160, 161, 162,163, 164, 165, 168, 172, 173, 178,179, CTXM-b = 10, 12, 15, 52, 54, 62, 71, 80, CTXM-c = 19, 81, 99, 147, TEM-f = 1, 162, 183, 192, 197, 198, 209, KPC-g = 3, 4, 6, 7, 8, 11, 12, 14, 15, 16, 17, OKP-h = 5, 6, A1, A2, A4, A5, A6, A7, A8, A9 A10, A11, A12, A13, A14, A15, A16, B1, B2, B3, B4, B5, B6, B7, B8, B9, B10, B11, B12, B13, B17, B18, B19, B20, LEN-i = 5, 6, 7, 8, 9, 10, 11, 12, 13, 18, 19, 20, 21, 22, 23, 24, GES-j = 1, 3, 4, VIM-a = 4, 26, 27, 28, 33, 34, IMP-b = 32, 38, NDM-c= 1, 9, 10, ACT-3, CMY-a = 25, 31, 56, 87, FOX-d = 8, 9, OXA-a = 1, 30, 47, OXA-1, OXA-a = 181, 204, 247, OXA-9.

Beta lactam antibiotics are classified into four classes, penams, cephems, carbapenem and monobactam. In Figure 17, one antibiotic name is listed as an example under each class. The beta lactamase, which can open the beta lactam ring is indicated by R. For example, carbapenem is inactivated by KPC. To re-sensitize bacteria to carbapenem, the spacer sequence 5'-TTGTTGCTGAAGGAGTTGGG (SEQ ID NO: 45) should be employed into the spacer array and inactivate KPC genes.

### Example 6

In this example, a modified Cas DNA-binding polypeptide is created that deletes and reseals rather than leaving a DSB.

As described above, DSBs caused by Cas DNA-binding polypeptide such as CRISPR-Cas9 can be lethal to the replicon targeted and can result in cell death rather than, for example, re-sensitisation to antibiotics, if an antibiotic resistance gene in the replicon is targeted for inactivation. Immediate cell death rather than such re-sensitisation to antibiotics for subsequent killing by antibiotic may increase selection pressure against delivery of the targeting constructs. Instead of DSBs, we may modify the Cas9 gene to allow the resealing of the targeted sequence after gene inactivation by introducing a deletion or inversion.

Tsai et al. (2014) have constructed a fusion between a catalytically inactive Cas9 (dCas9) protein to the wild-type nuclease domain of the restriction endonuclease *Fok1* to increase the specificity of cleavage in eukaryotic cells. Proudfoot et al. (2011) have similarly fused zinc-finger DNA recognition domains to the catalytic domains of recombinases to programme site-specific recombination at designated DNA sequences.

We here add appropriate recombinase domains to dCas9 to catalyse a recombination reaction rather than a DSB at a CRISPR-mediated RNA-guided desired position on the target genes.

The Cas9 resolvase fusion, called Cas9R in Figure 18, is directed to the desired sites determined by the Cas9 domain:guide RNA spacer sequences; and the fused resolvase is positioned at the recombination site. To generate a deletion between two recombination sites: A and B, resolvases must dimerise at each recombination site. Thus two Cas9Rs need to be positioned in close proximity at each recombination site A1A2 and B1B2 as designated by the sequences encoded by the spacers S1-S4. The correct orientations of A1 relative to A2 and B1 relative to B2 will need to be determined experimentally.

The orientation of the gRNA as shown Fig. 19 is already proved in the case of dCas9 fused *Fok*I (Tsai el al., 2014) - i.e. PAM sequences of each protospacer sequence in the recombination sites are directly positioned at the outer boundaries of the full-length recombination site. Thus, we employ the same configuration of the annealed gRNA polarity.

Figures 19 and 20 show a schematic process as to how this Cas9R fusion resolvase resolves the synapse and parental replicon DNA is recircularised.

### Example 7

The following experiments describe some proof-of-concept experiments performed to demonstrate that the CRISPR-Cas9 system can be used in a single construct to inactivate a large number of different beta-lactamase genes that may be found amongst microbial pathogens as well as amongst the non-pathogenic members of the microbiome.

In these exemplifications, plasmids are constructed that carry the CRISPR-Cas9 system plus derivatives carrying spacer sequences, flanked by direct repeats, targeted against up to eight of the following beta-lactamase families of resistance genes: SHV, CTX-M, TEM, KPC, VIM, IMP, NDM and OXA.

For each of these eight families of beta-lactamase genes, a single spacer is designed that will target a number of gene members of that family. These are: SHV-a = 1, 1a, 2, 2a, 5, 5a, 11, 12, 14, 18, 20, 21, 22, 23, 26, 27, 28, 31, 32, 33, 38, 43, 44, 48, 52, 55, 56, 60, 61, 62, 71, 73, 74, 75, 76, 77, 78, 79, 80, 81, 82, 85, 89, 92, 98, 99, 100, 101, 103, 106, 107, 108, 109, 110, 111, 121, 136, 134, 137, 140, 143, 144, 147, 148, 149, 150, 151, 152, 153, 154, 155, 157, 158, 159, 160, 161, 162, 163, 164, 165, 168, 172, 173, 178, 179; CTXM-b= 1, 3, 10, 12, 15, 19, 22, 32, 52, 54, 59, 60, 62, 68, 71, 80, 81, 99, 141, 147; TEM-c = 1, 1B, 3, 139, 162, 183, 192, 197, 198, 209; KPC-d = 1, 2, 3, 4, 6, 7, 8, 11, 12, 14, 15, 16, 17 ; VIM-e = 1, 2, 4, 19, 26, 27, 33, 34; IMP-f = 4, 8, 32, 38; and NDM-g = 1, 9, 10.

Figure 21 shows the eight spacer sequences that were designed to target the eight beta-lactamase families of resistance genes: SHV, CTX-M, TEM, KPC, VIM, IMP, NDM and OXA-48. The primer sequences used in the construction of the plasmids are listed in a table in Figure 22.

In one exemplification, a plasmid derivative of pNB100 (Figure 12), pNB104A (Figure 23), a generally applicable DNA cassette, is described in the Examples that carries the CRISPR-Cas9 system plus derivatives carrying spacer sequences, flanked by direct repeats, targeted against four beta-lactamase families of antibiotic resistance genes in bacteria and are expressed off one promotor: NDM, IMP, VIM and KPC.

In another exemplification, a plasmid derivative of pNB100, pNB104B (Figure 24), a generally applicable DNA cassette, is described in the Examples that carries the CRISPR-Cas9 system plus derivatives carrying spacer sequences, flanked by direct repeats, targeted against four beta-lactamase families of antibiotic resistance genes in bacteria and are expressed off one promotor: OXA-48, SHV, TEM and CTX-M.

In another exemplification, a plasmid derivative of pNB100, pNB108 (Figure 25), a generally applicable DNA cassette, is described in the Examples that carries the CRISPR-Cas9 system plus derivatives carrying spacer sequences, flanked by direct repeats, targeted against eight beta-lactamase families of antibiotic resistance genes in bacteria and are expressed off one promotor: SHV, CTX-M, TEM, KPC, VIM, IMP, NDM and OXA-48.

In another exemplification, a plasmid derivative of pNB100 is constructed where spacer sequences can be expressed from a choice of two different promotors. This plasmid, pNB200 (Figure 26), carries a different unique restriction site downstream of each promotor to clone any desired spacer sequences flanked by their own direct repeats between two direct repeats in the CRISPR locus. Derivatives of pNB200 are described that carry the CRISPR-Cas9 system plus derivatives carrying spacer sequences, flanked by direct repeats, targeted against eight beta-lactamase families of resistance genes: SHV, CTX-M, TEM, KPC, VIM, IMP, NDM and OXA-48. In pNB202 (Figure 27), the second promotor is used to express spacers flanked by direct repeats to target the four beta-lactamase families of resistance genes: OXA-48, SHV, TEM and CTX-M. And in pNB203 (Figure 28), which is derived from pNB202, both promotors are used: the first to express spacers flanked by direct repeats to target four beta-lactamase families of resistance genes: NDM, IMP, VIM and KPC and the second promotor to express spacers flanked by direct repeats to target four beta-lactamase families of resistance genes: OXA-48, SHV, TEM and CTX-M.

### Construction of pNB104A

The tetramer spacer concatemer a+b+c+d shown in Figure 29A was digested with *Bsa*I*,* whose restriction site is located in A1 and A2, and ligated to *Bsa*I spacer cloning sites on pNB100. The structure of the single promoter and spacer region (6221-7001) on pNB104A is shown in Figure 23. The four-space concatemer contains spacer sequences targeting NDM, IMP, VIM and KPC from the proximal to the distal end of the single promoter.

### Construction of pNB104B

The tetramer spacer sequence concatemer e+f+g+h shown in Figure 29A was digested with *Sap*I*,* whose restriction site is located in B1 and B2, and ligated to *Sap*I spacer cloning sites on pNB200. While screening pNB202 screening this construct was found and confirmed by sequencing. A deletion event between the direct repeats adjacent to leader sequences had occurred and restored the direct repeat sequence to give pNB104B. The single promoter regions (6221-6987) on pNB104B is shown in Figure 24. The concatenated spacers (targeted against OXA-48, SHV, TEM and CTX-M) are located under the single promoter.

### Construction of pNB108

The concatenated spacer array sequences A and B were amplified from the subcloned vector pCR Blunt II-TOPO_SpacerA and pCR Blunt ll-TOPO_SpacerB with the primer set NB026 and NB029, NB030 and NB033, respectively. At the 3' end of amplicon of spacer A and 5' end of amplicon of spacer B are 20 bases of overlapped sequence from KPC spacer sequence. These two amplicons were gel purified and used for PCR-based pairwise cycle extension reaction in the absence of the primer. The extended material was re-amplified with primer set NB037 (5'-GGGCTGGCAAGCCACGTTTGGTG-3'; SEQ ID NO. 150) and NB038 (5'-CCGGGAGCTGCATGTGTCAGAGG-3'; SEQ ID NO. 151) to generate the full 8 spacer array concatemer. This eight-spacer concatemer was cloned into pCR Blunt II-TOPO vector and sequence was confirmed.

*Bsa*I digestion of this pCR Blunt ll-TOPO subclone removes the full 8 spacer array concatemer as a subclone from the pCR Blunt ll-TOPO vector, which contains 5' protruding four base compatible bases on both ends for the sites created on pNB100 by *Bsa*I digestion. Then pNB100 was digested with *Bsa*I followed by agarose gel purification. The eight-spacer concatemer cassette, released from the pCR Blunt ll-TOPO was ligated into pNB100 by T4 DNA ligase and transformed to DH5α competent cells (purchased from New England Biolabs). The transformants were selected on chloramphenicol LB plates and were screened by PCR with the reverse primer NB021: 5'- GGTGACTGATGGCCACGT (SEQ ID NO: 149) and a forward primer NB020: 5'- CCAACTACCTCCCCTTGCTTAAC (SEQ ID NO: 148), which anneal at 6368-6385 region and 7203-7225 region, respectively on the recombinant plasmid to generate 858 bp PCR amplicon. PCR-positive clones were sequenced to confirm the eight-spacer concatemer sequence and this recombinant clone is designated as pNB108 and used to demonstrate CRISPR/Cas9-mediated inactivation of targeted beta lactamase genes following DNA delivery to bacterial strains carrying such genes. A plasmid map of pNB108 is shown in Figure 25.

### Construction of pNB200

The plasmid pNB200 contains two identical promotors for gRNA expression and carries a different unique restriction site *Bsa*I and Sapl downstream of each promotor to clone any desired spacer sequences flanked by their own direct repeats between two direct repeats in the CRISPR locus. The desired structure of the CRISPR array locus in pNB200 consists two sets of cassettes harbouring promotor-leader-direct repeat-spacer cloning region-direct repeat-tail tandemly. The forward primer NB018 anneals at the 5' end of the leader sequence to the promotor in pNB100 and introduces the first spacer cloning region, second direct repeat and tail sequence. The reverse primer NB019 anneals at the 3' end of leader sequence in pNB100 and introduces a third direct repeat, second spacer cloning region. This amplicon is cloned between BsaI sites on pNB100 to give pNB200.

### The amplified sequence (SEQ ID NO: 146) with primer NB018 and NB019 from pNB100 as template

The forward primer NB018 and the reverse primer NB019 are underlined and the initial annealing sites, for the first PCR cycle, are indicated by bold letters and italicised bold letters, respectively. This amplicon was digested with *Bbv*I (5'-GCAGCN8/N12) to create four bases compatible protruding 5' ends to *Bsa*I digested pNB100. *Bbv*I recognition sites are depicted by lower bold cases.

The PCR conditions to generate the dual promotor-leader cassette were:

| **Component** | |
|---|---|
| Nuclease-Free water | 41.25 µL |
| 10 X PCR Buffer | 5 µL |
| 10 mM dNTPs | 1 µL |
| 10 µM Forward Primer NB018 | 1 µL |
| 10 µM Reverse Primer NB019 | 1 µL |
| pNB100 | 0.5 µL |
| QIAGEN Hot Start Taq | 0.25 µL |

### Cycle conditions

| **STEP** | **TEMP** | **TIME** |
|---|---|---|
| Initial Denat. | 95CC | 15 min |
| 35 Cycles | 94CC | 30 sec |
| | 55 °C | 30 sec |
| | 72 °C | 30 sec |
| Final Extension | 72°C | 10 min |

### The sequence of the modified CRISPR array in pNB200 (SEQ ID NO: 147)

The total number of nucleotides of pNB200 is 9919 bp. The sequence in the modified region is shown below, which replaces the small *Bsa*I fragment in pNB100. The first and the second promotor sequences are underlined. Leader sequences are in bold case. Direct repeats are italicised and the spacer cloning region between direct repeats are indicated by italicised and underlined.

The first spacer array cloning region contains two inverted *Bsa*I sites indicated by bold italicised underlined letters 5'-GAGACC-3' and 5'-GGTCTC-3' for creating 5' four bases protruding spacer cloning sites 5'-GTTT-3' and 5'-TTTT-3' on the vector, respectively and one unique *Aat*II (5'-GACGTC-3') site also indicated by bold italicised underlined lettes to reduce self-ligation in the event of incomplete *Bsa*I digestion.

The second spacer cloning region contains two inverted Sapl sites indicated by bold italicised underlined letters 5'-GAAGAGC-3' and 5'-GCTCTTC-3' for creating 5' three bases protruding spacer cloning sites 5'-GTT-3' and 5'-TTT-3' on the vector, respectively.

A plasmid map of pNB200 is shown in Figure 26. The desired spacer array sequences can be cloned in the clockwise direction between two *Bsa*I sites and two *Sap*I sites independently, for each promotor respectively.

### Construction of pNB202

The plasmid pNB201 contains the dual promotors of pNB200 from which it is derived but a spacer array sequence targeted to OXA-48, SHV, TEM and CTX-M beta lactamase gene families are expressed from the second promotor.

To construct pNB202, pNB200 was digested with *Bsa*I followed by agarose gel purification. The concatenated spacer array sequence B was generated by PCR-mediated pair-wise oligo nucleotide concatenation. This four-spacer concatemer array B was cloned to the pCR Blunt ll-TOPO vector, purchased from Life Technologies, and the sequence was confirmed. Sapl cuts out spacer array sequence B as a subclone from the TOPO vector and yields 5' protruding three base compatible bases on both ends for the sites created on pNB200 by Sapl digestion. This four-spacer concatemer B cassette was ligated to pNB200 by T4 DNA ligase and transformed to DH5α competent cells (purchased from New England Biolabs). The transformants were selected on chloramphenicol LB plate and were screened by PCR with the reverse primer NB021: 5'- GGTGACTGATGGCCACGT (SEQ ID NO: 149) and a forward primer NB020: 5'- CCAACTACCTCCCCTTGCTTAAC (SEQ ID NO: 148), which anneal at 6368-6385 region and 7307-7329 region, respectively on the recombinant plasmid to generate 962 bp PCR amplicon. PCR positive clones were sequenced to confirm the four-spacer concatemer B sequence and this recombinant clone is designated as pNB202 and used to demonstrate CRISPR/Cas9-mediated inactivation of targeted beta lactamase genes following DNA delivery to bacterial strains carrying such genes. A plasmid map of pNB202 is shown in Figure 27.

### Construction of pNB203

The plasmid pNB203 contains the dual promotors of pNB200 but each promotor expresses four spacers. The first promotor expresses spacer sequence targeted to NDM, IMP, VIM and KPC, the second promotor expresses OXA, SHV, TEM and CTX-M beta lactamase genes. The plasmid pNB202 was digested with *Bsa*I followed by agarose gel purification. The concatenated spacer array sequence A was cut out from pCR Blunt ll-TOPO vector harbouring spacer concatemer A with *Bsa*I*. Bsa*I cuts out spacer region, which contains 5' protruding four base compatible bases on both ends for the sites created on pNB202 by *Bsa*I digestion. This four-spacer concatemer A cassette was ligated to pNB202 by T4 DNA ligase and transformed to DH5α competent cells (purchased from New England Biolabs). The transformants were selected on chloramphenicol LB plate and were screened by PCR with the reverse primer NB021: 5'- GGTGACTGATGGCCACGT and a forward primer NB020: 5'- CCAACTACCTCCCCTTGCTTAAC , which anneal at 6368-6385 region and 7479-7501 region, respectively on the recombinant plasmid to generate 1134 bp PCR amplicon. PCR positive clones were sequenced to confirm the four-spacer concatemer A and B sequence and this recombinant clone is designated as pNB203 and used to demonstrate CRISPR/Cas9-mediated inactivation of targeted beta lactamase genes following DNA delivery to bacterial strains carrying such genes. A plasmid map of pNB203 is shown in Figure 28.

### Schematic representation of the structure of concatenated spacer arrays

Spacer sequences were determined to maximise the coverage of the target beta-lactamase gene family. Each unit oligo contains the direct repeat flanking the appropriate spacer sequence at each end. Concatenation reactions are performed between pairwise oligos, *i.e.* the nearest neighbour unit oligos are concatenated first to generate two unit length oligo, then two unit length oligos are concatenated to generate four unit length of oligo etc.

The schematic structure of tetramer and octamer spacer structures are shown in Figure 29. S: spacer, R: direct repeat, A and B contain *Bsa*I site to create ligation compatible sites for cloning into pNB100 and downstream of the first promotor of pNB200. C and D contain a *Sap*I site to create ligation a compatible site for cloning downstream of the second promotor of pNB200. In this example, we employed spacer sequence S1 targeting NDM, S2 targeting IMP, S3 targeting VIM, S4 targeting KPC, S5 targeting OXA, S6 targeting SHV, S7 targeting TEM and S8 targeting CTX-M.

### Spacer concatenation reaction

Each oligo has overlapped sequence in the 3' and 5' end to anneal to the nearest neighbour oligo except the first and the last oligo. The first and the last oligo have the overlapping sequence to the second and the penultimate oligo in the 5' end only. In order to concatenate four spacers, four oligos are synthesised. In other words, oligo No.1 consists spacer 1 and 2 in the 5' and 3' ends. Oligo No.2 contains reverse complement of spacer 2 and 3 in the 3' and 5' ends. Oligo No.3 contains spacer 3 and 4 in the 5' and 3' ends. Oligo No.4 contains reverse complement of spacer 4 in the 3' end. Thus the oligo No.2 can link oligo No.1 and oligo No.3, oligo 4 anneals to 3' end of oligo No.3. Oligo No.1 and oligo No. 2, oligo No. 3 and oligo No. 4 are concatenated in a separate tube using the following PCR reaction conditions.

| **Component** | **A1** | **A2** | **B1** | **B2** | **x 4** |
|---|---|---|---|---|---|
| Nuclease-Free water | 33.5 µL | 33.5 µL | 33.5 µL | 33.5 µL | 134 |
| 5X Q5 Reaction Buffer | 10 µL | 10 µL | 10 µL | 10 µL | 40 |
| 10 mM dNTPs | 1 µL | 1 µL | 1 µL | 1 µL | 4 |
| Q5 High-Fidelity DNA Polymerase | 0.5 µL | 0.5 µL | 0.5 µL | 0.5 µL | 2 |
| 10 µM Forward Primer NB026 | 2.5 µL | | | | |
| 10 µM Reverse Primer NB027 | 2.5 µL | | | | |
| 10 µM Forward Primer NB028 | | 2.5 µL | | | |
| 10 µM Reverse Primer NB034 | | 2.5 µL | | | |
| 10 µM Forward Primer NB035 | | | 2.5 µL | | |
| 10 µM Reverse Primer NB031 | | | 2.5 µL | | |
| 10 µM Forward Primer NB032 | | | | 2.5 µL | |
| 10 µM Reverse Primer NB036 | | | | 2.5 µL | |

### Cycle conditions

| **STEP** | **TEMP** | **TIME** |
|---|---|---|
| Initial Denat. | 98°C | 60 sec |
| 35 cycles | 98°C | 10 sec |
| | 55°C | 10 sec |
| | 72°C | 20 sec |
| Final Extension | 72°C | 2 minutes |
| Hold | 4°C | |

In this example, NB026 and NB027, NB028 and NB034, NB035 and NB031, NB032 and NB036 are concatenated. Each concatenated product A1, A2, B1 and B2 was gel purified and set up the second concatenation reaction using the purified A1 and A2, B1 and B2 dimer product in the following PCR condition.

| **Component** | **A** | **B** | **x 2** |
|---|---|---|---|
| Nuclease-Free water | 35.75 µL | 35.75 µL | 71.5 |
| 10 X PCR Buffer | 5 µL | 5 µL | 10 |
| 10 mM dNTPs | 1 µL | 1 µL | 2 |
| QIAGEN Hot Start Taq | 0.25 µL | 0.25 µL | 0.5 |
| Gel extracted A1 | 4 µL | | |
| Gel extracted A2 | 4 µL | | |
| Gel extracted B1 | | 4 µL | |
| Gel extracted B2 | | 4 µL | |

### Cycle conditions

| **STEP** | **TEMP** | **TIME** |
|---|---|---|
| Initial Denat. | 95°C | 15 min |
| 35 Cycles | 94°C | 30 sec |
| | A: 55 °C | 30 sec |
| | 72°C | 30 sec |
| Final Extension | 72°C | 10 min |

These extention products were amplified by NB037 and NB038 with Q5 DNA polymerase. The final amplicons were cloned to pCR Blunt II TOPO vector and the concatemer sequences were confirmed.

In case of eight spacer concatenation, spacer concatemer A and spacer concatemer B on pCR Blunt II TOPO vector were amplified with primer pairs NB026 and NB029, NB030 and NB033, respectively and amplicons were gel purified. Purified spacer A and B were utilised as a long primer in the following cycle extension reaction.

| **Component** | **A** |
|---|---|
| Nuclease-Free water | 35.75 µL |
| 10 X PCR Buffer | 5 µL |
| 10 mM dNTPs | 1 µL |
| QIAGEN Hot Start Taq | 0.25 µL |
| Gel extracted A | 4 µL |
| Gel extracted B | 4 µL |

### Cycle conditions

| **STEP** | **TEMP** | **TIME** |
|---|---|---|
| Initial Denat. | 95 °C | 15 min |
| 25 Cycles | 94 °C | 30 sec |
| | 55 °C | 30 sec |
| | 72 °C | 30 sec |
| Final Extension | 72 °C | 10 min |
| Hold | 4 °C | o/n |

These extention products were amplified by NB037 and NB038 with Q5 DNA polymerase. The final amplicons were cloned into pCR Blunt II TOPO vector and the concatemer sequences were confirmed.

### Delivery of CRISPR-Cas9 constructs to bacteria

Constructs that are able to inactivate target genes, including antibiotic resistant genes, via the CRISPR-Cas system, and which are an aspect of the present invention are also referred to herein as "Nemesis symbiotics". The CRISPR-Cas9 plasmid derivatives, pNB104A, pNB104B, pNB202 and pNB203 all carry spacer insertions targeted against the selected families of beta-lactamase (*bla*) genes described, and so provide exemplars to demonstrate that a single plasmid construct possesses Nemesis symbiotic activity (NSA) and is therefore able to inactivate representative genes from all 8 different families of beta-lactam antibiotics. The plasmid pNB104A (see Fig. 23) is tested for its ability to inactivate representative genes members of the NDM, IMP, VIM and KPC families; pNB104B (see Fig. 24) and pNB202 (ss Fig. 27) are tested for their ability to inactivate representative genes members of the OXA, SHV, TEM and CTX-M families; and pNB108 (see Fig. 25) and pNB203 (see Fig. 28) are tested for their ability to inactivate representative genes members of the SHV, CTX-M, TEM, KPC, VIM, IMP, NDM and OXA families.

### Nemesis symbiotic activity (NSA) assay by plasmid transformation

The NSA assay described in Example 2 showed that DNA transformation of an *E. coli* strain, DH5α also carrying the TEM-3 beta lactamase gene on the plasmid pBR322, with plasmid pNB102 converts the transformant to ampiciilin sensitivity (ApS). Here, the plasmid pNB102 encodes resistance to chloramphenicol and the DH5α (pBR322) transformants now carrying pNB102 were selected on LB Cm plates and then screened for ApS (see Fig. 14). Here, the plasmid pNB102, in expressing the CRISPR/Cas9 system with the spacer sequence encoding the gRNA targeting the TEM-3 gene, inactivated the TEM-3 gene. In contrast in a negative control experiment, when DH5α (pBR322) was transformed with the parental plasmid pNB100 carrying the expressing the CRISPR/Cas9 system but lacking the gRNA targeting the TEM-3 gene, no conversion to ApS occurred.

For exemplification purposes an equivalent experiment is described where plasmid derivatives of pBR322 are constructed where the TEM-3 is replaced by representative genes from the other 7 different families of beta-lactam antibiotics: SHV, CTX-M, KPC, VIM, IMP, NDM and OXA. Such genes are obtained from suitable bacterial strains carrying such genes. This allows a direct comparison to the proof of concept experiments described in Example 2 in isogenic genetic backgrounds.

A set of *E*. *coli* and *K. pneumoniae* strains carrying representative genes from these seven different families of beta-lactam antibiotics were purchased from Culture Collections, Public Health England, Porton Down, Salisbury, SP4 0JG, UK. These are: NCTC13368, a *K*. *pneumoniae* strain carrying the SHV-18 gene; NCTC13353 an *E. coli* strain carrying the CTX-M-15 gene; NCTC13438 a *K. pneumoniae* strain carrying the KPC-3 gene; NCTC13440 a *K. pneumoniae* strain carrying the VIM-1 gene; NCTC13476 an *E. coli* strain carrying an uncharacterised IMP gene; NCTC13443 a *K. pneumoniae* strain carrying the NDM-1 gene and NCTC13442 a *K. pneumoniae* strain carrying the OXA-48 gene. All seven genes encode beta lactamases that are also able to degrade and inactivate the penam class of antibiotics (see Fig. 16, 17). All strains were tested and, as expected, found to be resistant to the penam class antibiotic, ampicillin.

Beta lactamase coding sequences are amplified from the cell with appropriate forward and reverse primer set shown below:

| Strain | NCTC No. | Resistance gene | Forward primer 5' to 3' | Reverse primer 5' to 3' |
|---|---|---|---|---|
| NBKp001 | 13443 | NDM-1 | | |
| NBKp002 | 13442 | OXA-48 | | |
| NBKp003 | 13368 | SHV-18 | | |
| NBKp004 | 13440 | VIM-1 | | |
| NBKp005 | 13438 | KPC-3 | | |
| NBEc018 | 13476 | IMP-4 | | |
| NBEc019 | 13353 | CTX-M-15 | | |

Each forward primer contains a 17 base sequence to restore the beta-lactamase promoter on pBR322, and each reverse primer contains *Bsa*I site (for NDM-1, OXA-48, SHV-18, KPC-3, IMP-4 and CTX-M15) or *Fok*I site (for VIM-1) to create 5'-ACCG four base protruding 5' end. After amplifying each beta lactamase gene with high fidelity DNA polymerase such as Q5 DNA polymerase, the amplicon is digested with the appropriate restriction enzyme located in the reverse primer, described above. The digested amplicons are ready to ligate using T4 ligase between the Sspl and *Bsa*I sites on the plasmid pBR322 (purchased from New England Biolabs), after removal of the TEM-3 fragment. *Ssp*I creates a blunt end and *Bsa*I creates a 5'-CGGT protruding end. The reverse complement of the coding sequences of the each amplicons after restriction digestion are shown below. The 5' protruding end is underlined and 3' end of the promotor sequence is in bold small letters. The reverse complement CAT of the methionine initiating codons ATG of these seven genes, also shown in bold, yields a precise fusion of the coding region of the seven other beta-lactamases to the translational signal sequences of the TEM-3 beta-lactamase of pBR322.
NDM-1 (SEQ ID NO: 166)
OXA-48 (SEQ ID NO: 167)
SHV-18 (SEQ ID NO: 168)
VIM-1 (SEQ ID NO: 169)
KPC-3 (SEQ ID NO: 170)
IMP-4 (SEQ ID NO: 171)
CTX-M-15 (SEQ ID NO: 172)

Then DH5α competent cells purchased from New England Biolabs are transformed with these ligations followed by selection for the desired recombinants on LB Ampicillin (100µg/mL) plates.

Plasmid DNA samples are isolated from these transformants and submitted to DNA sequence analysis to confirm that the correct sequence for each of the seven different beta lactamases genes is present in each construct giving the plasmids:
These pBR322 derivative plasmids so derived are named:
i. pNB010 carrying the SHV-18 gene;
ii. pNB011 carrying the CTX-M-15 gene;
iii. pNB012 carrying the KPC-3 gene;
iv. pNB013 carrying the VIM-1 gene;
v. pNB014 carrying the IMP gene;
vi. pNB015 carrying the NDM-1 gene;
vii. pNB016 carrying the OXA-48 gene; in addition to, as described,
viii. pBR322 carrying the TEM-3 gene

Then 7 recipient *E*. *coli* strains, DH5α, each carrying one of these seven beta lactamase genes on the plasmids pNB010-016 are subsequently transformed with the plasmids pNB104A, pNB104B, pNB108 and pNB203 and selected for chloramphenicol resistance to select for acquisition of these Nemesis symbiotic plasmids, along with the negative control pNB100 as well as with transformation of DH5α (pBR322) by pNB102 as the positive control, and then tested for conversion to ampicillin sensitivity as described in Example 2 (see Fig 14.)

### "Nemesis symbiotic activity" (NSA) assay by plasmid conjugation to test activity of constructs carrying multiple spacers targeting beta lactamase gene families

The plasmid conjugation assay described in Example 2 may also be used to test the Nemesis symbiotic activity of the new CRISPR/Cas9 plasmid constructs carrying spacer sequences targeting multiple families of beta lactamase genes. The assay involves mating a donor cell carrying the beta lactamase gene on a conjugative plasmid, and hence ampicillin resistant, with a recipient cell carrying the Nemesis symbiotic on a non-mobilisable plasmid encoding chloramphenicol resistance. Exconjugants are selected on LB plates containing both 100µg/mL ampicillin and 35µg/mL chloramphenicol. Successful Nemesis symbiotic activity is seen in a reduction in the efficiency of transfer of the ampicillin resistance gene.

As donors, the same strain used in Example 2 was used. This strain, JA200 (F+ thr-1, leu-6, DE(trpE)5, recA, lacY, thi, gal, xyl, ara, mtl), also carries plasmid pNT3 as described by Saka et al. (DNA Research 12, 63-68, 2005). The plasmid pNT3 is a mobilisable plasmid carrying the TEM-1 beta lactamase gene of Tn1. Conjugation of pNT3 and hence transfer of ampicillin resistance is effected by the transfer functions of the co-resident F+ plasmid.

The other potential donors are the set of *E. coli* and *K. pneumoniae* strains carrying representative genes from these seven different families of beta-lactam antibiotics that were purchased from Culture Collections, Public Health England, as described above. These strains need to be chloramphenicol sensitive in order to allow selection for exconjugants and were tested for growth on LB chloramphenicol 35µg/mL plates. NCTC13368, a *K. pneumoniae* strain carrying the SHV-18 gene; NCTC13438 a *K*. *pneumoniae* strain carrying the KPC-3 gene; NCTC13476 an *E. coli* strain carrying an uncharacterised IMP gene; NCTC13443 a *K. pneumoniae* strain carrying the NDM-1 gene and NCTC13442 a *K. pneumoniae* strain carrying the OXA-48 gene were all found to be resistant to chloramphenicol, but NCTC13353, an *E. coli* strain carrying the CTX-M-15 gene, and NCTC13440, a *K. pneumoniae* strain carrying the VIM-1 gene, were found to be chloramphenicol sensitive and were taken forward to be tested in matings with the recipients.

As recipients, the strains used in Example 2 serve as controls for testing the new plasmid constructs. Thus the negative control is DH5α (F- endA1 glnV44 thi-1 recA1 relA1 gyrA96 deoR nupG Φ80dlacZΔM15 Δ(lacZYA-argF)U169, hsdR17(rK- mK+), λ with the plasmid pNB100 encoding the CRISPR/Cas9 cassette but no spacer sequence targeting any antibiotic resistance gene; and the positive control is DH5α with the plasmid pNB102 encoding the CRISPR/Cas9 cassette as well as the spacer sequence targeting the TEM beta-lactamase family. To be tested are DH5α strains with the plasmids pNB104A, pNB104B and pNB108. These plasmids also encode the CRISPR/Cas9 cassette in addition to the spacer sequences, all driven off one promotor in the following order, proximal to distal from the promotor (pNB104A): NDM-IMP-VIM-KPC; (pNB104B): OXA-48-SHV-TEM-CTX-M (pNB108): NDM-IMP-VIM-KPC-OXA-48-SHV-TEM-CTX-M.

To set up the matings, colonies of donors were picked from LB Ap100 (ampicillin 100µg/mL) and recipients from LB Cm35 (chloramphenicol 35µg/mL) plates into 200µL of LB and then mixed 100µL of recipients with 35µL of donors, then 5µL of the mixture were spotted onto LB plates and incubated at 37ºC for 5 hours to allow mating.

Figure 30A shows the results from mating the donor carrying the TEM-1 beta lactamase (JA200 (F+ thr-1, leu-6, DE(trpE)5, recA, lacY, thi, gal, xyl, ara, mtl)), and designated (5) in the figure, with the recipients DH5α pNB100, designated (-), DH5α pNB102, designated (+). Here a loopful of each mating mixture was resuspended in 220µL of LB and 200µL of the cells were plated on LB Ap100Cm35 plates to select for exconjugants. As expected from Example 2, the cross between the donor of TEM-1 and the recipient with pNB100 gives efficient mating and a lawn of cells (Fig. 30A, top left, 5-), and mating the donor of TEM-1 with the recipient with pNB102 encoding the single TEM spacer shows strong inhibition of transfer of the TEM-1 gene (see Fig. 30A, 5+, top right, where only a few isolated colonies are seen). The mating of the donor carrying the TEM-1 gene with the recipient carrying pNB104 (see bottom plate, 5B, of the figure) also shows strong inhibition of transfer of the TEM-1 gene and demonstrates that in the pNB104B construct, carrying the four spacers OXA-48-SHV-TEM-CTX-M 4, the TEM spacer is active.

Figure 30B shows the results from mating the strain NCTC13440, a *K. pneumoniae* strain carrying the VIM-1, designated (2), and the strain NCTC13353, an *E. coli* strain carrying the CTX-M-15 gene, designated (4). Again a loopful of each such mating mixture was resuspended in 220µL of LB and 200µL of the cells were plated on LB Ap100Cm35 plates to select for exconjugants. The results show that these strains are able to act as donors and transfer the VIM-1 and the CTX-M-15 genes respectively to the DH5α pNB100 negative control recipient lacking Nemesis symbiotic activity (see Fig. 30B, top left and top right respectively). However transfer of the VIM-1 and the CTX-M-15 genes was found to be strongly inhibited in matings with recipients carrying spacers targeted against these genes: pNB104A and pNB104B (see plates 2A and 4B, bottom left and right respectively).

In another experiment, an equivalent mating was done to test Nemesis symbiotic activity in recipients carrying all 8 spacer sequences in the plasmid pNB108. Here cells were picked directly from the mating mixture and streaked onto LBAp100Cm35 plates. Figure 30C plate, top left, again shows successful mating of donors 2, 4 and 5 with the recipient carrying pNB100 (see 2^{_},4^{_} and 5^{_} in Fig. 30C). In all cases, however, mating with the recipient carrying pNB108 (see Fig. 30C, plate top right, 2/8, 4/8 and 5/8) gives strong inhibition of transfer of ampicillin resistance gene. And for comparison to a positive control in this assay the bottom plate with 5+ (see Fig. 30C) shows strong inhibition of transfer of TEM-1 ampicillin resistance gene to the recipient carrying pNB102.

The experiments reported above provide the proof-of-concept that, in the model organism, *Escherichia coli,* DNA constructs carrying the Cas9 CRISPR region plus a spacer region with sequences directed against a target region of the beta-lactamase gene can inactivate ampicillin resistance when delivered by naked DNA transformation and bacteriophage infection as well as prevent transfer of ampicillin resistance by plasmid conjugation. It is apparent that Nemesis symbiotics of the invention can be applied to pathogenic bacteria and for other antibiotic resistance genes.

## Claims

1. A delivery vehicle for introducing a polynucleotide into an antibiotic-resistant microorganism, wherein the delivery vehicle is selected from the group consisting of a plasmid (such as a conjugative plasmid or other plasmid replicon), a nucleotide vector, linear double-stranded DNA, a non-virulent bacteriophage and a lysogenic bacteriophage, and comprises a recombinant polynucleotide for inactivation of DNA carrying at least one antibiotic resistance gene encoding an enzyme which confers antibiotic resistance to a microrganism, wherein the recombinant polynucleotide comprises a clustered regularly interspaced short palindromic repeat (CRISPR) array nucleic acid sequence having or transcribing multiple RNA guide molecules, wherein each RNA guide molecule:
(i) is transcribed from its own promoter sequence;
(ii) mediates the binding of a CRISPR associated (Cas) DNA-binding polypeptide or its functional equivalent or its modified version thereof to the at least one antibiotic resistance gene(s); and
(iii) has a spacer sequence sufficiently complementary to a target DNA sequence of the at least one antibiotic resistance gene for the at least one antibiotic resistance gene to be targeted and inactivated in the presence of a CRISPR associated (Cas) DNA-binding polypeptide or a functional equivalent or a modified version thereof, and wherein:
either the Cas DNA-binding polypeptide or a functional equivalent or a modified version thereof is a modified Cas DNA-binding polypeptide which creates a deletion and reseals the target DNA sequence to inactivate the at least one antibiotic resistance gene to prevent double-strand break (DSB)-induced killing of the microorganism, such that the microorganism becomes sensitised to an antibiotic, or
the Cas DNA-binding polypeptide or a functional equivalent or a modified version thereof creates a DSB in the target DNA sequence to inactivate the at least one antibiotic resistance gene, and the delivery vehicle further comprises a nucleotide sequence encoding a gene product which prevents direct killing of the microorganism due to the generation of the DSB in the at least one antibiotic resistance gene, such that the microorganism becomes sensitised to an antibiotic.

2. The delivery vehicle according to claim 1, further comprising a nucleic acid sequence which encodes the Cas DNA-binding polypeptide or its functional equivalent or its modified version.

3. The delivery vehicle according to either of claim 1 or claim 2, in which the Cas DNA-binding polypeptide is Cas9 or a functional equivalent or a modified version thereof.

4. The delivery vehicle according to any of the preceding claims, comprising a further RNA guide molecule which targets a gene involved in pathogenicity or other aspects of microbial metabolism, for example a gene involved in bacterial metabolism for biofilm production.

5. The delivery vehicle according to any of the preceding claims, in which the modified Cas DNA-binding polypeptide comprises a recombinase catalytic domain to reseal target DNA sequence.

6. The delivery vehicle according to any of claims 1 to 4, wherein the gene product that prevents direct killing of the microorganism is a protein encoded by a replicon subject to degradation due to the DSB caused by the Cas DNA-binding polypeptide, or an antitoxin that neutralises the effect of a toxin or killer function carried by a replicon on which the target DNA sequence is located.

7. The delivery vehicle according to any of the preceding claims, in which the CRISPR array nucleic acid sequence has or transcribes one or more RNA guide molecules each comprising a spacer sequence sufficiently complementary to a target sequence of one or more beta-lactamase genes, for example one or more or all of the genes selected from the group consisting of: NDM, VIM, IMP, KPC, OXA, TEM, SHV, CTX, OKP, LEN, GES, MIR, ACT, ACC, CMY, LAT, and FOX.

8. A composition comprising the delivery vehicle according to any of claims 1 to 7.

9. The composition according to claim 8, in which the composition is a pharmaceutical composition, a non-pathogenic microorganism such as a commensal bacterium for example in a probiotic formulation, or a dietary supplement.

10. The composition according to either of claims 8 or 9, formulated for topical, enteral or parenteral administration.

11. The composition according to any of claims 8 to 10, for use as a medicament.

12. The composition according to any of claims 8 to 11, for use in the treatment or prevention of an infection caused by an antibiotic-resistant microorganism comprising one or more antibiotic resistance genes targeted by the RNA guide molecules of the recombinant polynucleotide.

13. A method of inactivating antibiotic resistance in an antibiotic-resistant microorganism, the method comprising introducing into the microorganism the delivery vehicle according to any of claims 1 to 7.

14. A host cell comprising the recombinant polynucleotide as defined in any of claims 1 to 7.

15. The host cell according to claim 14, in which the host cell is a commensal bacterium.

## Patentansprüche

1. Zuführungsvehikel zum Einführen eines Polynucleotids in einen antibiotikaresistenten Mikroorganismus, wobei das Zuführungsvehikel ausgewählt ist aus der Gruppe bestehend aus einem Plasmid (wie ein konjugatives Plasmid oder ein anderes Plasmid-Replikon), einem Nucleotidvektor, linearer doppelsträngiger DNA, einem nicht virulenten Bakteriophagen und einem lysogenen Bakteriophagen, und ein rekombinantes Polynucleotid zur Inaktivierung von DNA beinhaltet, die wenigstens ein Antibiotikaresistenzgen trägt, das ein Enzym kodiert, das einem Mikroorganismus Antibiotikaresistenz verleiht, wobei das rekombinante Polynucleotid eine CRISPR-(Clustered Regularly Interspaced Short Palindromic Repeat)-Array-Nucleinsäuresequenz beinhaltet, die mehrere RNA-Führungsmoleküle hat oder transkribiert, wobei jedes RNA-Führungsmolekül:
(i) von seiner eigenen Promotorsequenz transkribiert wird;
(ii) die Bindung eines CRISPR-assoziierten (CAS) DNA-Bindungspolypeptids oder eines funktionellen Äquivalents oder einer modifizierten Version davon an das wenigstens eine Antibiotikaresistenzgen vermittelt; und
(iii) eine Spacer-Sequenz hat, die ausreichend komplementär zu einer Target-DNA-Sequenz des wenigstens einen Antibiotikaresistenzgens ist, damit das wenigstens eine Antibiotikaresistenzgen anvisiert und in Anwesenheit eines CRISPR-assoziierten (Cas) DNA-Bindungspolypeptids oder eines funktionellen Äquivalents oder einer modifizierten Version davon inaktiviert werden kann, und wobei:
das Cas DNA-Bindungspolypeptid oder ein funktionelles Äquivalent oder eine modifizierte Version davon ein modifiziertes Cas DNA-Bindungspolypeptid ist, das eine Deletion erzeugt und die Target-DNA-Sequenz erneut versiegelt, um das wenigstens eine Antibiotikaresistenzgen zu inaktivieren, um eine Doppelstrangbruch-(DSB)-induzierte Abtötung des Mikroorganismus zu verhindern, so dass der Mikroorganismus gegenüber einem Antibiotika sensibilisiert wird, oder
das Cas DNA-Bindungspolypeptid oder ein funktionelles Äquivalent oder eine modifizierte Version davon einen DSB in der Target-DNA-Sequenz erzeugt, um das wenigstens eine Antibiotikaresistenzgen zu inaktivieren, und das Zuführungsvehikel ferner eine Nucleotidsequenz umfasst, die ein Genprodukt kodiert, das eine direkte Abtötung des Mikroorganismus infolge der Erzeugung des DSB in dem wenigstens einen Antibiotikaresistenzgen verhindert, so dass der Mikroorganismus gegenüber einem Antibiotika sensibilisiert wird.

2. Zuführungsvehikel nach Anspruch 1, das ferner eine Nucleinsäuresequenz beinhaltet, die das Cas DNA-Bindungspolypeptid oder sein funktionelles Äquivalent oder seine modifizierte Version kodiert.

3. Zuführungsvehikel nach Anspruch 1 oder Anspruch 2, wobei das Cas DNA-Bindungspolypeptid Cas9 oder ein funktionelles Äquivalent oder eine modifizierte Version davon ist.

4. Zuführungsvehikel nach einem der vorherigen Ansprüche, das ein weiteres RNA-Führungsmolekül beinhaltet, das ein Gen anvisiert, das in Pathogenität oder anderen Aspekten des mikrobiellen Metabolismus involviert ist, zum Beispiel ein Gen, das im bakteriellen Metabolismus zur Biofilmproduktion involviert ist.

5. Zuführungsvehikel nach einem der vorherigen Ansprüche, wobei das modifizierte Cas DNA-Bindungspolypeptid eine Rekombinase-Katalysedomäne beinhaltet, um die Target-DNA-Sequenz erneut zu versiegeln.

6. Zuführungsvehikel nach einem der Ansprüche 1 bis 4, wobei das Genprodukt, das die direkte Abtötung des Mikroorganismus verhindert, ein Protein ist, das durch ein Replikon kodiert ist, das aufgrund eines DSB infolge des Cas DNA-Bindungspolypeptids einem Abbau unterliegt, oder ein Antitoxin ist, das den Effekt eines Toxins oder einer Killerfunktion neutralisiert, das/die von einem Replikon getragen wird, auf dem sich die Target-DNA-Sequenz befindet.

7. Zuführungsvehikel nach einem der vorherigen Ansprüche, wobei die CRISPR-Array-Nucleinsäuresequenz ein oder mehrere RNA-Führungsmoleküle hat oder transkribiert, die jeweils eine Spacer-Sequenz beinhalten, die ausreichend komplementär zu einer Targetsequenz von einem oder mehreren Beta-Lactamasegenen ist, zum Beispiel eines oder mehrere oder alle der Gene, die ausgewählt sind aus der Gruppe bestehend aus: NDM, VIM, IMP, KPC, OXA, TEM, SHV, CTX, OKP, LEN, GES, MIR, ACT, ACC, CMY, LAT und FOX.

8. Zusammensetzung, die das Zuführungsvehikel nach einem der Ansprüche 1 bis 7 beinhaltet.

9. Zusammensetzung nach Anspruch 8, wobei die Zusammensetzung eine pharmazeutische Zusammensetzung, ein nicht pathogener Mikroorganismus wie ein kommensales Bakterium zum Beispiel in einer probiotischen Formulierung, oder ein Nahrungsergänzungsmittel ist.

10. Zusammensetzung nach Anspruch 8 oder 9, die für eine topische, enterale oder parenterale Verabreichung formuliert ist.

11. Zusammensetzung nach einem der Ansprüche 8 bis 10 zur Verwendung als Medikament.

12. Zusammensetzung nach einem der Ansprüche 8 bis 11 zur Verwendung bei der Behandlung oder Verhütung einer Infektion, die durch einen antibiotikaresistenten Mikroorganismus verursacht wird, der ein oder mehrere Antibiotikaresistenzgene beinhaltet, die durch die RNA-Führungsmoleküle des rekombinanten Polynucleotids anvisiert werden.

13. Verfahren zum Inaktivieren von Antibiotikaresistenz in einem antibiotikaresistenten Mikroorganismus, wobei das Verfahren das Einführen des Zuführungsvehikels nach einem der Ansprüche 1 bis 7 in den Mikroorganismus beinhaltet.

14. Wirtszelle, die das rekombinante Polynucleotid nach einem der Ansprüche 1 bis 7 beinhaltet.

15. Wirtszelle nach Anspruch 14, wobei die Wirtszelle ein kommensales Bakterium ist.

## Revendications

1. Véhicule d'administration destiné à introduire un polynucléotide dans un micro-organisme résistant aux antibiotiques, dans lequel le véhicule d'administration est choisi dans le groupe constitué d'un plasmide (tel qu'un plasmide conjugatif ou un autre réplicon de plasmide), un vecteur nucléotidique, de l'ADN linéaire double brin, un bactériophage non virulent et un bactériophage lysogène, et comprend un polynucléotide recombinant pour l'inactivation de l'ADN portant au moins un gène de résistance aux antibiotiques codant pour une enzyme qui confère une résistance aux antibiotiques à un micro-organisme, dans lequel le polynucléotide recombinant comprend une séquence d'acide nucléique de réseau de courtes répétitions palindromiques groupées et régulièrement espacées (CRISPR) ayant ou transcrivant plusieurs molécules guides d'ARN, dans lequel chaque molécule guide d'ARN :
(i) est transcrite à partir de sa propre séquence promotrice ;
(ii) assure la médiation de la liaison d'un polypeptide de liaison à l'ADN (Cas) associé à CRISPR ou de son équivalent fonctionnel ou de sa version modifiée à l'au moins un gène de résistance aux antibiotiques ; et
(iii) présente une séquence d'espacement suffisamment complémentaire à une séquence d'ADN cible de l'au moins un gène de résistance aux antibiotiques pour que l'au moins un gène de résistance aux antibiotiques soit ciblé et inactivé en présence d'un polypeptide de liaison à l'ADN (Cas) associé à CRISPR ou d'un équivalent fonctionnel ou d'une version modifiée de celui-ci, et dans lequel :
soit le polypeptide de liaison à l'ADN Cas, soit un équivalent fonctionnel ou une version modifiée de celui-ci est un polypeptide de liaison à l'ADN Cas qui crée une délétion et referme la séquence d'ADN cible pour inactiver l'au moins un gène de résistance aux antibiotiques afin d'empêcher la destruction induite par cassure double brin (DBS) du micro-organisme, de telle sorte que le micro-organisme devient sensibilisé à un antibiotique, ou que
le polypeptide de liaison à l'ADN Cas ou un équivalent fonctionnel ou une version modifiée de celui-ci crée un DSB dans la séquence d'ADN cible pour inactiver l'au moins un gène de résistance aux antibiotiques, et le véhicule d'administration comprend en outre une séquence nucléotidique codant pour un produit génique qui empêche la destruction directe du micro-organisme en raison de la génération du DSB dans l'au moins un gène de résistance aux antibiotiques, de telle sorte que le micro-organisme devient sensibilisé à un antibiotique.

2. Véhicule d'administration selon la revendication 1, comprenant en outre une séquence d'acide nucléique qui code pour le polypeptide de liaison à l'ADN Cas ou son équivalent fonctionnel ou sa version modifiée.

3. Véhicule d'administration selon la revendication 1 ou la revendication 2, dans lequel le polypeptide de liaison à l'ADN Cas est Cas9 ou un équivalent fonctionnel ou une version modifiée de celui-ci.

4. Véhicule d'administration selon l'une quelconque des revendications précédentes, comprenant une autre molécule guide d'ARN qui cible un gène impliqué dans la pathogénicité ou d'autres aspects du métabolisme microbien, par exemple un gène impliqué dans le métabolisme bactérien pour la production de biofilm.

5. Véhicule d'administration selon l'une quelconque des revendications précédentes, dans lequel le polypeptide de liaison à l'ADN Cas modifié comprend un domaine catalytique de recombinase pour refermer la séquence d'ADN cible.

6. Véhicule d'administration selon l'une quelconque des revendications 1 à 4, dans lequel le produit génique qui empêche la destruction directe du micro-organisme est une protéine codée par un réplicon soumis à une dégradation due au DSB causée par le polypeptide de liaison à l'ADN Cas, ou une antitoxine qui neutralise l'effet d'une fonction toxine ou destructrice portée par un réplicon sur lequel se trouve la séquence d'ADN cible.

7. Véhicule d'administration selon l'une quelconque des revendications précédentes, dans lequel la séquence d'acide nucléique du réseau CRISPR a ou transcrit une ou plusieurs molécules guides d'ARN comprenant chacune une séquence d'espacement suffisamment complémentaire à une séquence cible d'un ou plusieurs gènes de bêta-lactamase, par exemple un ou plusieurs ou tous les gènes choisis dans le groupe constitué de: NDM, VIM, IMP, KPC, OXA, TEM, SHV, CTX, OKP, LEN, GES, MIR, ACT, ACC, CMY, LAT et FOX.

8. Composition comprenant le véhicule d'administration selon l'une quelconque des revendications 1 à 7.

9. Composition selon la revendication 8, dans laquelle la composition est une composition pharmaceutique, un micro-organisme non pathogène tel qu'une bactérie commensale, par exemple dans une formulation probiotique, ou un complément alimentaire.

10. Composition selon l'une des revendications 8 ou 9, formulée pour une administration topique, entérale ou parentérale.

11. Composition selon l'une quelconque des revendications 8 à 10, destinée à être utilisée comme médicament.

12. Composition selon l'une quelconque des revendications 8 à 11, destinée à être utilisée dans le traitement ou la prévention d'une infection causée par un micro-organisme résistant aux antibiotiques comprenant un ou plusieurs gènes de résistance aux antibiotiques ciblés par les molécules guides d'ARN du polynucléotide recombinant.

13. Procédé d'inactivation de la résistance aux antibiotiques dans un micro-organisme résistant aux antibiotiques, le procédé comprenant l'introduction dans le micro-organisme du véhicule d'administration selon l'une quelconque des revendications 1 à 7.

14. Cellule hôte comprenant le polynucléotide recombinant tel que défini dans l'une quelconque des revendications 1 à 7.

15. Cellule hôte selon la revendication 14, dans laquelle la cellule hôte est une bactérie commensale.


### SEQUENCE LISTING

<110> Nemesis Bioscience Ltd
<120> Therapeutic
<130> P/71337.WO01/MAR
<150> GB1406674.0
   <151> 2014-04-14
<150> GB1413719.4
   <151> 2014-08-01
<150> GB1418508.6
   <151> 2014-10-17
<160> 172
<170> PatentIn version 3.5
<210> 1
   <211> 12
   <212> DNA
   <213> Unknown
<220>
   <223> CR05 bla gene sequence
<400> 1
   gatacgggag gg 12
<210> 2
   <211> 12
   <212> DNA
   <213> Unknown
<220>
   <223> CR05 bla gene reverse strand
<400> 2
   ccctcccgta tc 12
<210> 3
   <211> 10
   <212> DNA
   <213> Unknown
<220>
   <223> CR05 short bacterial off-target sequence
<400> 3
   cgatacggga 10
<210> 4
   <211> 11
   <212> DNA
   <213> Unknown
<220>
   <223> CR05 short bacterial off-target sequence
<400> 4
   cgatacggga g 11
<210> 5
   <211> 11
   <212> DNA
   <213> Unknown
<220>
   <223> CR05 short bacterial off-target sequence
<400> 5
   gatacgggag g 11
<210> 6
   <211> 19
   <212> DNA
   <213> Unknown
<220>
   <223> CR30 bla gene sequence
<400> 6
   tgctcatcat tggaaaacg 19
<210> 7
   <211> 19
   <212> DNA
   <213> Unknown
<220>
   <223> CR30 bla gene sequence reverse strand
<400> 7
   cgttttccaa tgatgagca 19
<210> 8
   <211> 12
   <212> DNA
   <213> Unknown
<220>
   <223> CR30 short bacterial off-target sequence
<400> 8
   tcattggaaa ac 12
<210> 9
   <211> 10
   <212> DNA
   <213> Unknown
<220>
   <223> CR30 short bacterial off-target sequence
<400> 9
   tgctcatcat 10
<210> 10
   <211> 11
   <212> DNA
   <213> Unknown
<220>
   <223> CR30 short bacterial off-target sequence - 3
<400> 10
   gctcatcatt g 11
<210> 11
   <211> 13
   <212> DNA
   <213> Unknown
<220>
   <223> CR30 short bacterial off-target sequence
<400> 11
   ctcatcattg gaa 13
<210> 12
   <211> 17
   <212> DNA
   <213> Unknown
<220>
   <223> CR30 short bacterial off-target sequence
<400> 12
   ctcagcattg caaaacg 17
<210> 13
   <211> 15
   <212> DNA
   <213> Unknown
<220>
   <223> CR30 short bacterial off-target sequence
<400> 13
   tcatcattga aaaac 15
<210> 14
   <211> 12
   <212> DNA
   <213> Unknown
<220>
   <223> CR30 short bacterial off-target sequence
<400> 14
   tcattggaaa ac 12
<210> 15
   <211> 19
   <212> DNA
   <213> Unknown
<220>
   <223> CR70 bla gene sequence
<400> 15
   tcgccagtta atagtttgc 19
<210> 16
   <211> 19
   <212> DNA
   <213> Unknown
<220>
   <223> CR70 bla sequence reverse strand
<400> 16
   agcggtcaat tatcaaacg 19
<210> 17
   <211> 11
   <212> DNA
   <213> Unknown
<220>
   <223> CR30 short bacterial off-target sequence - 1
<400> 17
   tcgccagtta a 11
<210> 18
   <211> 12
   <212> DNA
   <213> Unknown
<220>
   <223> CR70 short bacterial off-target sequence
<400> 18
   tcgccagtta at 12
<210> 19
   <211> 11
   <212> DNA
   <213> Unknown
<220>
   <223> CR70 short bacterial off-target sequence
<400> 19
   cgccagttaa t 11
<210> 20
   <211> 12
   <212> DNA
   <213> Unknown
<220>
   <223> CR70 short bacterial off-target sequence
<400> 20
   cgccagttaa ta 12
<210> 21
   <211> 15
   <212> DNA
   <213> Unknown
<220>
   <223> CR70 short bacterial off-target sequence
<400> 21
   cgccagctaa tagtt 15
<210> 22
   <211> 11
   <212> DNA
   <213> Unknown
<220>
   <223> CR70 short bacterial off-target sequence
<400> 22
   ccagttaata g 11
<210> 23
   <211> 11
   <212> DNA
   <213> Unknown
<220>
   <223> CR70 short bacterial off-target sequence
<400> 23
   taatagtttg c 11
<210> 24
   <211> 20
   <212> DNA
   <213> Unknown
<220>
   <223> CR90 bla sequence
<400> 24
   ccgcgccaca tagcagaact 20
<210> 25
   <211> 20
   <212> DNA
   <213> Unknown
<220>
   <223> CR90 bla sequence reverse strand
<400> 25
   agttctgcta tgtggcgcgg 20
<210> 26
   <211> 11
   <212> DNA
   <213> Unknown
<220>
   <223> CR90 short bacterial off-target sequence
<400> 26
   acatagcaga a 11
<210> 27
   <211> 11
   <212> DNA
   <213> Unknown
<220>
   <223> CR90 short bacterial off-target sequence
<400> 27
   cgcgccacat a 11
<210> 28
   <211> 13
   <212> DNA
   <213> Unknown
<220>
   <223> CR90 short bacterial off-target sequence
<400> 28
   cgcgccacat agc 13
<210> 29
   <211> 11
   <212> DNA
   <213> Unknown
<220>
   <223> CR90 short bacterial off-target sequence
<400> 29
   gcgccacata g 11
<210> 30
   <211> 11
   <212> DNA
   <213> Unknown
<220>
   <223> CR90 short bacterial off-target sequence
<400> 30
   gccacatagc a 11
<210> 31
   <211> 11
   <212> DNA
   <213> Unknown
<220>
   <223> CR90 short bacterial off-target sequence
<400> 31
   acatagcaga a 11
<210> 32
   <211> 11
   <212> DNA
   <213> Unknown
<220>
   <223> CR90 short bacterial off-target sequence
<400> 32
   catagcagaa c 11
<210> 33
   <211> 16
   <212> DNA
   <213> Unknown
<220>
   <223> CR90 short bacterial off-target sequence
<400> 33
   gcgccatata gcagaa 16
<210> 34
   <211> 20
   <212> RNA
   <213> Artificial Sequence
<220>
   <223> Artificial RNA sequence
<220>
   <221> stem_loop
   <222> (1)..(10)
<400> 34
   uagauaacua cgauacggga 20
<210> 35
   <211> 20
   <212> RNA
   <213> Artificial sequence
<220>
   <223> Artificial RNA sequence
<220>
   <221> stem_loop
   <222> (1)..(11)
<400> 35
   acuuuaaaag ugcucaucau 20
<210> 36
   <211> 20
   <212> RNA
   <213> Artificial sequence
<220>
   <223> Artificial RNA sequence
<220>
   <221> stem_loop
   <222> (3)..(13)
<400> 36
   acguugcgca aacuauuaac 20
<210> 37
   <211> 20
   <212> RNA
   <213> Artificial sequence
<220>
   <223> Artificial RNA sequence
<220>
   <221> stem_loop
   <222> (9)..(17)
<400> 37
   acuuuuaaag uucugcuaug 20
<210> 38
   <211> 861
   <212> DNA
   <213> Unknown
<220>
   <223> Beta-lactamase gene from pBR322
<400> 38
   atgagtattc aacatttccg tgtcgccctt attccctttt ttgcggcatt ttgccttcct      60
   gtttttgctc acccagaaac gctggtgaaa gtaaaagatg ctgaagatca gttgggtgca     120
   cgagtgggtt acatcgaact ggatctcaac agcggtaaga tccttgagag ttttcgcccc     180
   gaagaacgtt ttccaatgat gagcactttt aaagttctgc tatgtggcgc ggtattatcc     240
   cgtgttgacg ccgggcaaga gcaactcggt cgccgcatac actattctca gaatgacttg     300
   gttgagtact caccagtcac agaaaagcat cttacggatg gcatgacagt aagagaatta     360
   tgcagtgctg ccataaccat gagtgataac actgcggcca acttacttct gacaacgatc     420
   ggaggaccga aggagctaac cgcttttttg cacaacatgg gggatcatgt aactcgcctt     480
   gatcgttggg aaccggagct gaatgaagcc ataccaaacg acgagcgtga caccacgatg     540
   cctgcagcaa tggcaacaac gttgcgcaaa ctattaactg gcgaactact tactctagct     600
   tcccggcaac aattaataga ctggatggag gcggataaag ttgcaggacc acttctgcgc     660
   tcggcccttc cggctggctg gtttattgct gataaatctg gagccggtga gcgtgggtct     720
   cgcggtatca ttgcagcact ggggccagat ggtaagccct cccgtatcgt agttatctac     780
   acgacgggga gtcaggcaac tatggatgaa cgaaatagac agatcgctga gataggtgcc     840
   tcactgatta agcattggta a                                               861
<210> 39
   <211> 20
   <212> DNA
   <213> Unknown
<220>
   <223> Spacer sequence
<400> 39
   ccgcgtaggc atgatagaaa 20
<210> 40
   <211> 20
   <212> DNA
   <213> Unknown
<220>
   <223> Spacer sequence
<400> 40
   acgttaaaca ccgccattcc 20
<210> 41
   <211> 20
   <212> DNA
   <213> Unknown
<220>
   <223> Spacer sequence
<400> 41
   gcgctggaga aaagcagcgg 20
<210> 42
   <211> 20
   <212> DNA
   <213> Unknown
<220>
   <223> Spacer sequence
<400> 42
   aagctgattg cccatctggg 20
<210> 43
   <211> 20
   <212> DNA
   <213> Unknown
<220>
   <223> Spacer sequence
<400> 43
   acgctcaaca ccgcgatccc 20
<210> 44
   <211> 20
   <212> DNA
   <213> Unknown
<220>
   <223> Spacer sequence
<400> 44
   aactacttac tctagcttcc 20
<210> 45
   <211> 20
   <212> DNA
   <213> Unknown
<220>
   <223> Spacer sequence
<400> 45
   ttgttgctga aggagttggg 20
<210> 46
   <211> 20
   <212> DNA
   <213> Unknown
<220>
   <223> Spacer sequence
<400> 46
   agcgaaaaac accttgccga 20
<210> 47
   <211> 20
   <212> DNA
   <213> Unknown
<220>
   <223> Spacer sequence
<400> 47
   ctgggaaacg gcactgaatg 20
<210> 48
   <211> 20
   <212> DNA
   <213> Unknown
<220>
   <223> Spacer sequence
<400> 48
   tgggttgttg gagagaaaac 20
<210> 49
   <211> 20
   <212> DNA
   <213> Unknown
<220>
   <223> Spacer sequence
<400> 49
   aaacacagcg gcacttctcg 20
<210> 50
   <211> 20
   <212> DNA
   <213> Unknown
<220>
   <223> Spacer sequence
<400> 50
   aaaattgaag ttttttatcc 20
<210> 51
   <211> 20
   <212> DNA
   <213> Unknown
<220>
   <223> Spacer sequence
<400> 51
   tggcagccgc agtggaagcc 20
<210> 52
   <211> 20
   <212> DNA
   <213> Unknown
<220>
   <223> Spacer sequence
<400> 52
   tcacagctac ttgaaggttc 20
<210> 53
   <211> 20
   <212> DNA
   <213> Unknown
<220>
   <223> Spacer sequence
<400> 53
   atcaaaactg gcagccgcaa 20
<210> 54
   <211> 20
   <212> DNA
   <213> Unknown
<220>
   <223> Spacer sequence
<400> 54
   atcaaaactg gcagccgcaa 20
<210> 55
   <211> 20
   <212> DNA
   <213> Unknown
<220>
   <223> Spacer sequence
<400> 55
   cagtactcca accccagcat 20
<210> 56
   <211> 20
   <212> DNA
   <213> Unknown
<220>
   <223> Spacer sequence
<400> 56
   tcacctggcc gcaaatagtc 20
<210> 57
   <211> 20
   <212> DNA
   <213> Unknown
<220>
   <223> Spacer sequence
<400> 57
   acaacggatt aacagaagca 20
<210> 58
   <211> 20
   <212> DNA
   <213> Unknown
<220>
   <223> Spacer sequence
<400> 58
   agaacatcag cgcttggtca 20
<210> 59
   <211> 20
   <212> DNA
   <213> Unknown
<220>
   <223> Spacer sequence
<400> 59
   ataacggctt gacccagtca 20
<210> 60
   <211> 20
   <212> DNA
   <213> Unknown
<220>
   <223> Spacer sequence
<400> 60
   ggcaaccaga atatcagtgg 20
<210> 61
   <211> 20
   <212> DNA
   <213> Unknown
<220>
   <223> Spacer sequence
<400> 61
   ggatgccggt gacgaacagc 20
<210> 62
   <211> 20
   <212> DNA
   <213> Unknown
<220>
   <223> Spacer sequence
<400> 62
   gctacagtac agcgataacg 20
<210> 63
   <211> 20
   <212> DNA
   <213> Unknown
<220>
   <223> Spacer sequence
<400> 63
   gacgttgcgt cagcttacgc 20
<210> 64
   <211> 20
   <212> DNA
   <213> Unknown
<220>
   <223> Spacer sequence
<400> 64
   aactacttac tctagcttcc 20
<210> 65
   <211> 20
   <212> DNA
   <213> Unknown
<220>
   <223> Spacer sequence
<400> 65
   ttgttgctga aggagttggg 20
<210> 66
   <211> 20
   <212> DNA
   <213> Unknown
<220>
   <223> Spacer sequence
<400> 66
   agcgaaaaac accttgccga 20
<210> 67
   <211> 20
   <212> DNA
   <213> Unknown
<220>
   <223> Spacer sequence
<400> 67
   acctttaaag tgctgctgtg 20
<210> 68
   <211> 20
   <212> DNA
   <213> Unknown
<220>
   <223> Spacer sequence
<400> 68
   tgggttgttg gagagaaaac 20
<210> 69
   <211> 20
   <212> DNA
   <213> Unknown
<220>
   <223> Spacer sequence
<400> 69
   aaacacagcg gcacttctcg 20
<210> 70
   <211> 20
   <212> DNA
   <213> Unknown
<220>
   <223> Spacer sequence
<400> 70
   aaaattgaag ttttttatcc 20
<210> 71
   <211> 20
   <212> DNA
   <213> Unknown
<220>
   <223> Spacer sequence
<400> 71
   ggtttgatcg tcagggatgg 20
<210> 72
   <211> 20
   <212> DNA
   <213> Unknown
<220>
   <223> Spacer sequence
<400> 72
   gtggattaac gttccgaaag 20
<210> 73
   <211> 20
   <212> DNA
   <213> Unknown
<220>
   <223> Spacer sequence
<400> 73
   cagcgacagc aaagtggcat 20
<210> 74
   <211> 20
   <212> DNA
   <213> Unknown
<220>
   <223> Spacer sequence
<400> 74
   cttgccacct acagtgcggg 20
<210> 75
   <211> 20
   <212> DNA
   <213> Unknown
<220>
   <223> Spacer sequence
<400> 75
   cccccaaagg aatggagatc 20
<210> 76
   <211> 20
   <212> DNA
   <213> Unknown
<220>
   <223> Spacer sequence
<400> 76
   caccaagtct ttaagtggga 20
<210> 77
   <211> 20
   <212> DNA
   <213> Unknown
<220>
   <223> Spacer sequence
<400> 77
   ataacggctt gacccagtca 20
<210> 78
   <211> 20
   <212> DNA
   <213> Unknown
<220>
   <223> Spacer sequence
<400> 78
   ctgggaaacg gaactgaatg 20
<210> 79
   <211> 20
   <212> DNA
   <213> Unknown
<220>
   <223> Spacer sequence
<400> 79
   acgttaaaca ccgccattcc 20
<210> 80
   <211> 20
   <212> DNA
   <213> Unknown
<220>
   <223> Spacer sequence
<400> 80
   aactacttac tctagcttcc 20
<210> 81
   <211> 20
   <212> DNA
   <213> Unknown
<220>
   <223> Spacer sequence
<400> 81
   ttgttgctga aggagttggg 20
<210> 82
   <211> 20
   <212> DNA
   <213> Unknown
<220>
   <223> Spacer sequence
<400> 82
   aaacacagcg gcacttctcg 20
<210> 83
   <211> 34
   <212> DNA
   <213> Unknown
<220>
   <223> Spacer sequence
<400> 83
   ggctagttaa aaataaaatt gaagtttttt atcc 34
<210> 84
   <211> 20
   <212> DNA
   <213> Unknown
<220>
   <223> Spacer sequence
<400> 84
   ggtttgatcg tcagggatgg 20
<210> 85
   <211> 20
   <212> DNA
   <213> Unknown
<220>
   <223> Spacer sequence
<400> 85
   ataacggctt gacccagtca 20
<210> 86
   <211> 195
   <212> DNA
   <213> Artificial
<220>
   <223> Primer sequence
<400> 86
   ccaaaactga gacctgctgc ggacgtccaa aggtctcgtt ttagagctat gctgttttga 60
   atggtcccaa aacttgccga tgataacttg agaaagaggg ttaataccag cagtcggata 120
   ccttcctatt ctttctgtta aagcgttttc atgttataat aggcaaattt tagatgaaga 180
   ttatttctta ataac 195
<210> 87
   <211> 81
   <212> DNA
   <213> Artificial
<220>
   <223> Primer sequence
<400> 87
   tctaaaacga agagcgctgc gtccgagacg ctcttcagtt ttgggaccat tcaaaacagc 60
   atagctctaa aacctcgtag a 81
<210> 88
   <211> 54
   <212> DNA
   <213> Artificial
<220>
   <223> Primer sequence
<400> 88
   gggctggcaa gccacgtttg gtgggtctcg aaacggtttg atcgtcaggg atgg 54
<210> 89
   <211> 90
   <212> DNA
   <213> Artificial
<220>
   <223> Primer sequence
<400> 89
   ggataaaaaa cttcaatttt atttttaact agccgttttg ggaccattca aaacagcata 60
   gctctaaaac ccatccctga cgatcaaacc 90
<210> 90
   <211> 90
   <212> DNA
   <213> Artificial
<220>
   <223> Primer sequence
<400> 90
   ggctagttaa aaataaaatt gaagtttttt atccgtttta gagctatgct gttttgaatg 60
   gtcccaaaac aaacacagcg gcacttctcg 90
<210> 91
   <211> 76
   <212> DNA
   <213> Artificial
<220>
   <223> Primer sequence
<400> 91
   cccaactcct tcagcaacaa gttttgggac cattcaaaac agcatagctc taaaaccgag 60
   aagtgccgct gtgttt 76
<210> 92
   <211> 76
   <212> DNA
   <213> Artificial
<220>
   <223> Primer sequence
<400> 92
   ttgttgctga aggagttggg gttttagagc tatgctgttt tgaatggtcc caaaacgtcc 60
   atcccactta aagact 76
<210> 93
   <211> 76
   <212> DNA
   <213> Artificial
<220>
   <223> Primer sequence
<400> 93
   cattcagttc cgtttcccag gttttgggac cattcaaaac agcatagctc taaaacagtc 60
   tttaagtggg atggac 76
<210> 94
   <211> 76
   <212> DNA
   <213> Artificial
<220>
   <223> Primer sequence
<400> 94
   ctgggaaacg gaactgaatg gttttagagc tatgctgttt tgaatggtcc caaaacaact 60
   acttactcta gcttcc 76
<210> 95
   <211> 111
   <212> DNA
   <213> Artificial
<220>
   <223> Primer sequence
<400> 95
   ccgggagctg catgtgtcag aggggtctcc aaaacggaat ggcggtgttt aacgtgtttt 60
   gggaccattc aaaacagcat agctctaaaa cggaagctag agtaagtagt t 111
<210> 96
   <211> 111
   <212> DNA
   <213> Artificial
<220>
   <223> Primer sequence
<400> 96
   ccgggagctg catgtgtcag aggggtctcc aaaaccccaa ctccttcagc aacaagtttt 60
   gggaccattc aaaacagcat agctctaaaa ccgagaagtg ccgctgtgtt t 111
<210> 97
   <211> 54
   <212> DNA
   <213> Artificial
<220>
   <223> Primer sequence
<400> 97
   gggctggcaa gccacgtttg gtggctcttc aaacgtccat cccacttaaa gact 54
<210> 98
   <211> 111
   <212> DNA
   <213> Artificial
<220>
   <223> Primer sequence
<400> 98
   ccgggagctg catgtgtcag agggctcttc aaaacggaat ggcggtgttt aacgtgtttt 60
   gggaccattc aaaacagcat agctctaaaa cggaagctag agtaagtagt t 111
<210> 99
   <211> 1368
   <212> PRT
   <213> Streptococcus pyogenes M1 GAS
<400> 99
<210> 100
   <211> 20
   <212> DNA
   <213> Unknown
<220>
   <223> Anti-protospacer sequence
<400> 100
   tagataacta cgatacggga 20
<210> 101
   <211> 20
   <212> DNA
   <213> Unknown
<220>
   <223> Anti-protospacer sequence
<400> 101
   gatcgttgtc agaagtaagt 20
<210> 102
   <211> 20
   <212> DNA
   <213> Unknown
<220>
   <223> Anti-protospacer sequence
<400> 102
   actttaaaag tgctcatcat 20
<210> 103
   <211> 20
   <212> DNA
   <213> Unknown
<220>
   <223> Anti-protospacer sequence
<400> 103
   tttactttca ccagcgtttc 20
<210> 104
   <211> 20
   <212> DNA
   <213> Unknown
<220>
   <223> Anti-protospacer sequence
<400> 104
   attaatagac tggatggagg 20
<210> 105
   <211> 20
   <212> DNA
   <213> Unknown
<220>
   <223> Anti-protospacer sequence
<400> 105
   acaattaata gactggatgg 20
<210> 106
   <211> 20
   <212> DNA
   <213> Unknown
<220>
   <223> Anti-protospacer sequence
<400> 106
   gcaacaatta atagactgga 20
<210> 107
   <211> 20
   <212> DNA
   <213> Unknown
<220>
   <223> Anti-protospacer sequence
<400> 107
   cccggcaaca attaatagac 20
<210> 108
   <211> 20
   <212> DNA
   <213> Unknown
<220>
   <223> Anti-protospacer sequence
<400> 108
   aactacttac tctagcttcc 20
<210> 109
   <211> 20
   <212> DNA
   <213> Unknown
<220>
   <223> Anti-protospacer sequence
<400> 109
   acgttgcgca aactattaac 20
<210> 110
   <211> 20
   <212> DNA
   <213> Unknown
<220>
   <223> Anti-protospacer sequence
<400> 110
   tgtaactcgc cttgatcgtt 20
<210> 111
   <211> 20
   <212> DNA
   <213> Unknown
<220>
   <223> Anti-protospacer sequence
<400> 111
   atgtaactcg ccttgatcgt 20
<210> 112
   <211> 20
   <212> DNA
   <213> Unknown
<220>
   <223> Anti-protospacer sequence
<400> 112
   aacttacttc tgacaacgat 20
<210> 113
   <211> 20
   <212> DNA
   <213> Unknown
<220>
   <223> Anti-protospacer sequence
<400> 113
   agtcacagaa aagcatctta 20
<210> 114
   <211> 20
   <212> DNA
   <213> Unknown
<220>
   <223> Anti-protospacer sequence
<400> 114
   acttttaaag ttctgctatg 20
<210> 115
   <211> 25
   <212> DNA
   <213> Unknown
<220>
   <223> Adaptor sequence
<400> 115
   aaactagata actacgatac gggag 25
<210> 116
   <211> 25
   <212> DNA
   <213> Unknown
<220>
   <223> Adaptor sequence
<400> 116
   aaaactcccg tatcgtagtt atcta 25
<210> 117
   <211> 25
   <212> DNA
   <213> Unknown
<220>
   <223> Adaptor sequence
<400> 117
   aaacacttta aaagtgctca tcatg 25
<210> 118
   <211> 25
   <212> DNA
   <213> Unknown
<220>
   <223> Adaptor sequence
<400> 118
   aaaacatgat gagcactttt aaagt 25
<210> 119
   <211> 25
   <212> DNA
   <213> Unknown
<220>
   <223> Adaptor sequence
<400> 119
   aaacacgttg cgcaaactat taacg 25
<210> 120
   <211> 25
   <212> DNA
   <213> Unknown
<220>
   <223> Adaptor sequence
<400> 120
   aaaacgttaa tagtttgcgc aacgt 25
<210> 121
   <211> 25
   <212> DNA
   <213> Unknown
<220>
   <223> Adaptor sequence
<400> 121
   aaacactttt aaagttctgc tatgg 25
<210> 122
   <211> 25
   <212> DNA
   <213> Unknown
<220>
   <223> Adaptor sequence
<400> 122
   aaaaccatag cagaacttta aaagt 25
<210> 123
   <211> 81
   <212> DNA
   <213> Unknown
<220>
   <223> Adaptor sequence for dual targets
<400> 123
   aaacacttta aaagtgctca tcatgtttta gagctatgct gttttgaatg gtcccaaaac 60
   acttttaaag ttctgctatg g 81
<210> 124
   <211> 81
   <212> DNA
   <213> Unknown
<220>
   <223> Adaptor sequence for dual targets
<400> 124
   aaaaccatag cagaacttta aaagtgtttt gggaccattc aaaacagcat agctctaaaa 60
   catgatgagc acttttaaag t 81
<210> 125
   <211> 9326
   <212> DNA
   <213> Artificial
<220>
   <223> Plasmid sequence
<400> 125
   gaattccgga tgagcattca tcaggcgggc aagaatgtga ataaaggccg gataaaactt      60
   gtgcttattt ttctttacgg tctttaaaaa ggccgtaata tccagctgaa cggtctggtt     120
   ataggtacat tgagcaactg actgaaatgc ctcaaaatgt tctttacgat gccattggga     180
   tatatcaacg gtggtatatc cagtgatttt tttctccatt ttagcttcct tagctcctga     240
   aaatctcgat aactcaaaaa atacgcccgg tagtgatctt atttcattat ggtgaaagtt     300
   ggaacctctt acgtgccgat caacgtctca ttttcgccaa aagttggccc agggcttccc     360
   ggtatcaaca gggacaccag gatttattta ttctgcgaag tgatcttccg tcacaggtat     420
   ttattcggcg caaagtgcgt cgggtgatgc tgccaactta ctgatttagt gtatgatggt 480
   gtttttgagg tgctccagtg gcttctgttt ctatcagctg tccctcctgt tcagctactg 540
   acggggtggt gcgtaacggc aaaagcaccg ccggacatca gcgctagcgg agtgtatact 600
   ggcttactat gttggcactg atgagggtgt cagtgaagtg cttcatgtgg caggagaaaa 660
   aaggctgcac cggtgcgtca gcagaatatg tgatacagga tatattccgc ttcctcgctc 720
   actgactcgc tacgctcggt cgttcgactg cggcgagcgg aaatggctta cgaacggggc 780
   ggagatttcc tggaagatgc caggaagata cttaacaggg aagtgagagg gccgcggcaa 840
   agccgttttt ccataggctc cgcccccctg acaagcatca cgaaatctga cgctcaaatc 900
   agtggtggcg aaacccgaca ggactataaa gataccaggc gtttccccct ggcggctccc 960
   tcgtgcgctc tcctgttcct gcctttcggt ttaccggtgt cattccgctg ttatggccgc 1020
   gtttgtctca ttccacgcct gacactcagt tccgggtagg cagttcgctc caagctggac 1080
   tgtatgcacg aaccccccgt tcagtccgac cgctgcgcct tatccggtaa ctatcgtctt 1140
   gagtccaacc cggaaagaca tgcaaaagca ccactggcag cagccactgg taattgattt 1200
   agaggagtta gtcttgaagt catgcgccgg ttaaggctaa actgaaagga caagttttgg 1260
   tgactgcgct cctccaagcc agttacctcg gttcaaagag ttggtagctc agagaacctt 1320
   cgaaaaaccg ccctgcaagg cggttttttc gttttcagag caagagatta cgcgcagacc 1380
   aaaacgatct caagaagatc atcttattaa tcagataaaa tatttctaga tttcagtgca 1440
   atttatctct tcaaatgtag cacctgaagt cagccccata cgatataagt tgtaattctc 1500
   atgtttgaca gcttatcatc gataagcttt aatgcggtag tttatcacag ttaaattgct 1560
   aacgcagtca ggcaccgtgt atgaaatcta acaatgcgct catcgtcatc ctcggcaccg 1620
   tcaccctgga tgctgtaggc ataggcttgg ttatgccggt actgccgggc ctcttgcggg 1680
   attacgaaat catcctgtgg agcttagtag gtttagcaag atggcagcgc ctaaatgtag 1740
   aatgataaaa ggattaagag attaatttcc ctaaaaatga taaaacaagc gttttgaaag 1800
   cgcttgtttt tttggtttgc agtcagagta gaatagaagt atcaaaaaaa gcaccgactc 1860
   ggtgccactt tttcaagttg ataacggact agccttattt taacttgcta tgctgttttg 1920
   aatggttcca acaagattat tttataactt ttataacaaa taatcaagga gaaattcaaa 1980
   gaaatttatc agccataaaa caatacttaa tactatagaa tgataacaaa ataaactact 2040
   ttttaaaaga attttgtgtt ataatctatt tattattaag tattgggtaa tattttttga 2100
   agagatattt tgaaaaagaa aaattaaagc atattaaact aatttcggag gtcattaaaa 2160
   ctattattga aatcatcaaa ctcattatgg atttaattta aactttttat tttaggaggc 2220
   aaaaatggat aagaaatact caataggctt agatatcggc acaaatagcg tcggatgggc 2280
   ggtgatcact gatgaatata aggttccgtc taaaaagttc aaggttctgg gaaatacaga 2340
   ccgccacagt atcaaaaaaa atcttatagg ggctctttta tttgacagtg gagagacagc 2400
   ggaagcgact cgtctcaaac ggacagctcg tagaaggtat acacgtcgga agaatcgtat 2460
   ttgttatcta caggagattt tttcaaatga gatggcgaaa gtagatgata gtttctttca 2520
   tcgacttgaa gagtcttttt tggtggaaga agacaagaag catgaacgtc atcctatttt 2580
   tggaaatata gtagatgaag ttgcttatca tgagaaatat ccaactatct atcatctgcg 2640
   aaaaaaattg gtagattcta ctgataaagc ggatttgcgc ttaatctatt tggccttagc 2700
   gcatatgatt aagtttcgtg gtcatttttt gattgaggga gatttaaatc ctgataatag 2760
   tgatgtggac aaactattta tccagttggt acaaacctac aatcaattat ttgaagaaaa 2820
   ccctattaac gcaagtggag tagatgctaa agcgattctt tctgcacgat tgagtaaatc 2880
   aagacgatta gaaaatctca ttgctcagct ccccggtgag aagaaaaatg gcttatttgg 2940
   gaatctcatt gctttgtcat tgggtttgac ccctaatttt aaatcaaatt ttgatttggc 3000
   agaagatgct aaattacagc tttcaaaaga tacttacgat gatgatttag ataatttatt 3060
   ggcgcaaatt ggagatcaat atgctgattt gtttttggca gctaagaatt tatcagatgc 3120
   tattttactt tcagatatcc taagagtaaa tactgaaata actaaggctc ccctatcagc 3180
   ttcaatgatt aaacgctacg atgaacatca tcaagacttg actcttttaa aagctttagt 3240
   tcgacaacaa cttccagaaa agtataaaga aatctttttt gatcaatcaa aaaacggata 3300
   tgcaggttat attgatgggg gagctagcca agaagaattt tataaattta tcaaaccaat 3360
   tttagaaaaa atggatggta ctgaggaatt attggtgaaa ctaaatcgtg aagatttgct 3420
   gcgcaagcaa cggacctttg acaacggctc tattccccat caaattcact tgggtgagct 3480
   gcatgctatt ttgagaagac aagaagactt ttatccattt ttaaaagaca atcgtgagaa 3540
   gattgaaaaa atcttgactt ttcgaattcc ttattatgtt ggtccattgg cgcgtggcaa 3600
   tagtcgtttt gcatggatga ctcggaagtc tgaagaaaca attaccccat ggaattttga 3660
   agaagttgtc gataaaggtg cttcagctca atcatttatt gaacgcatga caaactttga 3720
   taaaaatctt ccaaatgaaa aagtactacc aaaacatagt ttgctttatg agtattttac 3780
   ggtttataac gaattgacaa aggtcaaata tgttactgaa ggaatgcgaa aaccagcatt 3840
   tctttcaggt gaacagaaga aagccattgt tgatttactc ttcaaaacaa atcgaaaagt 3900
   aaccgttaag caattaaaag aagattattt caaaaaaata gaatgttttg atagtgttga 3960
   aatttcagga gttgaagata gatttaatgc ttcattaggt acctaccatg atttgctaaa 4020
   aattattaaa gataaagatt ttttggataa tgaagaaaat gaagatatct tagaggatat 4080
   tgttttaaca ttgaccttat ttgaagatag ggagatgatt gaggaaagac ttaaaacata 4140
   tgctcacctc tttgatgata aggtgatgaa acagcttaaa cgtcgccgtt atactggttg 4200
   gggacgtttg tctcgaaaat tgattaatgg tattagggat aagcaatctg gcaaaacaat 4260
   attagatttt ttgaaatcag atggttttgc caatcgcaat tttatgcagc tgatccatga 4320
   tgatagtttg acatttaaag aagacattca aaaagcacaa gtgtctggac aaggcgatag 4380
   tttacatgaa catattgcaa atttagctgg tagccctgct attaaaaaag gtattttaca 4440
   gactgtaaaa gttgttgatg aattggtcaa agtaatgggg cggcataagc cagaaaatat 4500
   cgttattgaa atggcacgtg aaaatcagac aactcaaaag ggccagaaaa attcgcgaga 4560
   gcgtatgaaa cgaatcgaag aaggtatcaa agaattagga agtcagattc ttaaagagca 4620
   tcctgttgaa aatactcaat tgcaaaatga aaagctctat ctctattatc tccaaaatgg 4680
   aagagacatg tatgtggacc aagaattaga tattaatcgt ttaagtgatt atgatgtcga 4740
   tcacattgtt ccacaaagtt tccttaaaga cgattcaata gacaataagg tcttaacgcg 4800
   ttctgataaa aatcgtggta aatcggataa cgttccaagt gaagaagtag tcaaaaagat 4860
   gaaaaactat tggagacaac ttctaaacgc caagttaatc actcaacgta agtttgataa 4920
   tttaacgaaa gctgaacgtg gaggtttgag tgaacttgat aaagctggtt ttatcaaacg 4980
   ccaattggtt gaaactcgcc aaatcactaa gcatgtggca caaattttgg atagtcgcat 5040
   gaatactaaa tacgatgaaa atgataaact tattcgagag gttaaagtga ttaccttaaa 5100
   atctaaatta gtttctgact tccgaaaaga tttccaattc tataaagtac gtgagattaa 5160
   caattaccat catgcccatg atgcgtatct aaatgccgtc gttggaactg ctttgattaa 5220
   gaaatatcca aaacttgaat cggagtttgt ctatggtgat tataaagttt atgatgttcg 5280
   taaaatgatt gctaagtctg agcaagaaat aggcaaagca accgcaaaat atttctttta 5340
   ctctaatatc atgaacttct tcaaaacaga aattacactt gcaaatggag agattcgcaa 5400
   acgccctcta atcgaaacta atggggaaac tggagaaatt gtctgggata aagggcgaga 5460
   ttttgccaca gtgcgcaaag tattgtccat gccccaagtc aatattgtca agaaaacaga 5520
   agtacagaca ggcggattct ccaaggagtc aattttacca aaaagaaatt cggacaagct 5580
   tattgctcgt aaaaaagact gggatccaaa aaaatatggt ggttttgata gtccaacggt 5640
   agcttattca gtcctagtgg ttgctaaggt ggaaaaaggg aaatcgaaga agttaaaatc 5700
   cgttaaagag ttactaggga tcacaattat ggaaagaagt tcctttgaaa aaaatccgat 5760
   tgacttttta gaagctaaag gatataagga agttaaaaaa gacttaatca ttaaactacc 5820
   taaatatagt ctttttgagt tagaaaacgg tcgtaaacgg atgctggcta gtgccggaga 5880
   attacaaaaa ggaaatgagc tggctctgcc aagcaaatat gtgaattttt tatatttagc 5940
   tagtcattat gaaaagttga agggtagtcc agaagataac gaacaaaaac aattgtttgt 6000
   ggagcagcat aagcattatt tagatgagat tattgagcaa atcagtgaat tttctaagcg 6060
   tgttatttta gcagatgcca atttagataa agttcttagt gcatataaca aacatagaga 6120
   caaaccaata cgtgaacaag cagaaaatat tattcattta tttacgttga cgaatcttgg 6180
   agctcccgct gcttttaaat attttgatac aacaattgat cgtaaacgat atacgtctac 6240
   aaaagaagtt ttagatgcca ctcttatcca tcaatccatc actggtcttt atgaaacacg 6300
   cattgatttg agtcagctag gaggtgactg aagtatattt tagatgaaga ttatttctta 6360
   ataactaaaa atatggtata atactcttaa taaatgcagt aatacagggg cttttcaaga 6420
   ctgaagtcta gctgagacaa atagtgcgat tacgaaattt tttagacaaa aatagtctac 6480
   gaggttttag agctatgctg ttttgaatgg tcccaaaact gagaccagtc tcggaagctc 6540
   aaaggtctcg ttttagagct atgctgtttt gaatggtccc aaaacttcag cacactgaga 6600
   cttgttgagt tccatgtttt agagctatgc tgttttgaat ggactccatt caacattgcc 6660
   gatgataact tgagaaagag ggttaatacc agcagtcgga taccttccta ttctttctgt 6720
   taaagcgttt tcatgttata ataggcaaaa gaagagtagt gtgatcgtcc attccgacag 6780
   catcgccagt cactatggcg tgctgctagc gctatatgcg ttgatgcaat ttctatgcgc 6840
   acccgttctc ggagcactgt ccgaccgctt tggccgccgc ccagtcctgc tcgcttcgct 6900
   acttggagcc actatcgact acgcgatcat ggcgaccaca cccgtcctgt ggatcctcta 6960
   cgccggacgc atcgtggccg gcatcaccgg cgccacaggt gcggttgctg gcgcctatat 7020
   cgccgacatc accgatgggg aagatcgggc tcgccacttc gggctcatga gcgcttgttt 7080
   cggcgtgggt atggtggcag gccccgtggc cgggggactg ttgggcgcca tctccttgca 7140
   tgcaccattc cttgcggcgg cggtgctcaa cggcctcaac ctactactgg gctgcttcct 7200
   aatgcaggag tcgcataagg gagagcgtcg accgatgccc ttgagagcct tcaacccagt 7260
   cagctccttc cggtgggcgc ggggcatgac tatcgtcgcc gcacttatga ctgtcttctt 7320
   tatcatgcaa ctcgtaggac aggtgccggc agcgctctgg gtcattttcg gcgaggaccg 7380
   ctttcgctgg agcgcgacga tgatcggcct gtcgcttgcg gtattcggaa tcttgcacgc 7440
   cctcgctcaa gccttcgtca ctggtcccgc caccaaacgt ttcggcgaga agcaggccat 7500
   tatcgccggc atggcggccg acgcgctggg ctacgtcttg ctggcgttcg cgacgcgagg 7560
   ctggatggcc ttccccatta tgattcttct cgcttccggc ggcatcggga tgcccgcgtt 7620
   gcaggccatg ctgtccaggc aggtagatga cgaccatcag ggacagcttc aaggatcgct 7680
   cgcggctctt accagcctaa cttcgatcat tggaccgctg atcgtcacgg cgatttatgc 7740
   cgcctcggcg agcacatgga acgggttggc atggattgta ggcgccgccc tataccttgt 7800
   ctgcctcccc gcgttgcgtc gcggtgcatg gagccgggcc acctcgacct gaatggaagc 7860
   cggcggcacc tcgctaacgg attcaccact ccaagaattg gagccaatca attcttgcgg 7920
   agaactgtga atgcgcaaac caacccttgg cagaacatat ccatcgcgtc cgccatctcc 7980
   agcagccgca cgcggcgcat ctcgggcagc gttgggtcct ggccacgggt gcgcatgatc 8040
   gtgctcctgt cgttgaggac ccggctaggc tggcggggtt gccttactgg ttagcagaat 8100
   gaatcaccga tacgcgagcg aacgtgaagc gactgctgct gcaaaacgtc tgcgacctga 8160
   gcaacaacat gaatggtctt cggtttccgt gtttcgtaaa gtctggaaac gcggaagtcc 8220
   cctacgtgct gctgaagttg cccgcaacag agagtggaac caaccggtga taccacgata 8280
   ctatgactga gagtcaacgc catgagcggc ctcatttctt attctgagtt acaacagtcc 8340
   gcaccgctgt ccggtagctc cttccggtgg gcgcggggca tgactatcgt cgccgcactt 8400
   atgactgtct tctttatcat gcaactcgta ggacaggtgc cggcagcgcc caacagtccc 8460
   ccggccacgg ggcctgccac catacccacg ccgaaacaag cgccctgcac cattatgttc 8520
   cggatctgca tcgcaggatg ctgctggcta ccctgtggaa cacctacatc tgtattaacg 8580
   aagcgctaac cgtttttatc aggctctggg aggcagaata aatgatcata tcgtcaatta 8640
   ttacctccac ggggagagcc tgagcaaact ggcctcaggc atttgagaag cacacggtca 8700
   cactgcttcc ggtagtcaat aaaccggtaa accagcaata gacataagcg gctatttaac 8760
   gaccctgccc tgaaccgacg accgggtcga atttgctttc gaatttctgc cattcatccg 8820
   cttattatca cttattcagg cgtagcacca ggcgtttaag ggcaccaata actgccttaa 8880
   aaaaattacg ccccgccctg ccactcatcg cagtactgtt gtaattcatt aagcattctg 8940
   ccgacatgga agccatcaca gacggcatga tgaacctgaa tcgccagcgg catcagcacc 9000
   ttgtcgcctt gcgtataata tttgcccatg gtgaaaacgg gggcgaagaa gttgtccata 9060
   ttggccacgt ttaaatcaaa actggtgaaa ctcacccagg gattggctga gacgaaaaac 9120
   atattctcaa taaacccttt agggaaatag gccaggtttt caccgtaaca cgccacatct 9180
   tgcgaatata tgtgtagaaa ctgccggaaa tcgtcgtggt attcactcca gagcgatgaa 9240
   aacgtttcag tttgctcatg gaaaacggtg taacaagggt gaacactatc ccatatcacc 9300
   agctcaccgt ctttcattgc catacg 9326
<210> 126
   <211> 4107
   <212> DNA
   <213> Artificial
<220>
   <223> Cas9 coding sequence
<400> 126
   atggataaga aatactcaat aggcttagat atcggcacaa atagcgtcgg atgggcggtg        60
   atcactgatg aatataaggt tccgtctaaa aagttcaagg ttctgggaaa tacagaccgc       120
   cacagtatca aaaaaaatct tataggggct cttttatttg acagtggaga gacagcggaa       180
   gcgactcgtc tcaaacggac agctcgtaga aggtatacac gtcggaagaa tcgtatttgt       240
   tatctacagg agattttttc aaatgagatg gcgaaagtag atgatagttt ctttcatcga 300
   cttgaagagt cttttttggt ggaagaagac aagaagcatg aacgtcatcc tatttttgga 360
   aatatagtag atgaagttgc ttatcatgag aaatatccaa ctatctatca tctgcgaaaa 420
   aaattggtag attctactga taaagcggat ttgcgcttaa tctatttggc cttagcgcat 480
   atgattaagt ttcgtggtca ttttttgatt gagggagatt taaatcctga taatagtgat 540
   gtggacaaac tatttatcca gttggtacaa acctacaatc aattatttga agaaaaccct 600
   attaacgcaa gtggagtaga tgctaaagcg attctttctg cacgattgag taaatcaaga 660
   cgattagaaa atctcattgc tcagctcccc ggtgagaaga aaaatggctt atttgggaat 720
   ctcattgctt tgtcattggg tttgacccct aattttaaat caaattttga tttggcagaa 780
   gatgctaaat tacagctttc aaaagatact tacgatgatg atttagataa tttattggcg 840
   caaattggag atcaatatgc tgatttgttt ttggcagcta agaatttatc agatgctatt 900
   ttactttcag atatcctaag agtaaatact gaaataacta aggctcccct atcagcttca 960
   atgattaaac gctacgatga acatcatcaa gacttgactc ttttaaaagc tttagttcga 1020
   caacaacttc cagaaaagta taaagaaatc ttttttgatc aatcaaaaaa cggatatgca 1080
   ggttatattg atgggggagc tagccaagaa gaattttata aatttatcaa accaatttta 1140
   gaaaaaatgg atggtactga ggaattattg gtgaaactaa atcgtgaaga tttgctgcgc 1200
   aagcaacgga cctttgacaa cggctctatt ccccatcaaa ttcacttggg tgagctgcat 1260
   gctattttga gaagacaaga agacttttat ccatttttaa aagacaatcg tgagaagatt 1320
   gaaaaaatct tgacttttcg aattccttat tatgttggtc cattggcgcg tggcaatagt 1380
   cgttttgcat ggatgactcg gaagtctgaa gaaacaatta ccccatggaa ttttgaagaa 1440
   gttgtcgata aaggtgcttc agctcaatca tttattgaac gcatgacaaa ctttgataaa 1500
   aatcttccaa atgaaaaagt actaccaaaa catagtttgc tttatgagta ttttacggtt 1560
   tataacgaat tgacaaaggt caaatatgtt actgaaggaa tgcgaaaacc agcatttctt 1620
   tcaggtgaac agaagaaagc cattgttgat ttactcttca aaacaaatcg aaaagtaacc 1680
   gttaagcaat taaaagaaga ttatttcaaa aaaatagaat gttttgatag tgttgaaatt 1740
   tcaggagttg aagatagatt taatgcttca ttaggtacct accatgattt gctaaaaatt 1800
   attaaagata aagatttttt ggataatgaa gaaaatgaag atatcttaga ggatattgtt 1860
   ttaacattga ccttatttga agatagggag atgattgagg aaagacttaa aacatatgct 1920
   cacctctttg atgataaggt gatgaaacag cttaaacgtc gccgttatac tggttgggga 1980
   cgtttgtctc gaaaattgat taatggtatt agggataagc aatctggcaa aacaatatta 2040
   gattttttga aatcagatgg ttttgccaat cgcaatttta tgcagctgat ccatgatgat 2100
   agtttgacat ttaaagaaga cattcaaaaa gcacaagtgt ctggacaagg cgatagttta 2160
   catgaacata ttgcaaattt agctggtagc cctgctatta aaaaaggtat tttacagact 2220
   gtaaaagttg ttgatgaatt ggtcaaagta atggggcggc ataagccaga aaatatcgtt 2280
   attgaaatgg cacgtgaaaa tcagacaact caaaagggcc agaaaaattc gcgagagcgt 2340
   atgaaacgaa tcgaagaagg tatcaaagaa ttaggaagtc agattcttaa agagcatcct 2400
   gttgaaaata ctcaattgca aaatgaaaag ctctatctct attatctcca aaatggaaga 2460
   gacatgtatg tggaccaaga attagatatt aatcgtttaa gtgattatga tgtcgatcac 2520
   attgttccac aaagtttcct taaagacgat tcaatagaca ataaggtctt aacgcgttct 2580
   gataaaaatc gtggtaaatc ggataacgtt ccaagtgaag aagtagtcaa aaagatgaaa 2640
   aactattgga gacaacttct aaacgccaag ttaatcactc aacgtaagtt tgataattta 2700
   acgaaagctg aacgtggagg tttgagtgaa cttgataaag ctggttttat caaacgccaa 2760
   ttggttgaaa ctcgccaaat cactaagcat gtggcacaaa ttttggatag tcgcatgaat 2820
   actaaatacg atgaaaatga taaacttatt cgagaggtta aagtgattac cttaaaatct 2880
   aaattagttt ctgacttccg aaaagatttc caattctata aagtacgtga gattaacaat 2940
   taccatcatg cccatgatgc gtatctaaat gccgtcgttg gaactgcttt gattaagaaa 3000
   tatccaaaac ttgaatcgga gtttgtctat ggtgattata aagtttatga tgttcgtaaa 3060
   atgattgcta agtctgagca agaaataggc aaagcaaccg caaaatattt cttttactct 3120
   aatatcatga acttcttcaa aacagaaatt acacttgcaa atggagagat tcgcaaacgc 3180
   cctctaatcg aaactaatgg ggaaactgga gaaattgtct gggataaagg gcgagatttt 3240
   gccacagtgc gcaaagtatt gtccatgccc caagtcaata ttgtcaagaa aacagaagta 3300
   cagacaggcg gattctccaa ggagtcaatt ttaccaaaaa gaaattcgga caagcttatt 3360
   gctcgtaaaa aagactggga tccaaaaaaa tatggtggtt ttgatagtcc aacggtagct 3420
   tattcagtcc tagtggttgc taaggtggaa aaagggaaat cgaagaagtt aaaatccgtt 3480
   aaagagttac tagggatcac aattatggaa agaagttcct ttgaaaaaaa tccgattgac 3540
   tttttagaag ctaaaggata taaggaagtt aaaaaagact taatcattaa actacctaaa 3600
   tatagtcttt ttgagttaga aaacggtcgt aaacggatgc tggctagtgc cggagaatta 3660
   caaaaaggaa atgagctggc tctgccaagc aaatatgtga attttttata tttagctagt 3720
   cattatgaaa agttgaaggg tagtccagaa gataacgaac aaaaacaatt gtttgtggag 3780
   cagcataagc attatttaga tgagattatt gagcaaatca gtgaattttc taagcgtgtt 3840
   attttagcag atgccaattt agataaagtt cttagtgcat ataacaaaca tagagacaaa 3900
   ccaatacgtg aacaagcaga aaatattatt catttattta cgttgacgaa tcttggagct 3960
   cccgctgctt ttaaatattt tgatacaaca attgatcgta aacgatatac gtctacaaaa 4020
   gaagttttag atgccactct tatccatcaa tccatcactg gtctttatga aacacgcatt 4080
   gatttgagtc agctaggagg tgactga 4107
<210> 127
   <211> 69
   <212> DNA
   <213> Artificial
<220>
   <223> -10 promoter in pCas9
<400> 127
   agtatatttt agatgaagat tatttcttaa taactaaaaa tatggtataa tactcttaat 60
   aaatgcagt 69
<210> 128
   <211> 80
   <212> DNA
   <213> Artificial
<220>
   <223> Leader sequence in pCas9
<400> 128
   aatacagggg cttttcaaga ctgaagtcta gctgagacaa atagtgcgat tacgaaattt 60
   tttagacaaa aatagtctac 80
<210> 129
   <211> 194
   <212> DNA
   <213> Artificial
<220>
   <223> Spacer sequence
<400> 129
   aaaactgaga ccgggcagtg agcgcaacgc aattaacatt aggcacccca ggcttgacaa 60
   ttaatcatcg gctcgtataa tgtgtggaat tgtgagcgga taacaatttc acacggaggt 120
   cacatatgag ataataataa ctagctgaat tccccagccc gcctaatgag cgggcttttt 180
   tttggtctcg tttt 194
<210> 130
   <211> 86
   <212> RNA
   <213> Artificial
<220>
   <223> tracrRNA
<400> 130
   ggaaccauuc aaaacagcau agcaaguuaa aauaaggcua guccguuauc aacuugaaaa 60
   aguggcaccg agucggugcu uuuuuu 86
<210> 131
   <211> 91
   <212> DNA
   <213> Artificial
<220>
   <223> pre-crRNA
<400> 131
   guuuuagagc uaugctguuu ugaauggucc caaacacuuu uaaaguucug cuaugguuuu 60
   agagcuaugc tguuuugaau ggucccaaaa c 91
<210> 132
   <211> 11
   <212> RNA
   <213> Artificial
<220>
   <223> Cleaved tracrRNA after first processing event
<400> 132
   ggaaccauuc a 11
<210> 133
   <211> 75
   <212> DNA
   <213> Artificial
<220>
   <223> Cleaved tracrRNA
<400> 133
   aaacagcaua gcaaguuaaa auaaggcuag uccguuauca acuugaaaaa guggcaccga 60
   gucggugcuu uuuuu 75
<210> 134
   <211> 55
   <212> DNA
   <213> Artificial
<220>
   <223> cleaved pre-crRNA
<400> 134
   aaugguccca aacacuuuua aaguucugcu augguuuuag agcuaugctg uuuug 55
<210> 135
   <211> 42
   <212> DNA
   <213> Artificial
<220>
   <223> Cleaved pre-crRNA after 2nd processing event
<400> 135
   acuuuuaaag uucugcuaug guuuuagagc uaugctguuu ug 42
<210> 136
   <211> 20
   <212> DNA
   <213> Unknown
<220>
   <223> crRNA complementary sequence within target beta-lactamase DNA sequence
<400> 136
   acttttaaag ttctgctatg 20
<210> 137
   <211> 38
   <212> DNA
   <213> Unknown
<220>
   <223> CR90-containing target beta-lactamase DNA sequence anti-protospacer strand
<400> 137
   ccaagtcaac ttttaaagtt ctgctatgtg gcgcggta 38
<210> 138
   <211> 38
   <212> DNA
   <213> Unknown
<220>
   <223> CR90-containing target beta-lactamase DNA sequence protospacer strand
<400> 138
   taccgcgcca catagcagaa ctttaaaagt tgacttgg 38
<210> 139
   <211> 20
   <212> DNA
   <213> Unknown
<220>
   <223> crRNA complementary sequence within target beta-lactamase DNA sequence - reverse strand
<400> 139
   catagcagaa ctttaaaagt 20
<210> 140
   <211> 4758
   <212> DNA
   <213> Streptococcus pyogenes
<400> 140
   atgccggtac tgccgggcct cttgcgggat ccagaagtct ttttcttgca ctgtttcctt      60
   ttctttatga tagtttacga aatcatcctg tggagcttag taggtttagc aagatggcag 120
   cgcctaaatg tagaatgata aaaggattaa gagattaatt tccctaaaaa tgataaaaca 180
   agcgttttga aagcgcttgt ttttttggtt tgcagtcaga gtagaataga agtatcaaaa 240
   aaagcaccga ctcggtgcca ctttttcaag ttgataacgg actagcctta ttttaacttg 300
   ctatgctgtt ttgaatggtt ccaacaagat tattttataa cttttataac aaataatcaa 360
   ggagaaattc aaagaaattt atcagccata aaacaatact taatactata gaatgataac 420
   aaaataaact actttttaaa agaattttgt gttataatct atttattatt aagtattggg 480
   taatattttt tgaagagata ttttgaaaaa gaaaaattaa agcatattaa actaatttcg 540
   gaggtcatta aaactattat tgaaatcatc aaactcatta tggatttaat ttaaactttt 600
   tattttagga ggcaaaaatg gataagaaat actcaatagg cttagatatc ggcacaaata 660
   gcgtcggatg ggcggtgatc actgatgaat ataaggttcc gtctaaaaag ttcaaggttc 720
   tgggaaatac agaccgccac agtatcaaaa aaaatcttat aggggctctt ttatttgaca 780
   gtggagagac agcggaagcg actcgtctca aacggacagc tcgtagaagg tatacacgtc 840
   ggaagaatcg tatttgttat ctacaggaga ttttttcaaa tgagatggcg aaagtagatg 900
   atagtttctt tcatcgactt gaagagtctt ttttggtgga agaagacaag aagcatgaac 960
   gtcatcctat ttttggaaat atagtagatg aagttgctta tcatgagaaa tatccaacta 1020
   tctatcatct gcgaaaaaaa ttggtagatt ctactgataa agcggatttg cgcttaatct 1080
   atttggcctt agcgcatatg attaagtttc gtggtcattt tttgattgag ggagatttaa 1140
   atcctgataa tagtgatgtg gacaaactat ttatccagtt ggtacaaacc tacaatcaat 1200
   tatttgaaga aaaccctatt aacgcaagtg gagtagatgc taaagcgatt ctttctgcac 1260
   gattgagtaa atcaagacga ttagaaaatc tcattgctca gctccccggt gagaagaaaa 1320
   atggcttatt tgggaatctc attgctttgt cattgggttt gacccctaat tttaaatcaa 1380
   attttgattt ggcagaagat gctaaattac agctttcaaa agatacttac gatgatgatt 1440
   tagataattt attggcgcaa attggagatc aatatgctga tttgtttttg gcagctaaga 1500
   atttatcaga tgctatttta ctttcagata tcctaagagt aaatactgaa ataactaagg 1560
   ctcccctatc agcttcaatg attaaacgct acgatgaaca tcatcaagac ttgactcttt 1620
   taaaagcttt agttcgacaa caacttccag aaaagtataa agaaatcttt tttgatcaat 1680
   caaaaaacgg atatgcaggt tatattgatg ggggagctag ccaagaagaa ttttataaat 1740
   ttatcaaacc aattttagaa aaaatggatg gtactgagga attattggtg aaactaaatc 1800
   gtgaagattt gctgcgcaag caacggacct ttgacaacgg ctctattccc catcaaattc 1860
   acttgggtga gctgcatgct attttgagaa gacaagaaga cttttatcca tttttaaaag 1920
   acaatcgtga gaagattgaa aaaatcttga cttttcgaat tccttattat gttggtccat 1980
   tggcgcgtgg caatagtcgt tttgcatgga tgactcggaa gtctgaagaa acaattaccc 2040
   catggaattt tgaagaagtt gtcgataaag gtgcttcagc tcaatcattt attgaacgca 2100
   tgacaaactt tgataaaaat cttccaaatg aaaaagtact accaaaacat agtttgcttt 2160
   atgagtattt tacggtttat aacgaattga caaaggtcaa atatgttact gaaggaatgc 2220
   gaaaaccagc atttctttca ggtgaacaga agaaagccat tgttgattta ctcttcaaaa 2280
   caaatcgaaa agtaaccgtt aagcaattaa aagaagatta tttcaaaaaa atagaatgtt 2340
   ttgatagtgt tgaaatttca ggagttgaag atagatttaa tgcttcatta ggtacctacc 2400
   atgatttgct aaaaattatt aaagataaag attttttgga taatgaagaa aatgaagata 2460
   tcttagagga tattgtttta acattgacct tatttgaaga tagggagatg attgaggaaa 2520
   gacttaaaac atatgctcac ctctttgatg ataaggtgat gaaacagctt aaacgtcgcc 2580
   gttatactgg ttggggacgt ttgtctcgaa aattgattaa tggtattagg gataagcaat 2640
   ctggcaaaac aatattagat tttttgaaat cagatggttt tgccaatcgc aattttatgc 2700
   agctgatcca tgatgatagt ttgacattta aagaagacat tcaaaaagca caagtgtctg 2760
   gacaaggcga tagtttacat gaacatattg caaatttagc tggtagccct gctattaaaa 2820
   aaggtatttt acagactgta aaagttgttg atgaattggt caaagtaatg gggcggcata 2880
   agccagaaaa tatcgttatt gaaatggcac gtgaaaatca gacaactcaa aagggccaga 2940
   aaaattcgcg agagcgtatg aaacgaatcg aagaaggtat caaagaatta ggaagtcaga 3000
   ttcttaaaga gcatcctgtt gaaaatactc aattgcaaaa tgaaaagctc tatctctatt 3060
   atctccaaaa tggaagagac atgtatgtgg accaagaatt agatattaat cgtttaagtg 3120
   attatgatgt cgatcacatt gttccacaaa gtttccttaa agacgattca atagacaata 3180
   aggtcttaac gcgttctgat aaaaatcgtg gtaaatcgga taacgttcca agtgaagaag 3240
   tagtcaaaaa gatgaaaaac tattggagac aacttctaaa cgccaagtta atcactcaac 3300
   gtaagtttga taatttaacg aaagctgaac gtggaggttt gagtgaactt gataaagctg 3360
   gttttatcaa acgccaattg gttgaaactc gccaaatcac taagcatgtg gcacaaattt 3420
   tggatagtcg catgaatact aaatacgatg aaaatgataa acttattcga gaggttaaag 3480
   tgattacctt aaaatctaaa ttagtttctg acttccgaaa agatttccaa ttctataaag 3540
   tacgtgagat taacaattac catcatgccc atgatgcgta tctaaatgcc gtcgttggaa 3600
   ctgctttgat taagaaatat ccaaaacttg aatcggagtt tgtctatggt gattataaag 3660
   tttatgatgt tcgtaaaatg attgctaagt ctgagcaaga aataggcaaa gcaaccgcaa 3720
   aatatttctt ttactctaat atcatgaact tcttcaaaac agaaattaca cttgcaaatg 3780
   gagagattcg caaacgccct ctaatcgaaa ctaatgggga aactggagaa attgtctggg 3840
   ataaagggcg agattttgcc acagtgcgca aagtattgtc catgccccaa gtcaatattg 3900
   tcaagaaaac agaagtacag acaggcggat tctccaagga gtcaatttta ccaaaaagaa 3960
   attcggacaa gcttattgct cgtaaaaaag actgggatcc aaaaaaatat ggtggttttg 4020
   atagtccaac ggtagcttat tcagtcctag tggttgctaa ggtggaaaaa gggaaatcga 4080
   agaagttaaa atccgttaaa gagttactag ggatcacaat tatggaaaga agttcctttg 4140
   aaaaaaatcc gattgacttt ttagaagcta aaggatataa ggaagttaaa aaagacttaa 4200
   tcattaaact acctaaatat agtctttttg agttagaaaa cggtcgtaaa cggatgctgg 4260
   ctagtgccgg agaattacaa aaaggaaatg agctggctct gccaagcaaa tatgtgaatt 4320
   ttttatattt agctagtcat tatgaaaagt tgaagggtag tccagaagat aacgaacaaa 4380
   aacaattgtt tgtggagcag cataagcatt atttagatga gattattgag caaatcagtg 4440
   aattttctaa gcgtgttatt ttagcagatg ccaatttaga taaagttctt agtgcatata 4500
   acaaacatag agacaaacca atacgtgaac aagcagaaaa tattattcat ttatttacgt 4560
   tgacgaatct tggagctccc gctgctttta aatattttga tacaacaatt gatcgtaaac 4620
   gatatacgtc tacaaaagaa gttttagatg ccactcttat ccatcaatcc atcactggtc 4680
   tttatgaaac acgcattgat ttgagtcagc taggaggtga ctgatggcca cgtgaactat 4740
   atgattttcc gcagtata 4758
<210> 141
   <211> 276
   <212> DNA
   <213> Streptococcus pyogenes
<400> 141
   attgatttga gtcagctagg aggtgactga tggccacgtg aactatatga ttttccgcag 60
   tatattttag atgaagatta tttcttaata actaaaaata tggtataata ctcttaataa 120
   atgcagtaat acaggggctt ttcaagactg aagtctagct gagacaaata gtgcgattac 180
   gaaatttttt agacaaaaat agtctacgag gttttagagc tatgctgttt tgaatggtcc 240
   caaaactgag accagtctcg gacgtccaaa ggtctc 276
<210> 142
   <211> 10
   <212> DNA
   <213> Streptococcus pyogenes
<400> 142
   ggtctccatt 10
<210> 143
   <211> 10
   <212> DNA
   <213> Streptococcus pyogenes
<400> 143
   ggtcccaaaa 10
<210> 144
   <211> 452
   <212> DNA
   <213> Artificial
<220>
   <223> Fragment 3, amplified from genomic DNA of S. pyrogenes strain SF370
<400> 144
   gagaccagtc tcggacgtcc aaaggtctcg ttttagagct atgctgtttt gaatggtccc    60
   aaaacaacat tgccgatgat aacttgagaa agagggttaa taccagcagt cggatacctt   120
   cctattcttt ctgttaaagc gttttcatgt tataataggc aaaagaagag tagtgtgatg   180
   gaacaaacat tttttatgat taagccatat ggggttaagc aaggggaggt agttggagag   240
   gttttacggt ggattgaacg cctaagattt acgtttaagc gattcgagct aagacaagct   300
   agttcgaaat acttggctaa gcacgacgag gccttggtga taaacctttt gatcctaaac   360
   ttaaagctta catgacaagt ggtcctgttt taattgggat aattcttggg gactaaggtg   420
   gtatcgtcca ttccgacagc atcgccagtc ac                                 452
<210> 145
   <211> 9578
   <212> DNA
   <213> Artificial
<220>
   <223> Sequence of the final construct pNB100
<400> 145
   gaattccgga tgagcattca tcaggcgggc aagaatgtga ataaaggccg gataaaactt      60
   gtgcttattt ttctttacgg tctttaaaaa ggccgtaata tccagctgaa cggtctggtt     120
   ataggtacat tgagcaactg actgaaatgc ctcaaaatgt tctttacgat gccattggga     180
   tatatcaacg gtggtatatc cagtgatttt tttctccatt ttagcttcct tagctcctga     240
   aaatctcgat aactcaaaaa atacgcccgg tagtgatctt atttcattat ggtgaaagtt     300
   ggaacctctt acgtgccgat caacgtctca ttttcgccaa aagttggccc agggcttccc     360
   ggtatcaaca gggacaccag gatttattta ttctgcgaag tgatcttccg tcacaggtat     420
   ttattcggcg caaagtgcgt cgggtgatgc tgccaactta ctgatttagt gtatgatggt     480
   gtttttgagg tgctccagtg gcttctgttt ctatcagctg tccctcctgt tcagctactg     540
   acggggtggt gcgtaacggc aaaagcaccg ccggacatca gcgctagcgg agtgtatact     600
   ggcttactat gttggcactg atgagggtgt cagtgaagtg cttcatgtgg caggagaaaa     660
   aaggctgcac cggtgcgtca gcagaatatg tgatacagga tatattccgc ttcctcgctc     720
   actgactcgc tacgctcggt cgttcgactg cggcgagcgg aaatggctta cgaacggggc     780
   ggagatttcc tggaagatgc caggaagata cttaacaggg aagtgagagg gccgcggcaa     840
   agccgttttt ccataggctc cgcccccctg acaagcatca cgaaatctga cgctcaaatc     900
   agtggtggcg aaacccgaca ggactataaa gataccaggc gtttccccct ggcggctccc     960
   tcgtgcgctc tcctgttcct gcctttcggt ttaccggtgt cattccgctg ttatggccgc    1020
   gtttgtctca ttccacgcct gacactcagt tccgggtagg cagttcgctc caagctggac    1080
   tgtatgcacg aaccccccgt tcagtccgac cgctgcgcct tatccggtaa ctatcgtctt    1140
   gagtccaacc cggaaagaca tgcaaaagca ccactggcag cagccactgg taattgattt    1200
   agaggagtta gtcttgaagt catgcgccgg ttaaggctaa actgaaagga caagttttgg    1260
   tgactgcgct cctccaagcc agttacctcg gttcaaagag ttggtagctc agagaacctt    1320
   cgaaaaaccg ccctgcaagg cggttttttc gttttcagag caagagatta cgcgcagacc    1380
   aaaacgatct caagaagatc atcttattaa tcagataaaa tatttctaga tttcagtgca    1440
   atttatctct tcaaatgtag cacctgaagt cagccccata cgatataagt tgtaattctc    1500
   atgtttgaca gcttatcatc gataagcttt aatgcggtag tttatcacag ttaaattgct    1560
   aacgcagtca ggcaccgtgt atgaaatcta acaatgcgct catcgtcatc ctcggcaccg    1620
   tcaccctgga tgctgtaggc ataggcttgg ttatgccggt actgccgggc ctcttgcggg    1680
   atccagaagt ctttttcttg cactgtttcc ttttctttat gatagtttac gaaatcatcc    1740
   tgtggagctt agtaggttta gcaagatggc agcgcctaaa tgtagaatga taaaaggatt    1800
   aagagattaa tttccctaaa aatgataaaa caagcgtttt gaaagcgctt gtttttttgg   1860
   tttgcagtca gagtagaata gaagtatcaa aaaaagcacc gactcggtgc cactttttca    1920
   agttgataac ggactagcct tattttaact tgctatgctg ttttgaatgg ttccaacaag    1980
   attattttat aacttttata acaaataatc aaggagaaat tcaaagaaat ttatcagcca    2040
   taaaacaata cttaatacta tagaatgata acaaaataaa ctacttttta aaagaatttt    2100
   gtgttataat ctatttatta ttaagtattg ggtaatattt tttgaagaga tattttgaaa    2160
   aagaaaaatt aaagcatatt aaactaattt cggaggtcat taaaactatt attgaaatca    2220
   tcaaactcat tatggattta atttaaactt tttattttag gaggcaaaaa tggataagaa    2280
   atactcaata ggcttagata tcggcacaaa tagcgtcgga tgggcggtga tcactgatga    2340
   atataaggtt ccgtctaaaa agttcaaggt tctgggaaat acagaccgcc acagtatcaa    2400
   aaaaaatctt ataggggctc ttttatttga cagtggagag acagcggaag cgactcgtct    2460
   caaacggaca gctcgtagaa ggtatacacg tcggaagaat cgtatttgtt atctacagga    2520
   gattttttca aatgagatgg cgaaagtaga tgatagtttc tttcatcgac ttgaagagtc 2580
   ttttttggtg gaagaagaca agaagcatga acgtcatcct atttttggaa atatagtaga 2640
   tgaagttgct tatcatgaga aatatccaac tatctatcat ctgcgaaaaa aattggtaga 2700
   ttctactgat aaagcggatt tgcgcttaat ctatttggcc ttagcgcata tgattaagtt 2760
   tcgtggtcat tttttgattg agggagattt aaatcctgat aatagtgatg tggacaaact 2820
   atttatccag ttggtacaaa cctacaatca attatttgaa gaaaacccta ttaacgcaag 2880
   tggagtagat gctaaagcga ttctttctgc acgattgagt aaatcaagac gattagaaaa 2940
   tctcattgct cagctccccg gtgagaagaa aaatggctta tttgggaatc tcattgcttt 3000
   gtcattgggt ttgaccccta attttaaatc aaattttgat ttggcagaag atgctaaatt 3060
   acagctttca aaagatactt acgatgatga tttagataat ttattggcgc aaattggaga 3120
   tcaatatgct gatttgtttt tggcagctaa gaatttatca gatgctattt tactttcaga 3180
   tatcctaaga gtaaatactg aaataactaa ggctccccta tcagcttcaa tgattaaacg 3240
   ctacgatgaa catcatcaag acttgactct tttaaaagct ttagttcgac aacaacttcc 3300
   agaaaagtat aaagaaatct tttttgatca atcaaaaaac ggatatgcag gttatattga 3360
   tgggggagct agccaagaag aattttataa atttatcaaa ccaattttag aaaaaatgga 3420
   tggtactgag gaattattgg tgaaactaaa tcgtgaagat ttgctgcgca agcaacggac 3480
   ctttgacaac ggctctattc cccatcaaat tcacttgggt gagctgcatg ctattttgag 3540
   aagacaagaa gacttttatc catttttaaa agacaatcgt gagaagattg aaaaaatctt 3600
   gacttttcga attccttatt atgttggtcc attggcgcgt ggcaatagtc gttttgcatg 3660
   gatgactcgg aagtctgaag aaacaattac cccatggaat tttgaagaag ttgtcgataa 3720
   aggtgcttca gctcaatcat ttattgaacg catgacaaac tttgataaaa atcttccaaa 3780
   tgaaaaagta ctaccaaaac atagtttgct ttatgagtat tttacggttt ataacgaatt 3840
   gacaaaggtc aaatatgtta ctgaaggaat gcgaaaacca gcatttcttt caggtgaaca 3900
   gaagaaagcc attgttgatt tactcttcaa aacaaatcga aaagtaaccg ttaagcaatt 3960
   aaaagaagat tatttcaaaa aaatagaatg ttttgatagt gttgaaattt caggagttga 4020
   agatagattt aatgcttcat taggtaccta ccatgatttg ctaaaaatta ttaaagataa 4080
   agattttttg gataatgaag aaaatgaaga tatcttagag gatattgttt taacattgac 4140
   cttatttgaa gatagggaga tgattgagga aagacttaaa acatatgctc acctctttga 4200
   tgataaggtg atgaaacagc ttaaacgtcg ccgttatact ggttggggac gtttgtctcg 4260
   aaaattgatt aatggtatta gggataagca atctggcaaa acaatattag attttttgaa 4320
   atcagatggt tttgccaatc gcaattttat gcagctgatc catgatgata gtttgacatt 4380
   taaagaagac attcaaaaag cacaagtgtc tggacaaggc gatagtttac atgaacatat 4440
   tgcaaattta gctggtagcc ctgctattaa aaaaggtatt ttacagactg taaaagttgt 4500
   tgatgaattg gtcaaagtaa tggggcggca taagccagaa aatatcgtta ttgaaatggc 4560
   acgtgaaaat cagacaactc aaaagggcca gaaaaattcg cgagagcgta tgaaacgaat 4620
   cgaagaaggt atcaaagaat taggaagtca gattcttaaa gagcatcctg ttgaaaatac 4680
   tcaattgcaa aatgaaaagc tctatctcta ttatctccaa aatggaagag acatgtatgt 4740
   ggaccaagaa ttagatatta atcgtttaag tgattatgat gtcgatcaca ttgttccaca 4800
   aagtttcctt aaagacgatt caatagacaa taaggtctta acgcgttctg ataaaaatcg 4860
   tggtaaatcg gataacgttc caagtgaaga agtagtcaaa aagatgaaaa actattggag 4920
   acaacttcta aacgccaagt taatcactca acgtaagttt gataatttaa cgaaagctga 4980
   acgtggaggt ttgagtgaac ttgataaagc tggttttatc aaacgccaat tggttgaaac 5040
   tcgccaaatc actaagcatg tggcacaaat tttggatagt cgcatgaata ctaaatacga 5100
   tgaaaatgat aaacttattc gagaggttaa agtgattacc ttaaaatcta aattagtttc 5160
   tgacttccga aaagatttcc aattctataa agtacgtgag attaacaatt accatcatgc 5220
   ccatgatgcg tatctaaatg ccgtcgttgg aactgctttg attaagaaat atccaaaact 5280
   tgaatcggag tttgtctatg gtgattataa agtttatgat gttcgtaaaa tgattgctaa 5340
   gtctgagcaa gaaataggca aagcaaccgc aaaatatttc ttttactcta atatcatgaa 5400
   cttcttcaaa acagaaatta cacttgcaaa tggagagatt cgcaaacgcc ctctaatcga 5460
   aactaatggg gaaactggag aaattgtctg ggataaaggg cgagattttg ccacagtgcg 5520
   caaagtattg tccatgcccc aagtcaatat tgtcaagaaa acagaagtac agacaggcgg 5580
   attctccaag gagtcaattt taccaaaaag aaattcggac aagcttattg ctcgtaaaaa 5640
   agactgggat ccaaaaaaat atggtggttt tgatagtcca acggtagctt attcagtcct 5700
   agtggttgct aaggtggaaa aagggaaatc gaagaagtta aaatccgtta aagagttact 5760
   agggatcaca attatggaaa gaagttcctt tgaaaaaaat ccgattgact ttttagaagc 5820
   taaaggatat aaggaagtta aaaaagactt aatcattaaa ctacctaaat atagtctttt 5880
   tgagttagaa aacggtcgta aacggatgct ggctagtgcc ggagaattac aaaaaggaaa 5940
   tgagctggct ctgccaagca aatatgtgaa ttttttatat ttagctagtc attatgaaaa 6000
   gttgaagggt agtccagaag ataacgaaca aaaacaattg tttgtggagc agcataagca 6060
   ttatttagat gagattattg agcaaatcag tgaattttct aagcgtgtta ttttagcaga 6120
   tgccaattta gataaagttc ttagtgcata taacaaacat agagacaaac caatacgtga 6180
   acaagcagaa aatattattc atttatttac gttgacgaat cttggagctc ccgctgcttt 6240
   taaatatttt gatacaacaa ttgatcgtaa acgatatacg tctacaaaag aagttttaga 6300
   tgccactctt atccatcaat ccatcactgg tctttatgaa acacgcattg atttgagtca 6360
   gctaggaggt gactgatggc cacgtgaact atatgatttt ccgcagtata ttttagatga 6420
   agattatttc ttaataacta aaaatatggt ataatactct taataaatgc agtaatacag 6480
   gggcttttca agactgaagt ctagctgaga caaatagtgc gattacgaaa ttttttagac 6540
   aaaaatagtc tacgaggttt tagagctatg ctattttgaa tggtcccaaa actgagacca 6600
   gtctcggacg tccaaaggtc tcgttttaga gctatgctgt tttgaatggt cccaaaacaa 6660
   cattgccgat gataacttga gaaagagggt taataccagc agtcggatac cttcctattc 6720
   tttctgttaa agcgttttca tgttataata ggcaaaagaa gagtagtgtg atggaacata 6780
   cattttttat gattaagcca tatggggtta agcaagggga ggtagttgga gaggttttac 6840
   ggtggattga acgcctaaga tttacgttta agcgattcga gctaagacaa gctagttcga 6900
   aatacttggc taagcacgac gaggccttgg tgataaacct tttgatccta aacttaaagc 6960
   ttacatgaca agtggtcctg ttttaattgg gataattctt ggggactaag gtggtatcgt 7020
   ccattccgac agcatcgcca gtcactatgg cgtgctgcta gcgctatatg cgttgatgca 7080
   atttctatgc gcacccgttc tcggagcact gtccgaccgc tttggccgcc gcccagtcct 7140
   gctcgcttcg ctacttggag ccactatcga ctacgcgatc atggcgacca cacccgtcct 7200
   gtggatcctc tacgccggac gcatcgtggc cggcatcacc ggcgccacag gtgcggttgc 7260
   tggcgcctat atcgccgaca tcaccgatgg ggaagatcgg gctcgccact tcgggctcat 7320
   gagcgcttgt ttcggcgtgg gtatggtggc aggccccgtg gccgggggac tgttgggcgc 7380
   catctccttg catgcaccat tccttgcggc ggcggtgctc aacggcctca acctactact 7440
   gggctgcttc ctaatgcagg agtcgcataa gggagagcgt cgaccgatgc ccttgagagc 7500
   cttcaaccca gtcagctcct tccggtgggc gcggggcatg actatcgtcg ccgcacttat 7560
   gactgtcttc tttatcatgc aactcgtagg acaggtgccg gcagcgctct gggtcatttt 7620
   cggcgaggac cgctttcgct ggagcgcgac gatgatcggc ctgtcgcttg cggtattcgg 7680
   aatcttgcac gccctcgctc aagccttcgt cactggtccc gccaccaaac gtttcggcga 7740
   gaagcaggcc attatcgccg gcatggcggc cgacgcgctg ggctacgtct tgctggcgtt 7800
   cgcgacgcga ggctggatgg ccttccccat tatgattctt ctcgcttccg gcggcatcgg 7860
   gatgcccgcg ttgcaggcca tgctgtccag gcaggtagat gacgaccatc agggacagct 7920
   tcaaggatcg ctcgcggctc ttaccagcct aacttcgatc attggaccgc tgatcgtcac 7980
   ggcgatttat gccgcctcgg cgagcacatg gaacgggttg gcatggattg taggcgccgc 8040
   cctatacctt gtctgcctcc ccgcgttgcg tcgcggtgca tggagccggg ccacctcgac 8100
   ctgaatggaa gccggcggca cctcgctaac ggattcacca ctccaagaat tggagccaat 8160
   caattcttgc ggagaactgt gaatgcgcaa accaaccctt ggcagaacat atccatcgcg 8220
   tccgccatct ccagcagccg cacgcggcgc atctcgggca gcgttgggtc ctggccacgg 8280
   gtgcgcatga tcgtgctcct gtcgttgagg acccggctag gctggcgggg ttgccttact 8340
   ggttagcaga atgaatcacc gatacgcgag cgaacgtgaa gcgactgctg ctgcaaaacg 8400
   tctgcgacct gagcaacaac atgaatggtc ttcggtttcc gtgtttcgta aagtctggaa 8460
   acgcggaagt cccctacgtg ctgctgaagt tgcccgcaac agagagtgga accaaccggt 8520
   gataccacga tactatgact gagagtcaac gccatgagcg gcctcatttc ttattctgag 8580
   ttacaacagt ccgcaccgct gtccggtagc tccttccggt gggcgcgggg catgactatc 8640
   gtcgccgcac ttatgactgt cttctttatc atgcaactcg taggacaggt gccggcagcg 8700
   cccaacagtc ccccggccac ggggcctgcc accataccca cgccgaaaca agcgccctgc 8760
   accattatgt tccggatctg catcgcagga tgctgctggc taccctgtgg aacacctaca 8820
   tctgtattaa cgaagcgcta accgttttta tcaggctctg ggaggcagaa taaatgatca 8880
   tatcgtcaat tattacctcc acggggagag cctgagcaaa ctggcctcag gcatttgaga 8940
   agcacacggt cacactgctt ccggtagtca ataaaccggt aaaccagcaa tagacataag 9000
   cggctattta acgaccctgc cctgaaccga cgaccgggtc gaatttgctt tcgaatttct 9060
   gccattcatc cgcttattat cacttattca ggcgtagcac caggcgttta agggcaccaa 9120
   taactgcctt aaaaaaatta cgccccgccc tgccactcat cgcagtactg ttgtaattca 9180
   ttaagcattc tgccgacatg gaagccatca cagacggcat gatgaacctg aatcgccagc 9240
   ggcatcagca ccttgtcgcc ttgcgtataa tatttgccca tggtgaaaac gggggcgaag 9300
   aagttgtcca tattggccac gtttaaatca aaactggtga aactcaccca gggattggct 9360
   gagacgaaaa acatattctc aataaaccct ttagggaaat aggccaggtt ttcaccgtaa 9420
   cacgccacat cttgcgaata tatgtgtaga aactgccgga aatcgtcgtg gtattcactc 9480
   cagagcgatg aaaacgtttc agtttgctca tggaaaacgg tgtaacaagg gtgaacacta 9540
   tcccatatca ccagctcacc gtctttcatt gccatacg 9578
<210> 146
   <211> 386
   <212> DNA
   <213> Artificial
<220>
   <223> Amplified sequence with primer NB018 and NB019 from pNB100 as template
<400> 146
   ccaaaactga gacctgctgc ggacgtccaa aggtctcgtt ttagagctat gctgttttga 60
   atggtcccaa aacttgccga tgataacttg agaaagaggg ttaataccag cagtcggata 120
   ccttcctatt ctttctgtta aagcgttttc atgttataat aggcaaattt tagatgaaga 180
   ttatttctta ataactaaaa atatggtata atactcttaa taaatgcagt aatacagggg 240
   cttttcaaga ctgaagtcta gctgagacaa atagtgcgat tacgaaattt tttagacaaa 300
   aatagtctac gaggttttag agctatgctg ttttgaatgg tcccaaaact gaagagcgtc 360
   tcggacgcag cgctcttcgt tttaga 386
<210> 147
   <211> 744
   <212> DNA
   <213> Artificial
<220>
   <223> Modified CRISPR array in pNB200
<400> 147
   ggtgactgat ggccacgtga actatatgat tttccgcagt atattttaga tgaagattat 60
   ttcttaataa ctaaaaatat ggtataatac tcttaataaa tgcagtaata caggggcttt 120
   tcaagactga agtctagctg agacaaatag tgcgattacg aaatttttta gacaaaaata 180
   gtctacgagg ttttagagct atgctatttt gaatggtccc aaaactgaga cctgctgcgg 240
   acgtccaaag gtctcgtttt agagctatgc tgttttgaat ggtcccaaaa cttgccgatg 300
   ataacttgag aaagagggtt aataccagca gtcggatacc ttcctattct ttctgttaaa 360
   gcgttttcat gttataatag gcaaatttta gatgaagatt atttcttaat aactaaaaat 420
   atggtataat actcttaata aatgcagtaa tacaggggct tttcaagact gaagtctagc 480
   tgagacaaat agtgcgatta cgaaattttt tagacaaaaa tagtctacga ggttttagag 540
   ctatgctgtt ttgaatggtc ccaaaactga agagcgtctc ggacgcagcg ctcttcgttt 600
   tagagctatg ctgttttgaa tggtcccaaa acaacattgc cgatgataac ttgagaaaga 660
   gggttaatac cagcagtcgg ataccttcct attctttctg ttaaagcgtt ttcatgttat 720
   aataggcaaa agaagagtag tgtg 744
<210> 148
   <211> 23
   <212> DNA
   <213> Artificial
<220>
   <223> Primer sequence
<400> 148
   ccaactacct ccccttgctt aac 23
<210> 149
   <211> 18
   <212> DNA
   <213> Artificial
<220>
   <223> Primer sequence
<400> 149
   ggtgactgat ggccacgt 18
<210> 150
   <211> 23
   <212> DNA
   <213> Artificial
<220>
   <223> Primer sequence
<400> 150
   gggctggcaa gccacgtttg gtg 23
<210> 151
   <211> 23
   <212> DNA
   <213> Artificial
<220>
   <223> Primer sequence
<400> 151
   ccgggagctg catgtgtcag agg 23
<210> 152
   <211> 43
   <212> DNA
   <213> Artificial
<220>
   <223> Primer sequence
<400> 152
   attgaaaaag gaagagtatg gaattgccca atattatgca ccc 43
<210> 153
   <211> 38
   <212> DNA
   <213> Artificial
<220>
   <223> Primer sequence
<400> 153
   agtcccgcta ggtctcaacc gtcagcgcag cttgtcgg 38
<210> 154
   <211> 42
   <212> DNA
   <213> Artificial
<220>
   <223> Primer sequence
<400> 154
   attgaaaaag gaagagtatg cgtgtattag ccttatcggc tg 42
<210> 155
   <211> 55
   <212> DNA
   <213> Artificial
<220>
   <223> Primer sequence
<400> 155
   agtcccgcta ggtctcaacc gctagggaat aattttttcc tgtttgagca cttct 55
<210> 156
   <211> 47
   <212> DNA
   <213> Artificial
<220>
   <223> Primer sequence
<400> 156
   attgaaaaag gaagagtatg cgttattttc gcctgtgtat tatctcc 47
<210> 157
   <211> 41
   <212> DNA
   <213> Artificial
<220>
   <223> Primer sequence
<400> 157
   agtcccgcta ggtctcaacc gttagcgttg ccagtgctcg a 41
<210> 158
   <211> 57
   <212> DNA
   <213> Artificial
<220>
   <223> Primer sequence
<400> 158
   attgaaaaag gaagagtatg ttaaaagtta ttagtagttt attggtctac atgaccg 57
<210> 159
   <211> 48
   <212> DNA
   <213> Artificial
<220>
   <223> Primer sequence
<400> 159
   agtcccgcta ggatgacctg gctgaccgct actcggcgac tgagcgat 48
<210> 160
   <211> 43
   <212> DNA
   <213> Artificial
<220>
   <223> Primer sequence
<400> 160
   attgaaaaag gaagagtatg tcactgtatc gccgtctagt tct 34
<210> 161
   <211> 39
   <212> DNA
   <213> Artificial
<220>
   <223> KPC-3 reverse primer
<400> 161
   agtcccgcta ggtctcaacc gttactgccc gttgacgcc 39
<210> 162
   <211> 60
   <212> DNA
   <213> Artificial
<220>
   <223> Primer sequence
<400> 162
   attgaaaaag gaagagtatg agcaagttat ctgtattctt tatatttttg ttttgtagca 60
<210> 163
   <211> 61
   <212> DNA
   <213> Artificial
<220>
   <223> Primer sequence
<400> 163
   agtcccgcta ggtctcaacc gttagttgct tagttttgat ggttttttac tttcgtttaa 60
c 61
<210> 164
   <211> 44
   <212> DNA
   <213> Artificial
<220>
   <223> Primer sequence
<400> 164
   attgaaaaag gaagagtatg gttaaaaaat cactgcgcca gttc 44
<210> 165
   <211> 47
   <212> DNA
   <213> Artificial
<220>
   <223> Primer sequence
<400> 165
   agtcccgcta ggtctcaacc gttacaaacc gtcggtgacg attttag 47
<210> 166
   <211> 834
   <212> DNA
   <213> Unknown
<220>
   <223> NDM-1 sequence
<400> 166
   accgtcagcg cagcttgtcg gccatgcggg ccgtatgagt gattgcggcg cggctatcgg      60
   gggcggaatg gctcatcacg atcatgctgg ccttggggaa cgccgcacca aacgcgcgcg     120
   ctgacgcggc gtagtgctca gtgtcggcat caccgagatt gccgagcgac ttggccttgc     180
   tgtccttgat caggcagcca ccaaaagcga tgtcggtgcc gtcgatccca acggtgatat     240
   tgtcactggt gtggccgggg ccggggtaaa ataccttgag cgggccaaag ttgggcgcgg     300
   ttgctggttc gacccagcca ttggcggcga aagtcaggct gtgttgcgcc gcaaccatcc     360
   cctcttgcgg ggcaagctgg ttcgacaacg cattggcata agtcgcaatc cccgccgcat     420
   gcagcgcgtc cataccgccc atcttgtcct gatgcgcgtg agtcaccacc gccagcgcga     480
   ccggcaggtt gatctcctgc ttgatccagt tgaggatctg ggcggtctgg tcatcggtcc     540
   aggcggtatc gaccaccagc acgcggccgc catccctgac gatcaaaccg ttggaagcga     600
   ctgccccgaa acccggcatg tcgagatagg aagtgtgctg ccagacattc ggtgcgagct     660
   ggcggaaaac cagatcgcca aaccgttggt cgccagtttc catttgctgg ccaatcgtcg     720
   ggcggatttc accgggcatg cacccgctca gcatcaatgc agcggctaat gcggtgctca     780
   gcttcgcgac cgggtgcata atattgggca attccatact cttccttttt caat           834
<210> 167
   <211> 819
   <212> DNA
   <213> Unknown
<220>
   <223> OXA-48 sequence
<400> 167
   accgctaggg aataattttt tcctgtttga gcacttcttt tgtgatggct tggcgcagcc       60
   ctaaaccatc cgatgtgggc atatccatat tcatcgcaaa aaaccacaca ttatcatcaa      120
   gttcaaccca accgacccac cagccaatct taggttcgat tctagtcgag tatccagttt      180
   tagcccgaat aatatagtca ccattggctt cggtcagcat ggcttgtttg acaatacgct      240
   ggctgcgctc cgatacgtgt aacttattgt gatacagctt tcttaaaaag ctgatttgct      300
   ccgtggccga aattcgaata ccaccgtcga gccagaaact gtctacattg cccgaaatgt      360
   cctcattacc ataatcgaaa gcatgtagca tcttgctcat acgtgcctcg ccaatttggc      420
   gggcaaattc ttgataaaca ggcacaactg aatatttcat cgcggtgatt agattatgat      480
   cgcgattcca agtggcgata tcgcgcgtct gtccatccca cttaaagact tggtgttcat      540
   ccttaaccac gcccaaatcg agggcgatca agctattggg aattttaaag gtagatgcgg      600
   gtaaaaatgc ttggttcgcc cgtttaagat tattggtaaa tccttgctgc ttattctcat      660
   tccagagcac aactacgccc tgtgatttat gttcagtaaa gtgagcattc caacttttgt      720
   tttcttgcca ttcctttgct accgcaggca ttccgataat cgatgccacc aaaaacacag      780
   ccgataaggc taatacacgc atactcttcc tttttcaat                             819
<210> 168
   <211> 882
   <212> DNA
   <213> Unknown
<220>
   <223> SHV-1 sequence
<400> 168
   accgttagcg ttgccagtgc tcgatcagcg ccgcgccgat cccggcgatt tgctgatttc      60
   gctcggccat gctcgccggc gtatcccgca gataaatcac cacaatccgc tctgctttgt     120
   tattcgggcc aagcagggcg acaatcccgc gcgcaccccg tttggcagct ccggtcttat     180
   cggcgataaa ccagcccgcc ggcagcacgg agcggatcaa cggtccggcg acccgatcgt     240
   ccaccatcca ctgcagcagc tgccgttgcg aacgggcgct cagacgctgg ctggtcagca     300
   gcttgcgcag ggtcgcggcc atgctggccg gggtagtggt gtcgcgggcg tcgccgggaa     360
   gcgcctcatt cagttccgtt tcccagcggt caaggcgggt gacgttgtcg ccgatctggc     420
   gcaaaaaggc agtcaatcct gcggggccgc cgacggtggc cagcagcaga ttggcggcgc     480
   tgttatcgct catggtaatg gcggcggcac agagttcgcc gaccgtcatg ccgtcggcaa     540
   ggtgtttttc gctgaccggc gagtagtcca ccagatcctg ctggcgatag tggatctttc     600
   gctccagctg ttcgtcaccg gcatccaccc gcgccagcac tgcgccgcag agcactactt     660
   taaaggtgct catcatggga aagcgttcat cggcgcgcca ggcggtcagc gtgcggccgc     720
   tggccagatc catttctatc atgcctacgc tgcccgacag ctggctttcg cttagtttaa     780
   tttgctcaag cggctgcggg ctggcgtgta ccgccagcgg cagggtggct aacagggaga     840
   taatacacag gcgaaaataa cgcatactct tcctttttca at                          882
<210> 169
   <211> 822
   <212> DNA
   <213> Unknown
<220>
   <223> VIM-1 sequence
<400> 169
   accgctactc ggcgactgag cgatttttgt gtgctttgac aacgttcgct gtgtgctgga 60
   gcaagtctag accgcccggt agaccgtgcc cgggaatgac gacctctgct tccgggtagt 120
   gtttttgaat ccgctcaacg gaggtgggcc attcagccag atcggcatcg gccacgttcc 180
   ccgcagacgt gcttgacaac tcatgaacgg cacaaccacc gtatagcacg ttcgctgacg 240
   ggacgtatac aaccagattg tcggtcgaat gcgcagcacc aggatagaag agctctactg 300
   gaccgaagcg cactgcgtcc ccgctcgatg agagtccttc tagagaatgc gtgggaatct 360
   cgttcccctc tgcctcggct agccggcgtg tcgacggtga tgcgtacgtt gccaccccag 420
   ccgcccgaag gacatcaacg ccgccgacgc ggtcgtcatg aaagtgcgtg gagactgcac 480
   gcgttacggg aagtccaatt tgcttttcaa tctccgcgag aagtgccgct gtgtttttcg 540
   caccccacgc tgtatcaatc aaaagcaact catcaccatc acggacaatg agaccattgg 600
   acgggtagac cgcgccatca aacgactgcg ttgcgatatg cgaccaaaca ccatcggcaa 660
   tctggtaaag tcggacctct ccgaccggaa tttcgttgac tgtcggatac tcaccactcg 720
   gctccccgga atgggctaac ggacttgcga cagccatgac agacgcggtc atgtagacca 780
   ataaactact aataactttt aacatactct tcctttttca at 822
<210> 170
   <211> 903
   <212> DNA
   <213> Unknown
<220>
   <223> KPC-3 sequence
<400> 170
   accgttactg cccgttgacg cccaatccct cgagcgcgag tctagccgca gcggcgatga 60
   cggcctcgct gtacttgtca tccttgttag gcgcccgggt gtagacggcc aacacaatag 120
   gtgcgcgccc agtgggccag acgacggcat agtcatttgc cgtgccatac actccgcagg 180
   ttccggtttt gtctccgact gcccagtctg ccggcaccgc cgcgcggatg cggtggttgc 240
   cggtcgtgtt tccctttagc caatcaacaa actgctgccg ctgcggcgca gccagtgcag 300
   agcccagtgt cagtttttgt aagctttccg tcacggcgcg cggcgatgag gtatcgcgcg 360
   catcgcctgg gatggcggag ttcagctcca gctcccagcg gtccagacgg aacgtggtat 420
   cgccgataga gcgcatgaag gccgtcagcc cggccgggcc gcccaactcc ttcagcaaca 480
   aattggcggc ggcgttatca ctgtattgca cggcggccgc ggacagctcc gccaccgtca 540
   tgcctgttgt cagatatttt tccgagatgg gtgaccacgg aaccagcgca tttttgccgt 600
   aacggatggg tgtgtccagc aagccggcct gctgctggct gcgagccagc acagcggcag 660
   caagaaagcc cttgaatgag ctgcacagtg ggaagcgctc ctcagcgcgg taacttacag 720
   ttgcgcctga gccggtatcc atcgcgtaca caccgatgga gccgccaaag tcctgttcga 780
   gtttagcgaa tggttccgcg acgaggttgg tcagcgcggt ggcagaaaag ccagccagcg 840
   gccatgagag acaagacagc agaactagac ggcgatacag tgacatactc ttcctttttc 900
   aat 903
<210> 171
   <211> 762
   <212> DNA
   <213> Unknown
<220>
   <223> IMP-4 sequence
<400> 171
   accgttagtt gcttagtttt gatggttttt tactttcgtt taacccttta accgcctgct 60
   ctaatgtaag tttcaagagt gatgcgtctc cagcttcact gtgacttgga acaaccagtt 120
   ttgccttacc atatttggat attaataatt tagcggactt tggccaagct tctaaatttg 180
   cgtcacccaa attacctaga ccgtacggtt taataaaaca accaccgaat aatattttcc 240
   tttcaggcag ccaaactact aggttatctg gagtgtgtcc tgggcctgga taaaaaactt 300
   caattttatt tttaactagc caatagttaa ccccgccaaa tgaattttta gcttgaacct 360
   taccgtcttt tttaagcagc tcattagtta attcagacgc atacgtgggg atggattgag 420
   aattaagcca ctctattccg cccgtgctgt cactatgaaa atgagaggaa atactgcctt 480
   ttattttata gccacgttcc acaaaccaag tgactaactt ttcagtatct ttagccgtaa 540
   atggagtgtc aattagataa gcttcagcat ctacaagaac aaccaaacca tgtttaggaa 600
   caacgcccca cccgttaact tcttcaaacg aagtatgaac ataaacgcct tcatcaagtt 660
   tttcaatttt taaatctggc aaagactctg ctgcggtagc aatgctacaa aacaaaaata 720
   taaagaatac agataacttg ctcatactct tcctttttca at 762
<210> 172
   <211> 897
   <212> DNA
   <213> Unknown
<220>
   <223> CTX-M-15 sequence
<400> 172
   accgttacaa accgtcggtg acgattttag ccgccgacgc taatacatcg cgacggcttt      60
   ctgccttagg ttgaggctgg gtgaagtaag tgaccagaat cagcggcgca cgatcttttg     120
   gccagatcac cgcgatatcg ttggtggtgc catagccacc gctgccggtt ttatccccca     180
   caacccagga agcaggcagt ccagcctgaa tgctcgctgc accggtggta ttgcctttca     240
   tccatgtcac cagctgcgcc cgttggctgt cgcccaatgc tttacccagc gtcagattcc     300
   gcagagtttg cgccattgcc cgaggtgaag tggtatcacg cggatcgccc ggaatggcgg     360
   tgtttaacgt cggctcggta cggtcgagac ggaacgtttc gtctcccagc tgtcgggcga     420
   acgcggtgac gctagccggg ccgccaacgt gagcaatcag cttattcatc gccacgttat     480
   cgctgtactg tagcgcggcc gcgctaagct cagccagtga catcgtccca ttgacgtgct     540
   tttccgcaat cggattatag ttaacaaggt cagatttttt gatctcaact cgctgattta     600
   acagattcgg ttcgctttca cttttcttca gcaccgcggc cgcggccatc actttactgg     660
   tgctgcacat cgcaaagcgc tcatcagcac gataaagtat ttgcgaatta tctgctgtgt     720
   taatcaatgc cacacccagt ctgcctcccg actgccgctc taattcggca agtttttgct     780
   gtacgtccgc cgtttgcgca tacagcggca cacttcctaa caacagcgtg acggttgccg     840
   tcgccatcag cgtgaactgg cgcagtgatt ttttaaccat actcttcctt tttcaat        897